# EUROPEAN PATENT SPECIFICATION

(11) **EP 1 933 878 B1**
(45) Date of publication and mention of the grant of the patent: **01.02.2012**
(21) Application number: 06791990.2
(22) Date of filing: 12.09.2006
(51) Int. Cl.: A61K 47/48, C08B 31/00

(54) **CONJUGATES OF HYDROXYALKYL STARCH AND AN ACTIVE SUBSTANCE, PREPARED BY CHEMICAL LIGATION VIA THIAZOLIDINE**
KONJUGATE VON HYDROXYALKYL-STÄRKE UND EINER WIRKSUBSTANZ, HERGESTELLT DURCH CHEMISCHE LIGATION ÜBER THIAZOLIDIN
CONJUGUÉS D'AMIDON HYDROXYALKYLE ET UNE SUBSTANCE ACTIVE OBTENUE PAR LIGATION CHIMIQUE VIA THIAZOLIDINE

(30) Priority: 12.09.2005 EP 05019768
(43) Date of publication of application: 25.06.2008
(73) Proprietor: Fresenius Kabi Deutschland GmbH, 61352 Bad Homburg (DE)
(72) Inventor: ZANDER, Norbert, 38527 Meine (DE); KNOLLER, Helmut, 61169 Friedberg (DE)
(74) Representative: Altmann, Andreas
(86) International application number: PCT/EP2006/008858
(87) International publication number: WO 2007/031266

(56) References cited:
- EP-A- 1 398 328
- WO-A-99/07719
- WO-A-03/000738
- DE-A1- 10 155 098
- FR-A- 2 378 094
- J. RADOMSKY AND A. TEMERIUSZ: "Thiazolidine-4(R)-carboxylic acids derived from sugars: part I, C-2-epimerisation in aqueous solutions" CARBOHYDRATE RESEARCH, vol. 187, 1989, pages 223-237, XP002357090 The Netherlands
- F. GUILLAUMIE ET AL.: "Immobilization of Pectin Fragments on solid supports: novel coupling by thiazolidine formation" BIOCONJUGATE CHEMISTRY, vol. 13, 2002, pages 285-294, XP002357091
- W. YANG ET AL.: "FUNCTIONAL CHANGES OF CARBOXYMETHYL POTATO STARCH BY CONJUGATION WITH AMINO ACIDS" BIOSCIENCE BIOTECHNOLOGY AND BIOCHEMISTRY, vol. 59, no. 12, 1995, pages 2203-2206, XP008056456
- "European Pharmacopoiea" 2002, EDQM COUNCIL OF EUROPE , STRASSBOURG , XP002357095 pa. 1127 last 6 lines
- JAQUES L.W. ET AL.: "NMR Spectroscopy and calcium binding of sialic acids: N-glycolylneuraminic acid and periodate-oxidized N-acetylneuraminic acid" CARBOHYDRATE RESEARCH, vol. 83, 1980, pages 21-32, XP002357104 The Netherlands cited in the application
- SHAO J. AND TAM J. P.: "Unprotected peptides as building blocks for the synthesis of peptide dendrimers with oxime, hydrazone and thiazolidine linkages" JOURNAL OF THE AMERICAN CHEMICAL SOCIETY, vol. 117, no. 14, 1995, pages 3893-3899, XP002172763 cited in the application
- DATABASE IMMUNE EPITOPE [Online] C Mayorga et Al.: "Epitope mapping of beta-lactam antibiotics with the use of monoclonal antibodies", Database accession no. 1005311

## Description

The present invention relates to a method for preparing conjugates of an active substance and hydroxyalkyl starch and to conjugates of an active substance and hydroxyalkyl starch, preferably hydroxyethyl starch, wherein the conjugates are prepared by covalently linking the hydroxyalkyl starch and the active substance by a chemical residue having a structure according to formula (I) 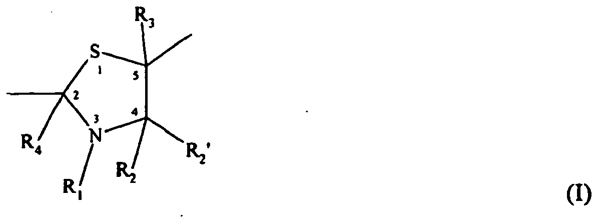 or formula (I') 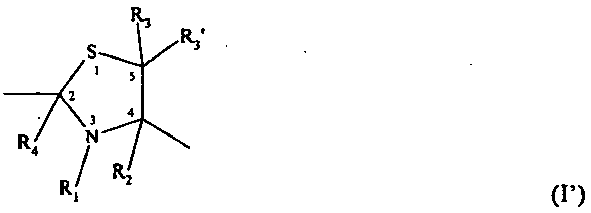 or formula (I'') 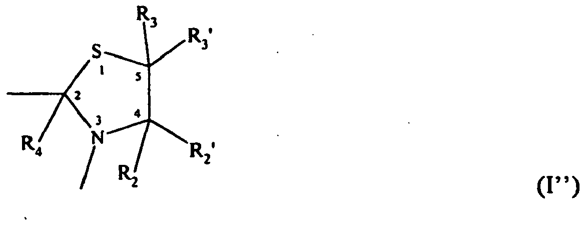 wherein R₁, R₂, R_{2'}, R₃, R₃, and R₄ are independently selected from the group consisting of hydrogen, an optionally suitably substituted, linear, cyclic and/or branched alkyl, aryl, heteroaryl, aralkyl, and heteroaralkyl group, preferably hydrogen.

WO 03/031581 A2 discloses a method of conjugating a polymer derivative to a polypeptide having a cysteine or histidine residue at the N-terminus, wherein said method comprises providing a polypeptide having a cysteine or histidine residue at the N-terminus, providing a thioester-terminated polymer, the polymer comprising a water soluble and non-peptidic polymer backbone, preferably a polyethylene glycole polymer, and reacting the polymer derivative and the polypeptide. As polymers, poly(alkylene glycol), copolymers of ethylene glycol and propylene glycol, poly(oxyethylated polyol), poly(olefinic alcohol), poly(vinylpyrrolidone), poly(alpha hydroxy acid), poly(vinyl alcohol), polyphosphazene, polyoxazoline, poly(N-acryloylmorpholine), polyacrylate, polyacrylamides, polysaccharides, and copolymers, terpolymers and mixtures thereof are used. All explicitly disclosed polymers are polyethylenglycol polymers.

Despite the progress of coupling methods and use of monofunctional PEG-molecules, a general disadvantage of PEGylated drugs is that the metabolization pathway of PEG as a non-natural polymer is not known in detail.

Further, a general approach for forming peptide dendrimers with oxime, hydrazone, and thiazolidine linkages is known from J. Am Chem Soc. 1995, 117, 3893-3899. Unprotected peptides as building blocks and selective ligation between an aldehyde and a weak base is described there.

WO 99/07719 A1 discloses prodrugs and conjugates of thiol- and seleonol-containing compounds and methods of use thereof. Among several other compounds, a prodrug is disclosed which is prepared by the reaction of L-cysteine ethyl ester and a D-ribose, a monosaccharide, said prodrug containing a thiazolidine ring. As general formula for the monosaccharide, (CHOH)ₙCH₂OH with n = 1 to 5 is disclosed. Not even di-saccharides, let alone high-molecular polymeric compounds such as starch, in particular hydroxyalkyl starch, are disclosed in combination with the formation of thiazolidine rings.

EP-A- 1 398 328 discloses a method of conjugating hydroxyalkyl starch to a polypeptide via an amino group comprising linker. No coupling via formation of a thiazolidine moiety is disclosed.

WO 99/07719 discloses prodrugs of thiol and selenol containing active agents, wherein the active agent is e.g. coupled to saccharides. The conjugates are formed for example via a direct thiazolidine linkage between the active agent and the oligosaccharides. Conjugates between hydroxylalkyl starch and an active agent comprising a linker are not mentioned.

Therefore, it was an object of the present invention to provide novel conjugates of an active substance and a polymer formed by covalent linking wherein no polyalkylene glycol, especially no polyethylene glycol is used as polymer.

Accordingly, it was another object of the present invention to provide a method for preparing these conjugates.

The solution of these problems is a method for preparing a conjugate of an active substance and hydroxyalkyl starch, wherein the active substance and the hydroxyalkyl starch are covalently linked by a chemical residue having a structure according to formula (I) 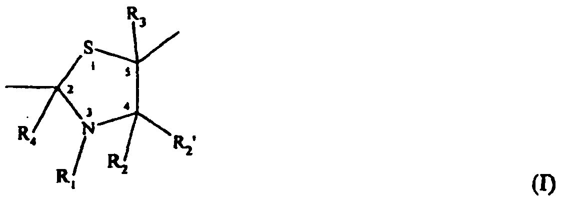 or formula (I') 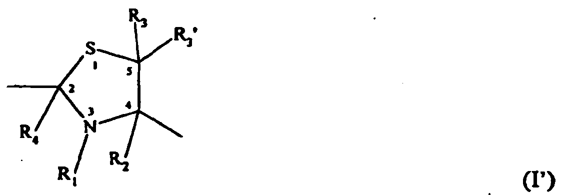 or formula (I'') 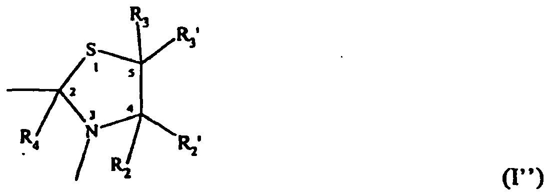 wherein R₁, R₂, R_{2'}, R₃, R_{3'} and R₄ are independently selected from the group consisting of hydrogen, an optionally suitably substituted, linear, cyclic and/or branched alkyl, aryl, heteroaryl, aralkyl, and heteroaralkyl group, preferably hydrogen,
said method comprising
(A) reacting an aldehyde group, a keto group or a hemiacetal group of a hydroxyalkyl starch derivative comprising said aldehyde group, keto group or hemiacetal group with an alpha-SH-beta amino group 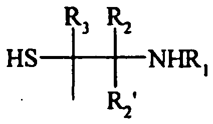 of an active substance or a derivative thereof comprising said alpha-SH-beta amino group, thereby preparing a conjugate of said active substance and said hydroxyalkyl starch being covalently linked by a chemical residue having a structure according to formula (I); or with an alpha-SH-beta amino group 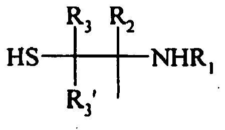 of an active substance or a derivative thereof comprising said alpha-SH-beta amino group, thereby preparing a conjugate of said active substance and said hydroxyalkyl starch being covalently linked by a chemical residue having a structure according to formula (I'), or with an alpha-SH-beta amino group 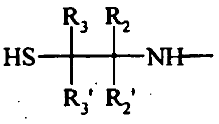 of an active substance or a derivative thereof comprising said alpha-SH-beta amino group, thereby preparing a conjugate of said active substance and said hydroxyalkyl starch being covalently linked by a chemical residue having a structure according to formula (I"), wherein R₁, R₂, R_{2'}, R₃, R_{3'} and R₄ are as defined above;
   or
(B) reacting an aldehyde group, a keto group or a hemiacetal group of an active substance or a derivative thereof comprising said aldehyde group, keto group or hemiacetal group with an alpha-SH-beta amino group 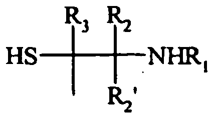 of a hydroxyalkyl starch derivative comprising said alpha-SH-beta amino group, thereby preparing a conjugate of said active substance and said hydroxyalkyl starch being covalently linked by a chemical residue having a structure according to formula (I); or with an alpha-SH-beta amino group 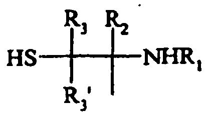 of a hydroxyalkyl starch derivative comprising said alpha-SH-beta amino group, thereby preparing a conjugate of said active substance and said hydroxyalkyl starch being covalently linked by a chemical residue having a structure according to formula (I'); or with an alpha-SH-beta amino group 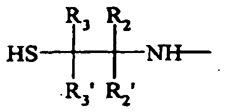 of a hydroxyalkyl starch derivative comprising said alpha-SH-beta amino group, thereby preparing a conjugate of said active substance and said hydroxyalkyl starch being covalently linked by a chemical residue having a structure according to formula (I''), wherein R₁, R₂, R_{2'}, R₃, R_{3'} and R₄ are as defined above, and wherein the hydroxyalkyl starch derivative comprising said aldehyde group, keto group, hemiacetal group or said alpha-SH-beta amino group is obtained by a method comprising reacting the hydroxyalkyl starch with at least one, at least bifunctional compound, said compound comprising two functional groups M₁ and Q, one functional group M₁ being reacted with the hydroxyalkyl starch and one functional group Q being
   (i) an aldehyde group, keto group, hemiacetal group or an alpha-SH-beta amino group; or
   (ii) a functional group being chemically modified to give the aldehyde group, keto group, hemiacetal group or the alpha-SH-beta amino group.

Therefore, the term "alpha-SH-beta-amino group" as used in the context of the present invention refers to an ethylene group in which an optionally protected SH group is bonded to a carbon atom and an optionally protected primary or secondary amino group is bonded to the neighbouring carbon atom. The bonds in the above formulas on which, at one end thereof, no residue is indicated are those bonds to which either hydroxyalkyl starch or the active substance are attached.

The term "alkyl" as used in the context of the invention is preferably an alkyl group having 1 to 20, more preferred 1 to 10 and in particular 1 to 4 carbon atoms, unless defined differently hereinafter.

The term "aryl" as used in the context of the invention is preferably an aryl group having 6 to 20, more preferred 6 to 14 and in particular 6 carbon atoms, unless defined differently hereinafter.

The term "heteroaryl" as used in the context of the invention is preferably an heteroaryl group having 6 to 20, more preferred 6 to 14 and in particular 6 carbon atoms and wherein at least one, preferably 1 to 3, in particular 1, of the carbon atoms is substituted by a heteroatom, such as S, N and/or O, unless defined differently hereinafter.

The term "aralkyl" as used in the context of the invention is preferably an aryl group bonded through an alkyl group to the reminder of the compound, unless defined differently hereinafter.

The term "alkaryl" as used in the context of the invention is preferably an alkyl group bonded through an aryl group to the reminder of the compound, unless defined differently hereinafter.

The term "heteroaralkyl" as used in the context of the invention is preferably an heteroaryl group bonded through an alkyl group to the reminder of the compound, unless defined differently hereinafter.

The term "optionally suitably substituted" as used in the context of the invention preferably means that 1 to 10, more preferred 1 to 4, in particular 1 substituent is present, unless defined differently hereinafter.

The term "active substance" as used in the context of the present invention relates to a substance which can affect any physical or biochemical property of a biological organism including, viruses, bacteria, fungi, plants, animals, and humans. In particular, the term "active substance" as used in the context of the present invention relates to a substance intended for diagnosis, cure, mitigation, treatment, or prevention of disease in humans or animals, or to otherwise enhance physical or mental well-being of humans or animals. Examples of active substances include, peptides, proteins, enzymes, small molecule drugs, dyes, lipids, nucleosides, nucleotides, oligonucleotides, polynucleotides, nucleic acids, cells, viruses, liposomes, microparticles, and micelles.

Examples of proteins include, erythropoietin (EPO) such as recombinant human EPO (rhEPO), colony-stimulating factors (CSF), such as G-CSF like recombinant human G-CSF (rhG-CSF), alpha-Interferon (IFN alpha), beta-Interferon (IFN beta) or gamma-Interferon (IFN gamma), such as recombinant human IFN alpha or IFN beta (rhIFN alpha or rhIFN beta), interleukines, e.g. IL-1 to IL-18 such as IL-2 or IL-3 like recombinant human IL-2 or IL-3 (rhIL-2 or rhIL-3), serum proteins such as coagulation factors II-XIII like factor VIII, alphal-antitrypsin (A1AT), activated protein C (APC), plasminogen activators such as tissue-type plasminogen activator (tPA), such as human tissue plasminogen activator (hTPA), AT III such as recombinant human AT III (rhAT III), myoglobin, albumin such as bovine serum albumin (BSA), growth factors, such as epidermal growth factor (EGF), thrombocyte growth factor (PDGF), fibroblast growth factor (FGF), brain-derived growth factor (BDGF), nerve growth factor (NGF), B-cell growth factor (BCGF), brain-derived neurotrophic growth factor (BDNF), ciliary neurotrophic factor (CNTF), transforming growth factors such as TGF alpha or TGF beta, BMP (bone morphogenic proteins), growth hormones such as human growth hormone, tumor necrosis factors such as TNF alpha or TNF beta, somatostatine (peptide), somatotropine, somatomedines, hemoglobin, hormones or prohormones such as insulin, gonadotropin, melanocyte-stimulating hormone (alpha-MSH), triptorelin, hypthalamic hormones such as antidiuretic hormones (ADH) and oxytocin as well as releasing hormones and release-inhibiting hormones, parathyroid hormone, thyroid hormones such as thyroxine, thyrotropin, thyroliberin, prolactin, calcitonin, glucagon, glucagon-like peptides (GLP-1, GLP-2 etc.), exendines such as exendin-4, leptin, vasopressin, gastrin, secretin, integrins, glycoprotein hormones (e.g. LH, FSH etc.), melanoside-stimulating hormones, lipoproteins and apo-lipoproteins such as apo-B, apo-E, apo-Lₐ, immunoglobulins such as IgG, IgE, IgM, IgA, IgD and fragments thereof, hirudin, tissue-pathway inhibitor, plant proteins such as lectin or ricin, bee-venom, snake-venom, immunotoxins, antigen E, alpha-proteinase inhibitor, ragweed allergen, melanin, oligolysine proteins, RGD proteins or optionally corresponding receptors for one of these proteins; or a functional derivative or fragment of any of these proteins or receptors. In a particularly preferred embodiment, the active substance is EPO, in particular oxidized EPO as described in the following.

Examples of enzymes include, carbohydrate-specific enzymes, proteolytic enzymes, oxidases, oxidoreductases, transferases, hydrolases, lyases, isomerases, kinases and ligases. Specific non-limiting examples are asparaginase, arginase, arginin deaminase, adenosin deaminase, glutaminase, glutaminase-asparaginase, phenylalanine, tryptophanase, tyrosinase, superoxide dismutase (SOD), endotoxinase, catalase, peroxidase, kallikrein, trypsin, chymotrypsin, elastase, thermolysin, lipase, uricase, adenosine diphosphatase, purine nucleoside phosphorylase, bilirubin oxidase, glucose oxidase, glucodase, gluconate oxidase, galactosidase, glucocerebrosidase, glucuronidase, hyaluronidase, tissue factor, streptokinase, urokinase, MAP-kinases, DNAses, RNAses, lactoferrin and functional derivatives or fragments thereof.

According to one alternative of the present invention, the active substance is a small molecule drug, a peptide, and/or a protein.

Among others, the following proteins are to be mentioned explicitly: erythropoietin (EPO) such as recombinant human EPO (rhEPO), colony-stimulating factors (CSF), such as G-CSF like recombinant human G-CSF (rhG-CSF), alpha-Interferon (IFN alpha), beta-Interferon (IFN beta) or gamma-Interferon (IFN gamma) such as recombinant human IFN alpha or IFN beta (rhIFN alpha or rhIFN beta)serum proteins such as coagulation factors II-XIII like factor VII, factor VIII, or factor IX, alpha1-antitrypsin (A1AT), activated protein C (APC), plasminogen activators such as tissue-type plasminogen activator (tPA), such as human tissue plasminogen activator (hTPA), AT III such as recombinant human AT III (rhAT III).

Examples for peptides include ACTH, adrenomedullin, amyloid beta-protein, angiotensin I, angiotensin II, atrial natriuretic peptide (ANP), antibody fragments, bradykinin, brain natriuretic peptide B-Type (BNP), calcitonin, corticotropin-releasing factor (CRF), endorphins, endothelins, enkephalins, gastrin, gastrin related peptide, gastric inhibitory polypeptide (GIP), gastrin releasing peptide (GRP), glucagon, glucagon-like peptides, growth hormone releasing factor (GRF), hepatocyte growth factor, insulins, gonadotropin-releasing hormone (LH-RH, GnRH), neurokinins, oxytocin, parathyroid hormone, somatostatin, substance P, thyrotropin releasing hormone (TRH), vasoactive intestinal peptide (VIP), and vasopressin.

The active substance is preferably selected from the group composed of antibiotics, antidepressants, antidiabetics, antidiuretics, anticholinergics, antiarrhythmics, antiemetics, antitussives, antiepileptics, antihistamines, antimycotics, antisympathotonics, antithrombotics, androgens, antiandrogens, estrogens, antiestrogens, antiosteoporotics, antitumor agents, vasodilators, other antihypertensive agents, antipyretic agents, analgesics, antiinflammatory agents, β-blockers, cytostatics, immunosuppressants and vitamins.

Some additional, non-restrictive examples of active substances are albuterol, alendronate, amikazin, ampicillin, amoxicillin, amphotericin B, atenolol, azathioprine, cefaclor, cefadroxil, cefotaxime, ceftazidime, ceftriaxone, cilastatin, cimetidine, ciprofloxacin, clonidine, colistin, cosyntropin, cycloserine, daunorubicin, doxorubicin, desmopressin, dihydroergotamine, dobutamine, dopamine, ephedrine, epinephrine, ε-aminocaproic acid, ergometrine, esmolol, famotidine, flecainide, folic acid, flucytosine, furosemide, ganciclovir, gentamicin, glucagon, hydrazaline, imipenem, isoproterenol, ketamine, liothyronine, LHRH, merpatricin, metaraminol, methyldopa, metoclopramide, metoprolol, mexiletine, mitomycin, neomicin, netilmicin, nimodipine, nystatin, octreotide, oxytocin, pamidronate, pentamidine, phentolamine, phenylephrine, procainamide, procaine, propranolol, ritodrine, sotalol, teicoplanin, terbutaline, thiamine, tiludronate, tolazoline, trimethoprim, tromethamine, tylosin, vancomycin, vasopressin, and vinblastine.

According to an alternative, one may also try to use rifamycine, tetracycline, spectomycine, streptomycine or erythromycine as active substances.

Examples for an oligonucleotide are aptamers, DNA, RNA, PNA or derivatives thereof.

In the context of the present invention, the term "hydroxyalkyl starch" (HAS) refers to a starch derivative which has been substituted by at least one hydroxyalkyl group. A preferred hydroxyalkyl starch of the present invention has a constitution according to formula (II) 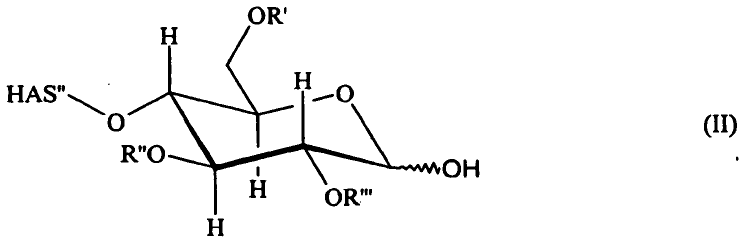 wherein R, R'' and R''' are independently hydrogen, a linear or branched hydroxyalkyl group or the group

-[(CR¹R²)ₘO]ₙ(CR³R⁴]ₒ-OH

wherein R¹, R², R³, and R⁴ are independently selected from the group, consisting of hydrogen, and alkyl group, preferably hydrogen and methyl group,
m is 2 to 4, wherein the residues R¹ and R² may be the same or different in the m groups CR¹R²; n is 0 to 20, preferably 0 to 4; o is 0 to 20, preferably 2 to 4, wherein in the case of n = 0, o is not 0, and wherein the residues R³ and R⁴ may be the same or different in the o groups CR³R⁴,
where the residue HAS" together with the terminal glucose moiety constitutes the HAS molecule, i.e. formula (II) shows a HAS molecule, the terminal carbohydrate moiety of which is explicitly shown, the remaining part of the starch molecule being HAS".

In formula (II) the reducing end of the starch molecule is shown in the non-oxidized form and the terminal saccharide unit of HAS is shown in the hemiacetal form which, depending on e.g. the solvent, may be in equilibrium with the aldehyde form. The abbreviation HAS" as used in the context of the present invention refers to the HAS molecule without the terminal saccharide unit at the reducing end of the HAS molecule.

The term hydroxyalkyl starch as used in the present invention is not limited to compounds where the terminal carbohydrate moiety comprises hydroxyalkyl groups R', R", and/or R"' as depicted, for the sake of brevity, in formula (II), but also refers to compounds in which at least one hydroxyalkyl group which is present anywhere, either in the terminal carbohydrate moiety and/or in the remaining part of the starch molecule, HAS", is substituted by a hydroxyalkyl group R', R", or R'''.

Hydroxyalkyl starch comprising two or more different hydroxyalkyl groups is also possible.

The at least one hydroxyalkyl group comprised in HAS may contain one or more, in particular two or more hydroxy groups. According to a preferred embodiment, the at least one hydroxyalkyl group comprised in HAS contains one hydroxy group.

The expression "hydroxyalkyl starch" also includes derivatives wherein the alkyl group is mono- or polysubstituted. In this context, it is preferred that the alkyl group is substituted with a halogen, especially fluorine, or with an aryl group. Furthermore, the hydroxy group of a hydroxyalkyl group may be esterified or etherified.

Furthermore, instead of alkyl, also linear or branched substituted or unsubstituted alkene groups may be used.

Hydroxyalkyl starch is an ether derivative of starch. Besides of said ether derivatives, also other starch derivatives can be used in the context of the present invention. For example, derivatives are useful which comprise esterified hydroxy groups. These derivatives may be e.g. derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially derivatives of acetic acid. In this context, acetyl starch, butyryl starch and propionyl starch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Whiley-VCH, Weinheim, New York, especially chapter 4.4, Esterification of Cellulose (ISBN 3-527-29489-9).

According to a preferred embodiment of the present invention, hydroxyalkyl starch according to above-mentioned formula (II) is employed. The other saccharide ring structures comprised in HAS" may be the same as or different from the explicitly described saccharide ring, with the difference that they lack a reducing end.

As far as the residues R', R" and R"' according to formula (II) are concerned there are no specific limitations. According to a preferred embodiment, R', R" and R"' are independently hydrogen or a hydroxyalkyl group, a hydroxyaralkyl group or a hydroxyalkaryl group having of from 2 to 10 carbon atoms in the respective alkyl residue. Hydrogen and hydroxyalkyl groups having of from 2 to 10 are preferred. More preferably, the hydroxyalkyl group has from 2 to 6 carbon atoms, more preferably from 2 to 4 carbon atoms, and even more preferably from 2 to 3 carbon atoms. In a preferred embodiment, hydroxyalkyl starch is hydroxyethyl starch in which R', R" and R''' are independently hydrogen or a group (CH₂CH₂O)ₙ-H, wherein n is an integer, preferably 0, 1, 2, 3, 4, 5, or 6.

"Hydroxyalkyl starch" therefore preferably comprises hydroxyethyl starch, hydroxypropyl starch and hydroxybutyl starch, wherein hydroxyethyl starch and hydroxypropyl starch are particularly preferred and hydroxyethyl starch is most preferred.

The alkyl, aralkyl and/or alkaryl group may be linear or branched and suitably substituted.

Therefore, the present invention also relates to a method and a conjugate as described above wherein R', R" and R''' are independently hydrogen or a linear or branched hydroxyalkyl group with from 2 to 6 carbon atoms.

Thus, R', R" and R''' preferably may be H, hydroxyhexyl, hydroxypentyl, hydroxybutyl, hydroxypropyl such as 2-hydroxypropyl, 3-hydroxypropyl, 2-hydroxyisopropyl, hydroxyethyl such as 2-hydroxyethyl, hydrogen and the 2-hydroxyethyl group being especially preferred.

Therefore, the present invention also relates to a method and a conjugate as described above wherein R', R" and R''' are independently hydrogen or a 2-hydroxyethyl group, an embodiment wherein at least one residue R', R" and R''' being 2-hydroxyethyl being especially preferred.

Hydroxyethyl starch (HES) is most preferred for all embodiments of the present invention.

Therefore, the present invention relates to the method and the conjugate as described above, wherein the polymer is hydroxyethyl starch and the polymer derivative is a hydroxyethyl starch derivative.

Hydroxyethyl starch (HES) is a derivative of naturally occurring amylopectin and is degraded by alpha-amylase in the body. HES is a substituted derivative of the carbohydrate polymer amylopectin, which is present in corn starch at a concentration of up to 95 % by weight. HES exhibits advantageous biological properties and is used as a blood volume replacement agent and in hemodilution therapy in the clinics (Sommermeyer et al., 1987, Krankenhauspharmazie, 8(8), 271-278; and Weidler et al., 1991, Arzneim.-Forschung/Drug Res., 41, 494-498).

Amylopectin consists of glucose moieties, wherein in the main chain alpha-1,4-glycosidic bonds are present and at the branching sites alpha-1,6-glycosidic bonds are found. The physico-chemical properties of this molecule are mainly determined by the type of glycosidic bonds. Due to the nicked alpha-1,4-glycosidic bond, helical structures with about six glucose-monomers per turn are produced. The physico-chemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

HES is mainly characterized by the molecular weight distribution and the degree of substitution. There are two possibilities of describing the substitution degree:
1. The degree of substitution can be described relatively to the portion of substituted glucose monomers with respect to all glucose moieties.
2. The degree of substitution can be described as the molar substitution, wherein the number of hydroxyethyl groups per glucose moiety is described.

In the context of the present invention, the degree of substitution, denoted as DS, relates to the molar substitution, as described above (see also Sommermeyer et al., 1987, Krankenhauspharmazie, 8(8), 271-278, as cited above, in particular p. 273).

HES solutions are present as polydisperse compositions, wherein each molecule differs from the other with respect to the polymerisation degree, the number and pattern of branching sites, and the substitution pattern. HES is therefore a mixture of compounds with different molecular weight. Consequently, a particular HES solution is determined by average molecular weight with the help of statistical means. In this context, Mₙ is calculated as the arithmetic mean depending on the number of molecules. Alternatively, M_{w} (or MW), the weight average molecular weight, represents a unit which depends on the mass of the HES.

Preferably, the hydroxyalkyl starch used in the invention has a mean molecular weight (weight mean) of from 1 to 300 kD. Hydroxyethyl starch can further exhibit a preferred molar substitution of from 0.1 to 3, preferably 0.1 to 2, more preferred 0.1 to 0.9 or 0.4 to 2, preferably 0.4 to 1.3, and a preferred ratio between C₂ : C₆ substitution in the range of from 2 to 20 with respect to the hydroxyethyl groups.

The term "mean molecular weight" as used in the context of the present invention relates to the weight as determined according to the LALLS-(low angle laser light scattering)-GPC method as described in Sommermeyer et al., 1987, Krankenhauspharmazie, 8(8), 271-278; and Weidler et al., 1991, Arzneim.-Forschung/Drug Res., 41, 494-498. For mean molecular weights of 10 kD and smaller, additionally, the calibration was carried out with a standard which had previously been qualified by LALLS-GPC.

According to a preferred embodiment of the present invention, the mean molecular weight of hydroxyethyl starch employed is from 1 to 300 kD, more preferably from 2 to 200 kD, more preferably of from 10 to 150 or 4 to 130 kD, more preferably of from 10 to 100 kD.

An example of HES having a mean molecular weight of about 1 to 300 kD, preferably 10 to 100 kD is a HES with a molar substitution of 0.1 to 3, preferably 0.4 to 1.3, such as 0.4, 0.5, 0.6, 0.7 0.8, 0.9, 1.0, 1.1, 1.2, or 1.3, preferably of 0.7 to 1.3, such as 0.7 0.8, 0.9, 1.0, 1.1, 1.2, or 1.3.

An example for HES with a mean molecular weight of about 130 kD is Voluven® from Fresenius. Voluven® is an artifical colloid, employed, e.g., for volume replacement used in the therapeutic indication for therapy and prophylaxis of hypovolaemia. The characteristics of Voluven® are a mean molecular weight of 130,000 +/- 20,000 D, a molar substitution of 0.4 and a C2 : C6 ratio of about 9:1.

The present invention also relates to a method and to conjugates as described above wherein the hydroxyalkyl starch is hydroxyethyl starch having a mean molecular weight of from 10 to 150 kD, preferably of from 10 to 100 kD.

Preferred ranges of the mean molecular weight are, e.g., 10 to 150 kD, or 10 to 130 kD, or 30 to 130 kD, or 50 to 130 kD, or 70 to 130 kD, or 100 to 130 kD, or 10 to 100 kD, or 4 to 100 kD, or 10 to 100 kD, or 12 to 100 kD, or 18 to 100 kD, or 50 to 100 kD, or 4 to 70 kD, or 10 to 70 kD, or 12 to 70 kD, or 18 to 70 kD, or 50 to 70 kD, or 4 to 50 kD or 10 to 50 kD, or 12 to 50 kD, or 18 to 50 kD, or 4 to 18 kD, or 10 to 18 kD, or 12 to 18 kD, or 4 to 12 kD, or 10 to 12 kD, or 4 to 10 kD.

According to particularly preferred embodiments of the present invention, the mean molecular weight of hydroxyethyl starch employed is in the range of from more than 4 kD and below 150 kD, such as about 10 kD, or in the range of from 9 to 10 kD or from 10 to 11 kD or from 9 to 11 kD, or about 12 kD, or in the range of from 11 to 12 kD or from 12 to 13 kD or from 11 to 13 kD, or about 15 kD, or in the range of 14 to 15 or from 15 to 16 kD, or about 18 kD, or in the range of from 17 to 18 kD or from 18 to 19 kD or from 17 to 19 kD, or about 50 kD, or in the range of from 49 to 50 kD or from 50 to 51 kD or from 49 to 51 kD, or about 56 kD, or in the range of 55 to 56 kD or from 56 to 57 kD.

According to another particularly preferred embodiment of the present invention, the mean molecular weight of hydroxyethyl starch employed is in the range of from more than 60 kD and up to 130 kD, such as about 70 kD or in the range of from 65 to 75 kD, or about 80 kD or in the range of from 75 to 85 kD, or about 90 kD or in the range of from 85 to 95 kD or about 100 kD, or in the range of from 95 to 105 kD, or about 110 kD or in the range of from 105 to 115 kD, or about 120 kD or in the range of 115 to 125 kD or about 130 kD or in the range of from 125 to 135 kD.

As to the upper limit of the molar substitution (DS), values of up to 3.0 such as 0.9, 1.0, 1.1, 1.2, 1.3, 1.4, 1.5, 1.6, 1.7, 1.8, 1.9 or 2.0 are also possible, values of below 2.0 being preferred, values of below 1.5 being more preferred, values of below 1.3 such as 0.7, 0.8, 0.9, 1.0, 1.1, 1.2, or 1.3 being still more preferred.

Therefore, preferred ranges of the molar substitution are from 0.1 to 2 or from 0.1 to 1.5 or from 0.1 to 1.3 or from 0.1 to 1.0 or from 0.1 to 0.9 or from 0.1 to 0.8. More preferred ranges of the molar substitution are from 0.2 to 2 or from 0.2 to 1.5 or from 0.2 to 1.0 or from 0.2 to 0.9 or from 0.2 to 0.8. Still more preferred ranges of the molar substitution are from 0.3 to 2 or from 0.3 to 1.5 or from 0.3 to 1.0 or from 0.3 to 0.9 or from 0.3 to 0.8. Even more preferred ranges of the molar substitution are from 0.4 to 2 or from 0.4 to 1.5 or from 0.4 to 1.3, or from 0.4 to 1.0 or from 0.4 to 0.9 or from 0.4 to 0.8.

As far as the molar substitution (DS) is concerned, DS is preferably at least 0.1, more preferably at least 0.2, more preferably at least 0.4 and more preferably at least 0.7. Preferred ranges of DS are from 0.1 to 3, preferably 0.1 to 2, more preferred 0.1 to 1.3, more preferred 0.1 to 0.9, more preferably from 0.1 to 0.8, more preferably from 0.2 to 0.8, more preferably from 0.3 to 0.8 and even more preferably from 0.4 to 0.8, still more preferably from 0.1 to 0.7, more preferably from 0.2 to 0.7, more preferably from 0.3 to 0.7 and more preferably from 0.4 to 0.7. Particularly preferred values of DS are, e.g., 0.1, 0.2, 0.3, 0.4, 0.5, 0.6, 0.7, 0.8, 0.9, 1.0, 1.2 or 1.3 with 0.2, 0.3, 0.4, 0.5, 0.6, 0.7 or 0.8 being more preferred, 0.3, 0.4, 0.5, 0.6, 0.7 or 0.8 being even more preferred, 0.4, 0.5, 0.6, 0.7 or 0.8 being still more preferred and, e.g. 0.4 or 0.5 and 0.7 or 0.8 being particularly preferred.

In the context of the present invention, a given value of the molar substitution such as 1.3 may be the exact value or may be understood as being in a range of from 1.25 to 1.34, or 1.0 may be the exact value or may be understood as being in a range of from 0.95 to 1.04, or 0.9 may be the exact value or may be understood as being in a range of from 0.85 to 0.94 or 0.8 may be the exact value or may be understood as being in a range of from 0.75 to 0.84. Therefore, for example, a given value of 0.1 may be the exact value of 0.1 or be in the range of from 0.05 to 0.14, a given value of 0.4 may be the exact value of 0.4 or in the range of from 0.35 to 0.44, or a given value of 0.7 may be the exact value of 0.7 or be in the range of from 0.65 to 0.74.

Particularly preferred combinations of molecular weight of the hydroxyalkyl starch, preferably hydroxyethyl starch, and its molar substitution DS are, e.g., 10 kD and 0.4 or 10 kD and 0.7 or 12 kD and 0.4 or 12 kD and 0.7 or 15 kD and 0.4 or 15 kD and 0.7 or 18 kD and 0.4 or 18 kD and 0.7 or 50 kD and 0.4 or 50 kD and 0.7 or 56 kD and 0.4 and 56 kD and 0.7 or 70 KD and 0.4 or 70 kD and 0.7, or 100 kD and 0.4 or 100 kD and 0.7 or 130 kD and 0.4 or 130 kD and 0.7.

As far as the ratio of C₂: C₆ substitution is concerned, said substitution is preferably in the range of from 2 to 20, more preferably in the range of from 2 to 15 and even more preferably in the range of from 3 to 12.

According to a further embodiment of the present invention, also mixtures of hydroxyethyl starches may be employed having different mean molecular weights and/or different molar substitution and/or different ratios of C₂ : C₆ substitution. Therefore, mixtures of hydroxyethyl starches may be employed having different mean molecular weights and different molar substitution and different ratios of C₂ : C₆ substitution, or having different mean molecular weights and different molar substitution and the same or about the same ratio of C₂ : C₆ substitution, or having different mean molecular weights and the same or about the same molar substitution and different ratios of C₂ : C₆ substitution, or having the same or about the same mean molecular weight and different molar substitution and different ratios of C₂ : C₆ substitution, or having different mean molecular weights and the same or about the same molar substitution and the same or about the same ratio of C₂ :C₆ substitution, or having the same or about the same mean molecular weights and different molar substitution and the same or about the same ratio of C₂ : C₆ substitution, or having the same or about the same mean molecular weight and the same or about the same molar substitution and different ratios of C₂ : C₆ substitution, or having about the same mean molecular weight and about the same molar substitution and about the same ratio of C₂ : C₆ substitution.

In different conjugates and/or different methods according to the present invention, different hydroxyalkyl starches, preferably different hydroxyethyl starches and/or different hydroxyalkyl starch mixtures, preferably different hydroxyethyl starch mixtures, may be employed.

In a preferred embodiment, the hydroxyalkyl starch or derivative thereof comprises 1 to 100, preferably 1 to 15, in particular 1 aldehyde group(s), keto group(s) and/or hemiacetal group(s) or wherein the hydroxyalkyl starch or derivative thereof comprises 1 to 100, preferably 1 to 15, in particular 1 alpha-SH-beta amino group(s).

In another preferred embodiment, the active substance comprises 1 to 15, preferably 1 to 8, in particular 1 aldehyde group(s), keto group(s) and/or hemiacetal group(s) or wherein the active substance comprises 1 to 15, preferably 1 to 8, in particular 1 alpha-SH-beta amino group(s).

The hydroxyalkyl starch, preferably hydroxyethyl starch, comprising the at least one aldehyde group, keto group, hemiacetal group or alpha-SH-beta amino group can be provided by every suitable method.

In a preferred embodiment the hydroxyalkyl starch derivative comprising said aldehyde group, keto group, hemiacetal group or said alpha-SH-beta amino group is obtained by a method comprising
(a)(2)reacting the hydroxyalkyl starch with at least one, at least bifunctional compound, said compound comprising two functional groups M₁ and Q, one functional group M₁ being reacted with the hydroxyalkyl starch and one functional group Q being
   (i) an aldehyde group, keto group, hemiacetal group or an alpha-SH-beta amino group; or
   (ii) a functional group being chemically modified to give the aldehyde group, keto group, hemiacetal group or an alpha-SH-beta amino group.

In one preferred embodiment of (a)(2) the functional group M₁ is selected from the group consisting of a carboxy group, a reactive carboxy group, carboxylic acid anhydride, carboxylic acid halogenide, isocyanate, isothiocyanate; chlorofomates, and epoxide groups and the functional group Q is an aldehyde group, keto group, hemiacetal group, alpha-SH-beta amino group, or a functional group being chemically modified to give an aldehyde group, a keto group, a hemiacetal group, or an alpha-SH-beta amino group.

According to one embodiment of the present invention, the method according to (a)(2) may comprise that the hydroxyalkyl starch is reacted via the optionally oxidized reducing end of the hydroxyalkyl starch with the at least one bifunctional compound comprising at least two functional groups M₁ and Q.

The term that the hydroxyalkyl starch, preferably hydroxyethyl starch is reacted "via the optionally oxidized reducing end" as used in the context of the present invention may relate to a process according to which the hydroxyalkyl starch is reacted predominantly via its optionally oxidized reducing end.

This term "predominantly via its optionally oxidized reducing end" relates to processes according to which statistically more than 50 %, preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of the hydroxyalkyl starch molecules employed for a given reaction are reacted via at least one optionally oxidized reducing end per hydroxyalkyl starch molecule, wherein a given hydroxalkyl starch molecule which is reacted via at least one reducing end can be reacted in the same given reaction via at least one further suitable functional group which is comprised in said hydroxyalkyl starch molecule and which is not a reducing end. If one or more hydroxyalkyl starch molecule(s) is (are) reacted via at least one reducing and simultaneously via at least one further suitable functional group which is comprised in this (these) hydroxyalkyl starch molecule(s) and which is not a reducing end, statistically preferably more than 50 %, preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of all reacted functional groups of these hydroxyalkyl starch molecules, said functional groups including the reducing ends, are reducing ends.

The term "reducing end" as used in the context of the present invention relates to the terminal aldehyde group of a hydroxyalkyl starch molecule which may be present as aldehyde group and/or as corresponding hemiacetal form. In case the reducing end is oxidized, the aldehyde or hemiacetal group is in the form of a carboxy group and/or of the corresponding lactone.

According to one embodiment of the present invention, the method according to (a)(2) may therefore comprise oxidizing hydroxyalkyl starch at its reducing end to give hydroxalkyl starch according to formula (IIIa) 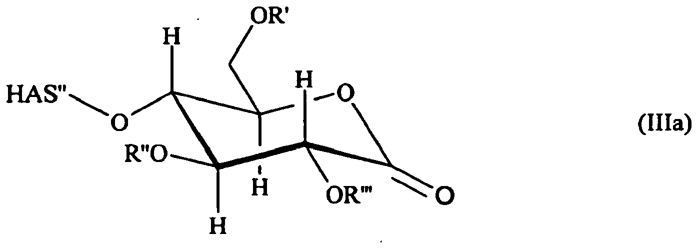 and/or according to formula (IIIb) 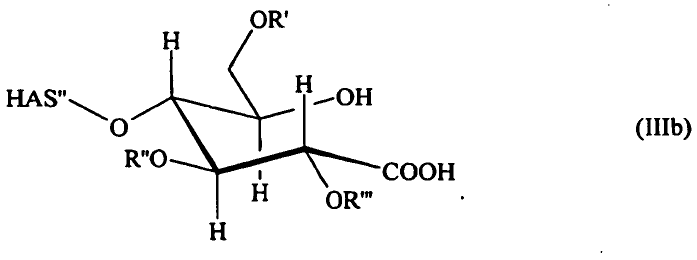 wherein R', R" and R''' are as defined for formula II, and reacting hydroxyalkyl starch oxidized at its reducing end with at least one suitable compound to give an aldehyde, keto, hemiacetal or alpha-SH-beta amino functionalized hydroxyalkyl starch.

Oxidation of the hydroxyalkyl starch, preferably hydroxyethyl starch, may be carried out according to each method or combination of methods which result in compounds having the above-mentioned structures (IIIa) and/or (IIIb). Although the oxidation may be carried out according to all suitable method or methods resulting in the oxidized reducing end of hydroxyalkyl starch, it is preferably carried out using an alkaline iodine solution as described, e.g., in DE 196 28 705 A1 (example A, column 9, lines 6 to 24)

According to (a)(2) it is preferred that the hydroxyalkyl starch, optionally oxidized at its reducing end, is reacted with an at least bifunctional compound comprising a functional group M₁ which is reacted with the hydroxyalkyl starch, preferably at the optionally oxidized reducing end, and a functional group Q which is an aldehyde group, a keto group, a hemiacetal group or an alpha-SH-beta amino group or a functional group which can be modified to give either of these groups.

As functional group M₁ of the at least bifunctional compound which is reacted with the hydroxyalkyl starch, especially a group is to be mentioned having the structure R*-NH-where R* is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogens such as F, Cl or Br may be mentioned. Especially preferred residues R* are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

According to another embodiment of the present invention, the functional group M₁ has the structure R*-NH-R**- where R**preferably comprises the structure unit -NH- and/or the structure unit -(C=G)- where G is O or S, and/or the structure unit -SO₂-. Specific examples for the functional group R** are 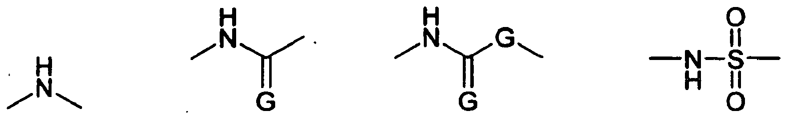 and 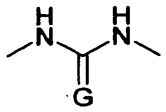 where, if G is present twice, it is independently O or S.

Therefore, the present invention also relates to a method and a conjugate as mentioned above wherein the functional group M₁ is selected from the group consisting of 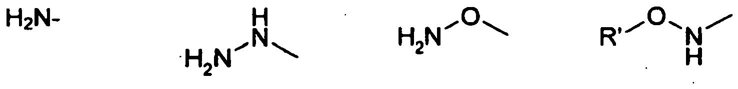 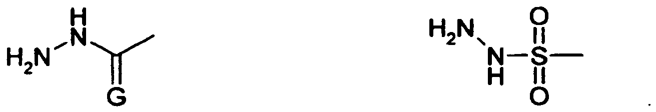 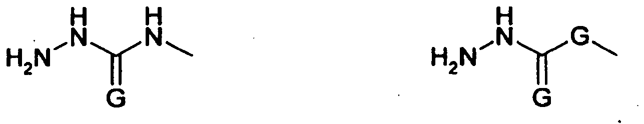 wherein G is O or S and, if present twice, independently O or S, and R' is methyl.

According to a particularly preferred embodiment of the present invention, the functional group M₁ is an amino group -NH₂.

With respect to the case when the group Q is an aldehyde group, a keto group, a hemiacetal group or an alpha-SH-beta amino group or a functional group being chemically modified to give one of these groups, the following functional groups are to be mentioned, among others:
- C-C-double bonds or C-C-triple bonds or aromatic C-C-bonds;
- the thio group or the hydroxy group;
- alkyl sulfonic acid hydrazide, aryl sulfonic acid hydrazide;
- 1,2-dioles;
- 1,2 amino-thioalcohols;
- azides;
- 1,2-aminoalcohols;
- the amino group -NH₂ or derivatives of the amino groups comprising the structure unit -NH- such as aminoalkyl groups, aminoaryl group, aminoaralkyl groups, or alkarlyaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
   -- -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
   wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C≡C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 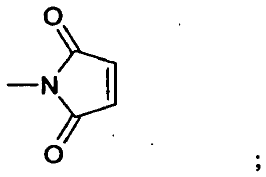
- the group

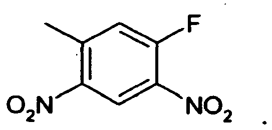

In a preferred embodiment of the method of this invention, the functional group M₁ is reacted with an OH-group on the hydroxyalkyl starch (or the optionally oxidized or non-oxidized reducing end of hydroxyalkyl starch). The functional group M₁ in this embodiment is preferably a carboxy group or a reactive carboxy group and the functional group Q is an aldehyde group, keto group or hemiacetal group, in particular the bifunctional compound comprising M₁ and Q is selected from the group consisting of formylbenzoic acid, 4-formylbenzoic acid pentafluorophenyl ester, 4-formylbenzoic acid N-hydroxysuccinimide ester, 4-(4-formyl-3,5-dimethoxyphenoxy)butyric acid, and 4-formylbenzoic acid anhydride, or a biocompatible compound selected from the group consisting of alpha-keto carboxylic acids, neuraminic acids or derivatives thereof and pyridoxal phosphate.

As regards alpha-keto carboxylic acids, those are preferably alpha-keto carboxylic acids derived from amino acids and can in most instances also be found in the human body. Preferred alpha-keto carboxylic acids derived from amino acids are selected from the group consisting of keto-valine, keto-leucine, keto-isoleucine and keto-alanine. In a preferred embodiment of the method of this invention, the carboxy group of the alpha-keto carboxylic acids is reacted with an OH-group on the hydroxyalkyl starch (or the optionally oxidized or non-oxidized reducing end of hydroxyalkyl starch) or is reacted with group Q of the hydroxyalkyl starch being an amino group. The remaining free keto group of the alpha-keto carboxylic acid may then be reacted to form the thiazolidine.

Accordingly, the present invention relates to a method according to (a)(2)(i) or (a)(2)(ii), wherein the hydroxyalkyl starch is reacted with an alpha-keto carboxylic acid.

As regards neuraminic or sialic acids or derivatives thereof those are preferably biocompatible, in particular they are sugars found in the human body, which are N- and/or O-acetylated. In a preferred embodiment, the neuramic acids or sialic acids are N-acetyl neuramic acids. These compounds show a desired rigidity because of the pyranose structure in order to fulfill the function as a spacer. On the other hand, it may be possible to introduce an aldeyhde group into these compounds through selective oxidation. Sialic acids are found in the human body e.g. as terminal monosaccharides in glycan chains of glycosylated proteins.

In a preferred embodiment, the sialic acid may be selectively oxidized to an aldehyde group.

Methods to selectively oxidize sialic acids or neuramic acids are known in the art, e.g. from L.W. Jaques, B.F. Riesco, W. Weltner, Carbohydrate Research, 83 (1980), 21 - 32 and T. Masuda, S. Shibuya, M. Arai, S. Yoshida, T. Tomozawa, A. Ohno, M. Yamashita, T. Honda, Bioorganic & Medicinal Chemistry Letters, 13 (2003), 669 - 673. Preferably the oxidation of the sialic acid may be conducted prior to the reaction with hydroxyalkyl starch.

The optionally oxidized sialic acid may then be reacted via its carboxylic acid group with hydroxyalkyl starch. In a preferred embodiment of the method of this invention, the carboxy group of the oxidized sialic acid is reacted with an OH-group on the hydroxyalkyl starch or the optionally oxidized or non-oxidized reducing end of hydroxyalkyl starch or is reacted with group Q of the hydroxyalkyl starch being an amino group. The remaining carbonyl group of the oxidized sialic acid may then be reacted to form the thiazolidine.

Accordingly, the present invention relates to a method according to (a)(2)(i) or (a)(2)(ii), wherein the hydroxyalkyl starch is reacted with an oxidized sialic acid.

As regards pyridoxal phosphate (PyP), this is a highly biocompatible bifunctional compound and is also called vitamine B6. PyP is a co-enzyme which participates in transaminations, decarboxylations, racemizations, and numerous modifications of amino acid side chains. All PyP requiring enzymes act via the formation of a Schiff's base between the amino acid and the co-enzyme.

In a preferred embodiment of the method of this invention, the phosphate group of the PyP is either reacted with an OH-group on the hydroxyalkyl starch or the optionally oxidized or non-oxidized reducing end of hydroxyalkyl starch forming a phosphate group or is reacted with group Q of the hydroxyalkyl starch being an amino group forming a phosphoramide. The remaining carbonyl group of PyP may then be reacted to form the thiazolidine.

In case of PyP, the functional group of the hydroxyalkyl starch is preferably introduced into the hydroxyalkyl starch by use of a diamino compound as described above.

Accordingly, the present invention relates to a method according to (a)(2)(i) or (a)(2)(ii), wherein the hydroxyalkyl starch is reacted with pyridoxal phosphate.

In a preferred embodiment in (a)(2)(i), the functional group M₁ is selected from the group consisting of a carboxy group, a reactive carboxy group, carboxylic acid anhydride, carboxylic acid halogenide, isocyanate, isothiocyanate, chloroformic acid ester, and epoxide groups and the functional group Q is an aldehyde group, keto group or hemiacetal group, wherein the functional group M₁ is reacted with OH-groups on the hydroxyalkyl starch.

In a preferred embodiment in (a)(2)(i), the functional group M₁ is selected from the group consisting of an amino group and alpha-SH-beta-amino group and the functional group Q is an alpha-SH-beta amino group, in particular wherein the functional group M₁ is reacted with the optionally oxidized reducing end of the hydroxyalkyl starch.

In a preferred embodiment in (a)(2)(i), the hydroxyalkyl starch comprising said alpha-SH-beta-amino group is obtained by a method comprising reacting hydroxyalkyl starch at the optionally oxidized reducing end with a compound comprising a functional group M₁ and a functional group Q being an alpha-SH-beta-amino group, in particular wherein the compound comprising M₁ and the alpha-SH-beta-amino group is 1,3-diamino-2-thio propane, or 2,3-diamino-1-thio propane.

In a preferred embodiment in (a)(2)(ii) the at least bifunctional compound comprises M₁ being a carboxy group or a reactive carboxy group and Q being a protected alpha-SH-beta amino group, in particular wherein the at least bifunctional compound is selected from the group consisting of D-, L-PG₁-Cys(PG₂)-OH, or a racemic mixture thereof, and their active ester, wherein PG₁ may be any suitable protecting group for an amino group, preferably selected from the group consisting of tert-butyloxycarbonyl (Boc) or 9-fluorenylmethoxycarbonyl (Fmoc), and PG₂ may be any suitable protecting group for a thiol group, preferably selected from the group consisting of trityl (Trt), p-methoxytrityl (Mmt) S-*tert*-butylthio (S-t-Bu), and acetamidomethyl (Acm).

According to (a)(2)(ii) the functional group Q is a group which is further modified to give an aldehyde group, a keto group, a hemiacetal group or an alpha-SH-beta amino group. According to this embodiment, the hydroxyalkyl starch derivative resulting from the reaction of hydroxyalkyl starch, optionally oxidized at its reducing end, with the at least bifunctional compound, is reacted with a further at least bifunctional compound comprising a functional group which is reacted with the functional group Q of the hydroxyalkyl starch derivative.

In the preferred case of (a)(2)(ii) both M₁ and Q are an amino group -NH₂, M₁ and Q may be separated by any suitable spacer. Among others, the spacer may be an optionally substituted, linear, branched and/or cyclic hydrocarbon residue. Generally, the hydrocarbon residue has from 1 to 40, preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an alkyl chain of from 1 to 20, preferably from 2 to 10, more preferably from 2 to 6, and especially preferably from 2 to 4 carbon atoms.

In one embodiment of the method of the invention according to (a)(2)(ii), it is preferred that the at least bifunctional compound comprising M₁ and Q is an optionally substituted diaminoalkane having from 1 to 20 carbon atoms, preferably a compound being selected from the group consisting of 1,2-diaminoethane, 1,3-diaminopropane, and 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diaminohexadecane, 1,17-diaminoheptadecane, 1,18-diaminooctadecane, 1,19-diaminononadecane, and 1,20-diaminoeicosane or a compound having the formula

H₂N-[(CR^{1'}R^{2'})ₚO]_{q}[CR^{3'}R^{4'}]ᵣ-NH₂

wherein R^{1'}, R^{2'}, R^{3'}, and R^{4'} are independently selected from the group, consisting of hydrogen, and alkyl group, preferably hydrogen and methyl group, p is 2 to 4, wherein the residues R^{1'} and R^{2'} may be the same or different in the p groups CR^{1'}R^{2'}, q is 0 to 20, preferably 0 to 10; r is 0 to 20, preferably 2 to 4, wherein in the case of q = 0, r is not 0, and wherein the residues R^{3'} and R^{4'} may be the same or different in the r groups CR^{3'}R^{4'}.

A preferred embodiment of the present invention also relates to a method as described above, wherein the hydroxyalkyl starch is reacted with a bifunctional compound that may be selected from the group consisting of 1,2-diaminoethane, 1,3-diaminopropane, and 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diaminohexadecane, 1,17-diaminoheptadecane, 1,18-diaminooctadecane, 1,19-diaminononadecane, and 1,20-diaminoeicosane or a compound having the formula

H₂N-[(CR^{1'}R^{2'})ₚO]_{q}[CR^{3'}R^{4'}]ᵣ-NH₂

wherein R^{1'}, R^{2'}, R^{3'}, and R^{4'} are independently selected from the group, consisting of hydrogen, and alkyl group, preferably hydrogen and methyl group, p is 2 to 4, wherein the residues R^{1'} and R^{2'} may be the same or different in the p groups CR^{1'}R^{2'}, q is 0 to 20, preferably 0 to 4, and r is 0 to 20, preferably 2 to 4, wherein in the case of q = 0, r is not 0, and wherein the residues R^{3'} and R^{4'} may be the same or different in the r groups CR^{3'}R^{4'}, in particular 1,4-diaminobutane, to give an amino functionalized hydroxyalkyl starch derivative and this amino functionalized hydroxyalkyl starch derivative is further reacted with an at least bifunctional compound containing one functional group reactive with the amino group of the amino functionalized hydroxyalkyl starch and one functional group being at least one aldehyde group, keto group, hemiacetal group or alpha-SH-beta amino group. This further bifunctional compound is preferably selected from the group consisting of formylbenzoic acid, 4-formylbenzoic acid, pentafluorophenyl ester, 4-formylbenzoic acid N-hydroxysuccinimide ester, 4-(4-formyl-3,5-dimethoxyphenoxy)butyric acid, and 4-formylbenzoic acid anhydride or a biocompatible compound selected from the group consisting of alpha-keto carboxylic acids, neuraminic acids or derivatives thereof and pyridoxal phosphate.

In another embodiment of the method in (a)(2)(ii), the hydroxyalkyl starch comprising said alpha-SH-beta-amino group is obtained by a method comprising optionally oxidizing hydroxyalkyl starch at its reducing end, reacting the oxidized or non-oxidized reducing end with a functional group M₁ of a compound comprising, in addition to M₁, a further functional group Q, to give a first hydroxyalkyl starch derivative, and reacting the functional group Q of the first hydroxyalkyl starch derivative with a functional group V of a compound comprising, in addition to V, an optionally protected alpha-SH-beta-amino group, to give the optionally protected alpha-SH-beta-amino functionalized hydroxyalkyl starch derivative. The functional groups M₁ and Q are preferably as described above.

In a preferred embodiment the compound comprising V and the optionally protected alpha-SH-beta-amino group is cysteine or a derivative thereof, V being a carboxy group or a reactive carboxy group, preferably a reactive ester or a carboxylic acid anhydride.

Preferably, in (a)(2)(ii), hydroxyalkyl starch in its non-oxidized form is reacted with a bifunctional compound having an amino group M₁ and an amino group Q by reductive amination. In particular, the bifunctional compound may be selected from the group consisting of primary amines, ammonia, 1,2-diaminoethane, 1,3-diaminopropane, and 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diaminohexadecane, 1,17-diaminoheptadecane, 1,18-diaminooctadecane, 1,19-diaminononadecane, and 1,20-diaminoeicosane or a compound having the formula 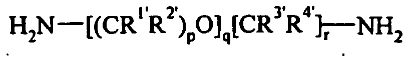 wherein R^{1'}, R^{2'}, R^{3'}, and R^{4'} are independently selected from the group, consisting of hydrogen, and alkyl group, preferably hydrogen and methyl group, p is 2 to 4, wherein the residues R^{1'} and R^{2'} may be the same or different in the p groups CR^{1'}R^{2'}, q is 0 to 20, preferably 0 to 4, and r is 0 to 20, preferably 2 to 4, wherein in the case of q = 0, r is not 0, and wherein the residues R^{3'} and R^{4'} may be the same or different in the r groups CR^{3'}R^{4'}, in particular 1,4 diaminobutane.

Preferably, in (a)(2)(ii), the hydroxyalkyl starch oxidized at its reducing end is reacted with a bifunctional compound having an amino group M₁ and an amino group Q by a lactone ring-opening reaction. In particular, the bifuntional compound may be selected from the group consisting of 1,2-diaminoethane, 1,3-diaminopropane, and 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diarninohexadecane, 1,17-diaminoheptadecane, 1,18-diaminooctadecane, 1,19-diaminononadecane, and 1,20-diaminoeicosane or a compound having the formula 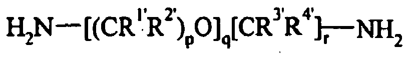 wherein R^{1'}, R^{2'}, R^{3'}, and R^{4'} are independently selected from the group, consisting of hydrogen, and alkyl group, preferably hydrogen and methyl group, p is 2 to 4, wherein the residues R^{1'} and R^{2'} may be the same or different in the p groups CR^{1'}R^{2'}, q is 0 to 20, preferably 0 to 4, and r is 0 to 20, preferably 2 to 4, wherein in the case of q = 0, r is not 0, and wherein the residues R^{3'} and R^{4'} may be the same or different in the r groups CR^{3'}R^{4'}, in particular 1,4 diaminobutane.

Preferably, in (a)(2)(ii), hydroxyalkyl starch is first reacted with a bifunctional compound having an amino group Q, preferably prepared as described above, and the obtained amino functionalized hydroxyalkyl starch is further reacted with a bifunctional compound having an activated carboxylic group and an aldehyde group, keto group or hemiacetal group.

In a particularly preferred embodiment, the method of the invention in (a)(2)(ii) comprises reacting the preferably oxidized hydroxyalkyl starch with a compound having an amino group M₁ and an amino group Q, in particular a diaminoalkyl compound, especially 1,4-diaminobutan, and then reacting the amino functionalized hydroxyalkyl starch with a compound selected from the group consisting of optionally protected cysteine and 4-formyl benzoic acid.

The active substance comprising the at least one aldehyde group, keto group, hemiacetal group or alpha-SH-beta amino group can be provided by every suitable method.

In a preferred embodiment the active substance, preferably an optionally modified protein, peptide, synthetic peptide or oligonucleotide, comprising said aldehyde group, keto group, hemiacetal group or said alpha-SH-beta-amino group is obtained by a method comprising
(b)(1)introducing at least one aldehyde group, keto group, hemiacetal group or at least one alpha-SH-beta-amino group into the active substance during its preparation or by chemical modification, or
(b)(2)reacting the active substance with an at least bifunctional compound, said compound comprising two functional groups M₂ and Q, one functional group M₂ being reacted with the active substance and one functional group Q being
   (i) an aldehyde group, keto group, hemiacetal group or an alpha-SH-beta amino group; or
   (ii) a functional group being chemically modified to give the aldehyde group, keto group, hemiacetal group or an alpha-SH-beta amino group.

Preferably in (b)(1), the active substance is a protein or peptide which was prepared by organic synthesis, preferably produced using a synthesis resin, allowing for an aldehyde functionalized, keto functionalized, hemiacetal functionalized or alpha-SH-beta-amino functionalized protein or peptide, or wherein in (b)(1), the active substance is a protein or peptide which was produced using an expression vector leading to an aldehyde functionalized, keto functionalized, hemiacetal functionalized or alpha-SH-beta-amino functionalized protein or peptide, or wherein in (b)(1), the active substance is a protein or a peptide and the backbone of the protein or peptide is substituted with an aldehyde group, keto group, hemiacetal group or alpha-SH-beta-amino group, or wherein in (b)(1), the active substance is a protein or a peptide where said aldehyde group, keto group, hemiacetal group or said alpha-SH-beta-amino group is linked directly to the backbone of the protein or peptide or is part of a side-chain of the backbone.

In a preferred embodiment (b)(1), the active substance is obtained by modification of the active substance, in particular of a protein or a peptide by oxidation in order to introduce an aldehyde group.

In a preferred embodiment the active substance is a protein or peptide and the aldehyde group, keto group or hemiacetal group is comprised in a carbohydrate moiety of the polypeptide, in particular wherein the carbohydrate moiety is selected from the group consisting of hydroxyaldehydes, hydroxyketones and chemical modifications thereof. The carbohydrate moiety may be a derivative of a naturally occurring carbohydrate moiety and is selected from the group consisting of glucose, galactose, mannose, and sialic acid, which are optionally chemically or enzymatically oxidized, preferably an oxidized galactose or an oxidized sialic acid residue of a carbohydrate side chain, more preferably the terminal galactose or sialic acid residue of a carbohydrate side chain, the oxidation of a terminal carbohydrate moiety preferably being performed either enzymatically or chemically, a chemical oxidation preferably carried out using a periodate.

In a preferred embodiment the carbohydrate moiety is a derivative of a naturally occurring carbohydrate moiety and is a terminal galactose, which is enzymatically or chemically oxidized, wherein the terminal galactose residue is optionally obtained after cleavage of a terminal sialic acid.

In a preferred embodiment the alpha-SH-beta-amino group is comprised in a cysteine residue of the active substance, preferably a protein or peptide, the cysteine residue preferably being a N-terminal cysteine residue of the active substance.

In a preferred embodiment the active substance is a modified protein or peptide with an N-terminal cysteine residue, which is not part of a disulfide bridge, in particular wherein the modified protein or peptide possessing an N-terminal cysteine residue is a mutant of a naturally occurring protein or peptide, obtained by (1) adding a cysteine residue to the N-terminal amino acid, (2) substituting the N-terminal amino acid with cysteine, or by (3) deleting the N-terminal amino acid(s) until a terminal cysteine is obtained.

In peptides, the mentioned cysteine can be introduced during synthesis. Peptide synthesis is known in the art. (W. Chang, P. D. White ; Fmoc solid phase peptide synthesis, a practical approach; Oxford University Press, Oxford, 2000, ISBN 0199637245).

Recombinant polypeptides are obtainable by way of standard molecular biological techniques as; for example, described in Molecular Cloning: A Laboratory Manual, 3rd edition, Eds. Sambrook et al., CSHL Press 2001. Briefly, polypeptides can be expressed from recombinant expression vectors comprising a nucleic acid encoding the desired polypeptide, which nucleic acid is operably linked to at least one regulator sequence allowing expression of the desired polypeptide. For example, a nucleic acid sequence encoding the desired polypeptide can be isolated and cloned into an expression vector and the vector can then be transformed into a suitable host cell for expression of the desired polypeptide. Such a vector can be a plasmid, a phagemid or a cosmid. For example, a nucleic acid molecule can be cloned in a suitable fashion into prokaryotic or eukaryotic expression vectors (*Molecular Cloning*, see above). Such expression vectors comprise at least one promoter and can also comprise a signal for translation initiation and - in the case of prokaryotic expression vectors - a signal for translation termination, while in the case of eukaryotic expression vectors preferably expression signals for transcriptional termination and for polyadenylation are comprised. Examples for prokaryotic expression vectors are, for expression in Escherichia coli e.g. expression vectors based on promoters recognized by T7 RNA polymerase as described in US 4,952,496, for eukaryotic expression vectors for expression in Saccharomyces cerevisiae, e.g. the vectors G426/Met25 or P526/Gal1 (Mumberg et al., (1994) Nucl. Acids Res., 22, 5767-5768), for the expression in insect cells, e.g. Baculovirus vectors, as e.g described in EP-B1-0127839 or EP-B1-0549721 or by Ciccarone et al. ("Generation of recombinant Baculovirus DNA in E.coli using baculovirus shuttle vector" (1997) Volume 13, U. Reischt, ed. (Totowa, NJ: Humana Press Inc.) and for the expression in mammalian cells, e.g. the vectors Rc/CMW and Rc/ASW and SW40-Vectors, which are commonly known and commercially available, or the EBNA-system described in Example 4, the Sindbis replicon-based pCytTS (Boorsma et al. (2002) Biotechnol. Bioeng. 79(6): 602-609), the Sindbis virus expression system (Schlesinger (1993) Trends Biotechnol. 11(1):18-22) or an Adenovirus expression system (He et al. (1998) Proc. Natl. Acad. Sci. USA 95:2509-2514). The molecular biological methods for the production of these expression vectors as well as the methods for transfecting host cells and culturing such transfected host cells as well as the conditions for producing and obtaining the polypeptides of the invention from said transformed host cells are well known to the skilled person.

Polypeptides with the desired N-terminal cysteine residue can be generated from the polypeptides expressed and purified as described above by cloning the polypeptide of interest behind an N-terminal leader sequence which is removable to yield the polypeptides with the desired N-terminal cysteine residue.

This can be achieved, for example, by proteolytic cleavage of the polypeptide expressed and purified as described above. In such a case, a fusion polypeptide was cloned, expressed and purified wherein a cysteine residue follows directly a highly selective protease cleavage site, for example a Cys residue immediately following the Factor Xa cleavage site: Ile (Glu/Asp) Gly Arg| (Cys/His), or a His or Cys residue immediately following the Enterokinase cleavage site: Asp Asp Asp Asp Lys| (Cys/His), wherein | denotes the site of cleavage by the protease.

This can further be achieved, for example, by cleavage of the polypeptide during expression, for example cleavage at the stage of ER translocation by the signal peptidase. In such a case, a fusion polypeptide was cloned and expressed wherein a Cys residue follows directly the signal peptide directing the recombinant polypeptide to the secretory pathway (for review see Rapoport et al., Annu Rev Biochem. 1996;65:271-303).

The molecular biological methods to manipulate the coding sequence of a recombinant polypeptide so that a coding sequence for a polypeptide with the desired Cys residue at the desired position is generated are well known in the art (Sambrook, above).

The preferred embodiments for (b)(2) are as described for (a)(2) above, in particular functional groups M₂ and Q are preferably as described above for functional groups M₁ and Q, as long as the active substance contains a functional group which is reactive with the group M₁ as described above.

In a particularly preferred embodiment the active substance is selected from a protein and peptide and is obtained according to (b)(1) above, preferably as described above.

In a particular preferred embodiment according to (A), hydroxyalkyl starch comprising an aldehyde group is obtained by (a)(2)(ii), preferably by reacting the preferably oxidized hydroxyalkyl starch with a compound having an amino group M₁ and an amino group Q, in particular a diaminoalkyl compound, especially 1,4-diaminobutan, and then reacting the amino functionalized hydroxyalkyl starch with a bifunctional compound comprising an aldehyde group, in particular 4-formyl benzoic acid, and then reacting the aldehyde group of the hydroxyalkyl starch with an optionally protected alpha-SH-beta-amino group of an active substance obtained by (b)(2)(i), preferably by introducing an optionally protected cysteine into the active substance, in particular a protein or a peptide.

In a particular preferred embodiment according to (B), the active substance comprising an aldehyde group is obtained by (b)(1), in particular by introducing an aldehyde group by chemical modification, preferably by oxidizing a protein or peptide, and then reacting the aldehyde group of the active substance with hydroxyalkyl starch comprising an alpha-SH-beta-amino group obtained by (a)(2)(ii), preferably by reacting the preferably oxidized hydroxyalkyl starch with a compound having an amino group M₁ and an amino group Q, in particular a diaminoalkyl compound, especially 1,4-diaminobutan, and then reacting the amino functionalized hydroxyalkyl starch with a bifunctional compound comprising an alpha-SH-beta-amino group, in particular cysteine.

The reaction of the invention according to (A) and (B) of the active substance with the hydroxyalkyl starch may be carried out in any suitable solvent or mixture of at least two solvents and at a suitable pH and at a suitable reaction temperature.

In a preferred embodiment, the reaction (A) or (B) is carried out at a temperature of 0 to 40 °C, preferably 0 to 25 °C, in particular 20 to 25 °C, in the presence of a solvent, and at a pH of 3.5 to 10, preferably 4 to 8, in particular 4.8 to 8.0 with a reaction time of preferably 0.1 to 24 h, in particular about 21 h.

The solvent is preferably selected from the group consisting of water, aqueous buffer, DMF (dimethylformamide), DMSO (dimethylsulfoxide), DMA (dimethylacetamide) and mixtures thereof.

The molecular ratio of hydroxalkyl starch to active substance is about 1:1 to 200:1, preferably 10:1 to 100:1, in particular 40:1 to 70:1.

Furthermore, the invention relates to a conjugate of an active substance and hydroxyalkyl starch, as obtainable by a method as described above.

In another embodiment, the invention relates to the conjugate of an active substance and hydroxyalkyl starch, wherein the active substance and the hydroxyalkyl starch are covalently linked by a chemical residue having a structure according to formula (I) 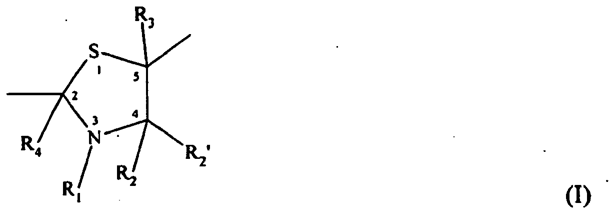 or formula (I') 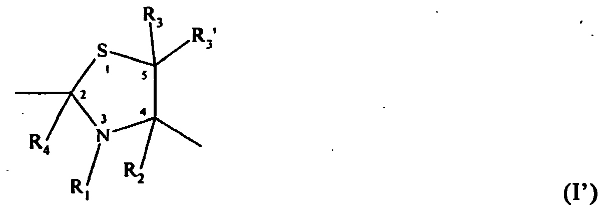 or formula (I'') 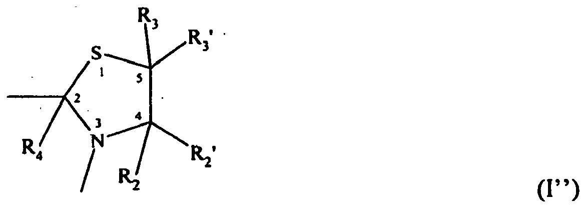 wherein R₁, R₂, R₂, R₃, R₃, and R₄ are independently selected from the group consisting of hydrogen, an optionally suitably substituted, linear, cyclic and/or branched alkyl, aryl, heteroaryl, aralkyl, and heteroaralkyl group, preferably hydrogen, said conjugate having a structure according to formula (IV), (IV'), or (IV") 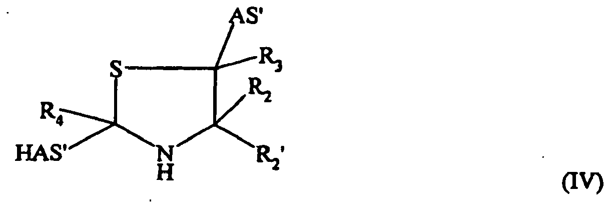 or 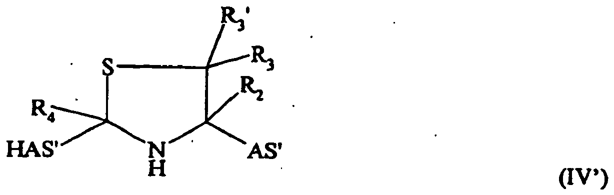 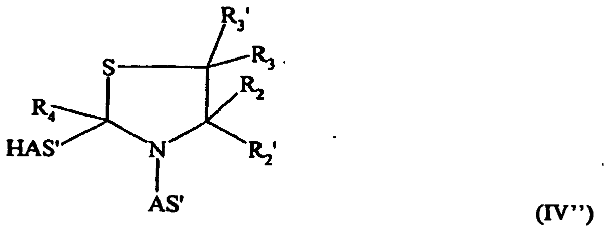 wherein HAS' is the residue of the hydroxyalkyl starch derivative which was linked to an aldehyde group, keto group or hemiacetal group by reacting hydroxyalkyl starch with at least one, at least bifunctional compound, said compound comprising two functional groups M₁ and Q, one functional group M₁ being reacted with the hydroxyalkyl starch and one functional group Q being
(i) an aldehyde group, keto group, hemiacetal group; or
(ii) a functional group being chemically modified to give the aldehyde group, keto group, hemiacetal group, and wherein AS' is the residue of the active substance or a derivative thereof which was linked to the alpha-SH-beta-amino group,
   or a structure according to formula (V), (V'), or (V"')

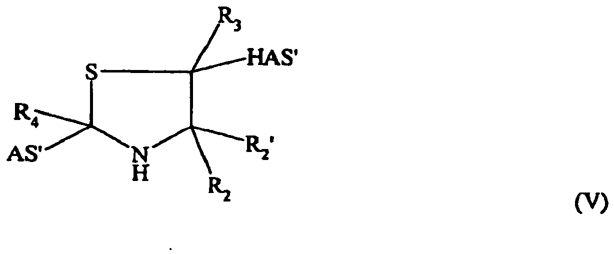

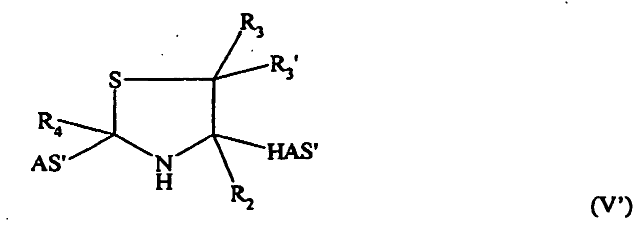

or 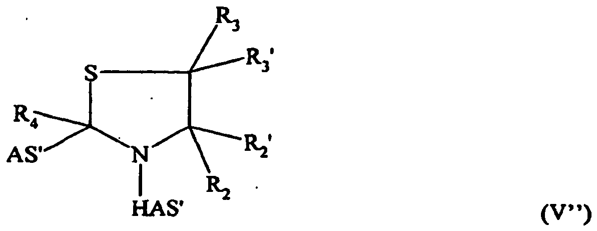
wherein HAS' is the residue of the hydroxyalkll starch derivative which was linked to the alpha-SH-beta-amino group 38a and wherein AS' is the residue of the active substance or a derivative thereof which was linked to the aldehyde group, keto group or hemiacetal group.

In a preferred embodiment, the conjugate is selected from the group consisting of 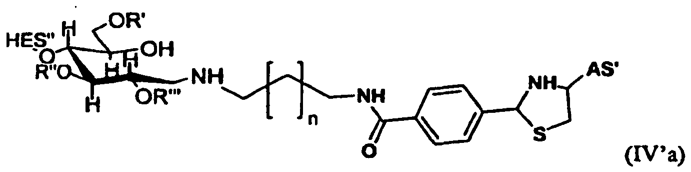 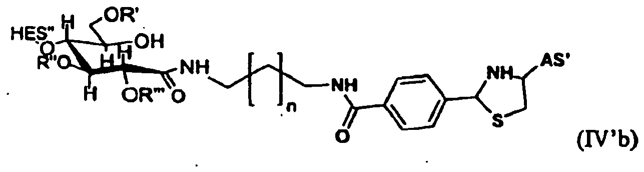 by a method comprising reacting the hydroxyalkyl starch with at least one, at least bifunctional compound, said compound comprising two functional groups M₁ and Q, one functional group M₁ being reacted with the hydroxyalkyl starch and one functional group Q being
(i) an alpha-SH-beta amino group; or
(ii) a functional group being chemically modified to give the alpha-SH-beta amino group, 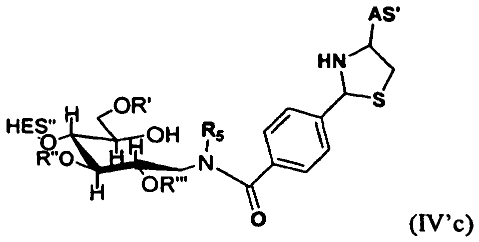 wherein R₅ is as defined for R₁ to R₄ above, 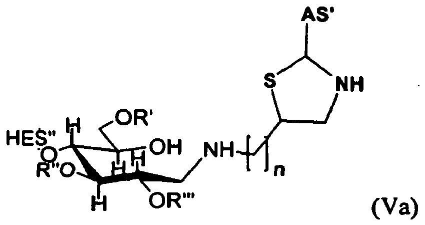 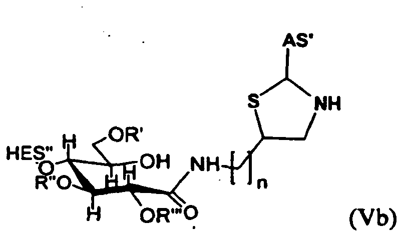 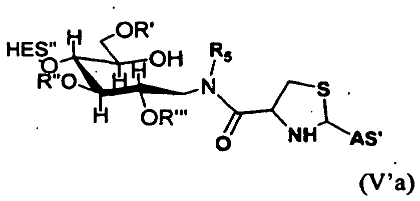 wherein R₅ is as defined for R₁ to R₄ above, 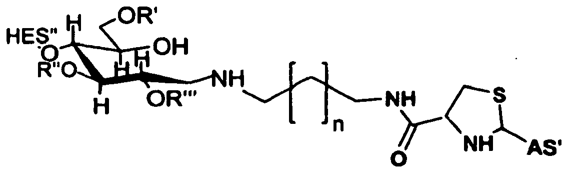 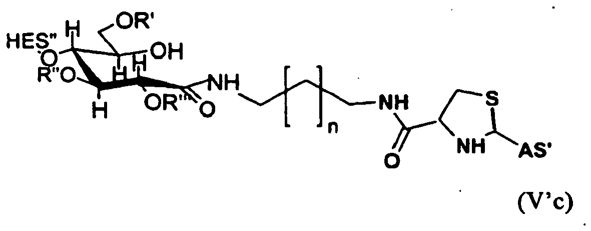 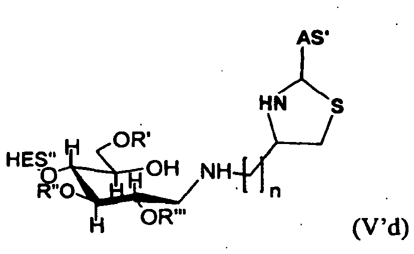 and 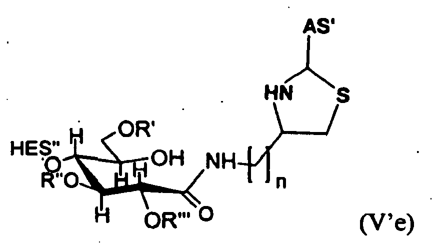
wherein in formula IV'a, IV'b, V'b, or V'c n is an integer, preferably n is 0 to 20, and where in formula Va, Vb, V'd, or V'e n is an integer, preferably n is 1 to 20. In a preferred embodiment of formula IV'b and of V'c, n is 2 to 4, in particular 2.

In a preferred embodiment the conjugate is 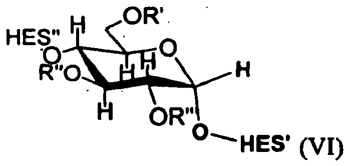 wherein R', R" and/or R"' are as defined for formula II and wherein, in at least one glucose unit of HES, at least one of R', R" and/or R"' is independently selected from the group consisting of 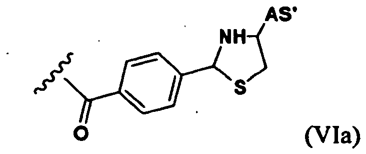 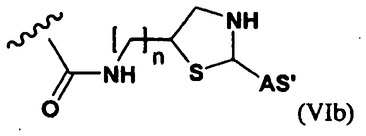 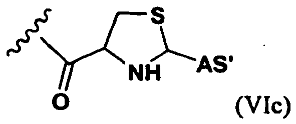 and 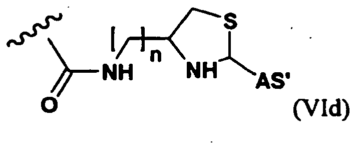 and wherein n is an integer, preferably 1 to 20 and/or wherein at least one of R', R*" and/or R"' is -(CH₂CH₂O)ₘ-R^{#}, wherein m is an integer, preferably 1 to 3, and R^{#} is selected from the group consisting of formula (VIa), (VIb), (VIc) and (VId).

In a further embodiment, the invention relates to an alpha-SH-beta amino functionalized hydroxyalkyl starch derivative selected from the group consisting of 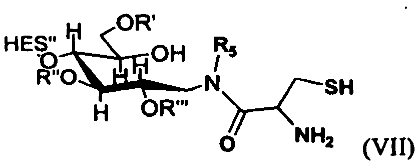 wherein R₅ is as defined for R₁ to R₄ above,
and 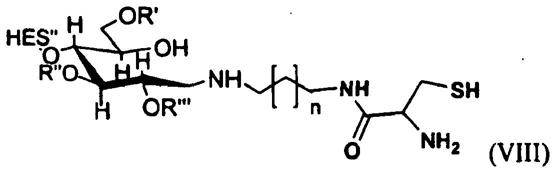 wherein n is an integer, preferably 0 to 20,
or the group consisting of 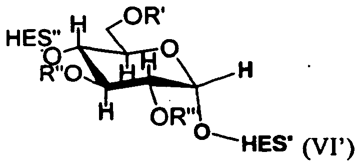 wherein R', R" and/or R"' are as defined for formula II and wherein in at least one glucose unit of HES, at least one of R', R" and/or R'" is independently selected from the group consisting of 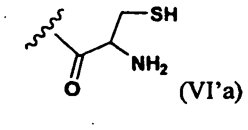 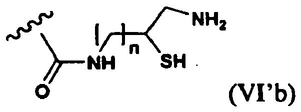 and 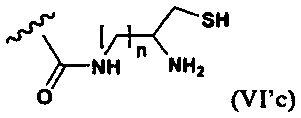 and wherein n is an integer, preferably 1 to 20 and/or wherein at least one of R', R" and/or R"' is -(CH₂CH₂O)ₘ-R^{##}, wherein m is an integer, preferably 1 to 3, and R^{##} is selected from the group consisting of formula (VI'a), (VI'b), and (VI'c).

The abbreviations HES" and HES' specifically used in the context of formulae (VI) and (VI') refer to residues of the HES molecule which, together with the carbohydrate moiety linking HES" and HES' as shown in (VI) and (VI'), constitute the HES molecule which in turn is part of the conjugates as defined in (VI) and (VI').

In a further embodiment, the invention relates to a conjugate as described above for use in a method for the treatment of the human or animal body or as a therapeutic agent.

The conjugates according to the invention may be at least 50% pure, even more preferred at least 70% pure, even more preferred at least 90%, in particular at least 95% or at least 99% pure. In a most preferred embodiment, the conjugates may be 100% pure, i.e. there are no other by-products present.

Therefore, according to another aspect, the present invention also relates to a composition which may comprise the conjugate(s) of the invention, wherein the amount of the conjugate(s) may be at least 50 wt-%, even more preferred at least 70 wt-%, even more preferred at least 90 wt-%, in particular at least 95 wt.-% or at least 99 wt.-%. In a most preferred embodiment, the composition may consist of the conjugate(s), i.e. the amount of the conjugate(s) is 100 wt.-%.

Accordingly, the present invention also relates to a pharmaceutical composition comprising a conjugate as described above or a conjugate, obtainable by a method as described above.

Moreover, the present invention also relates to a pharmaceutical composition comprising a conjugate as described above or a conjugate, obtainable by a method as described above, said pharmaceutical composition further comprising at least one pharmaceutically acceptable diluent, adjuvant, or carrier.

### Short description of the Figures

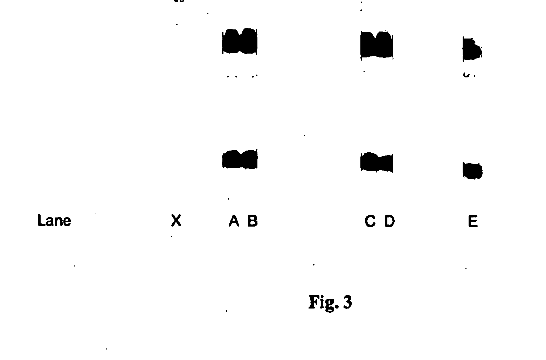

**Figure 1** shows an analysis of the crude protein-HES conjugates by SDS gel electrophoresis obtained by the thiazolidine formation from H-Cys(H)-HES10/0.4 and oxidized EPO.
   Lane X: Roti®-Mark STANDARD (Carl Roth GmbH + Co. KG, Karlsruhe, D) Molecular weight marker from top to bottom: 200 kDa, 119 kDa, 66 kDa, 43 kDa, 29 kDa, 20 kDa, 14.3 kDa
   Lane A: Conjugation of H=Cys(H)-HES10/0.4 to oxidized EPO
**Figure 2** shows an analysis of the crude protein-HES conjugates by SDS gel electrophoresis of thiazolidine formation from a H-Cys-Peptide-NH₂ and AldehydoHES.
   Lane X: Roti®-Mark STANDARD (Carl Roth GmbH + Co. KG, Karlsruhe, D) Molecular weight marker from top to bottom: 200 kDa, 119 kDa, 66 kDa, 43 kDa, 29 kDa, 20 kDa, 14.3 kDa.
   Lane A: Conjugation of AldehydoHES 10/0.7 at pH 4.6
   Lane B: Conjugation of AldehydoHES50/0.7 at pH 4.6
   Lane C: Reaction control: Peptide at pH 4.6
   Lane D: Conjugation of AldehydoHES 10/0.7 at pH 8.0
   Lane E: Conjugation of AldehydoHES50/0.7 at pH 8.0
   Lane F: Reaction control: Peptide at pH 8.0
**Figure 3** shows an analysis of the crude DNA-HES conjugates by agarose gel electrophoresis obtained by the thiazolidine formation from H-Cys(H)-HES50/0.7 and aldehyde-modified DNA as described in experimental sections 8.4 - 8.5 hereinunder.
   Lane X: pUC19/Msp I marker (Carl Roth GmbH + Co. KG, Karlsruhe, D). Molecular weight marker from top to bottom: 501/489 bp, 404 bp, 331 bp, 242 bp, 190 bp, 147 bp, 111/110 bp, 67 bp.
   - Lane A:: top row: Conjugation of H-Cys(H)-HES50/0.7 to FBA-modified DNA at pH 4.6 as described in experimental section 8.5;
   - Lane B:: top row: Reaction control; Conjugation of Oxo-HES50/0.7 to FBA-modified DNA at pH 4.6 as described in experimental section 8.5;
   - Lane C:: top row: Conjugation of H-Cys(H)-HES50/0.7 to FBA-modified DNA at pH 8.0 as described in experimental section 8.5;
   - Lane D:: top row: Reaction control; Conjugation of Oxo-HES50/0.7 to FBA-modified DNA at pH 8.0 as described in experimental section 8.5;
   - Lane E:: top row: Reaction control; FBA-modified DNA in water without HES-derivative;
   - Lane A:: bottom row: Conjugation of H-Cys(H)-HES50/0.7 to Formylindole-modified DNA at pH 4.6 as described in experimental section 8.5;
   - Lane B:: bottom row: Reaction control; Conjugation of Oxo-HES50/0.7 to Formylindole-modified DNA at pH 4.6 as described in experimental section 8.5;
   - Lane C:: bottom row: Conjugation of H-Cys(H)-HES50/0.7 to Formylindole-modified DNA at pH 8.0 as described in experimental section 8.5;
   - Lane D:: bottom row: Reaction control; Conjugation of Oxo-HES50/0.7 to Formylindole-modified DNA at pH 8.0 as described in experimental section 8.5;
   - Lane E:: bottom row: Reaction control; Formylindole-modified DNA in water without HES-derivative.

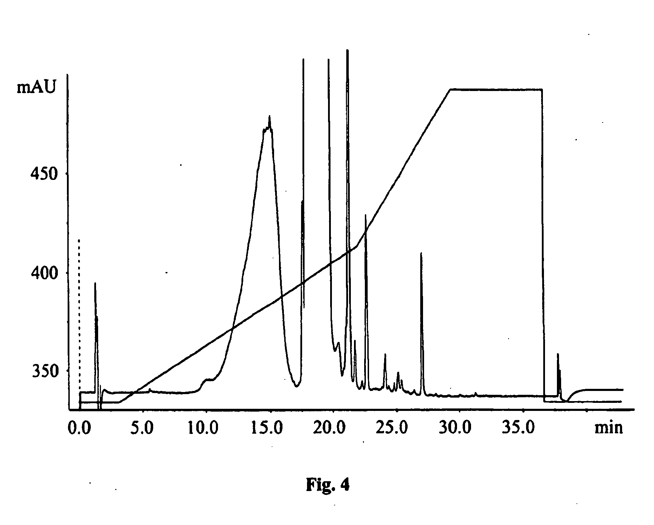

**Figures 4 to 6** show the analysis of the crude Daunorubicin-HES conjugate by HPLC reversed phase chromatography, UV-detection at 290 nm, obtained by thiazolidine. formation from H-Cys(H)-HES50/0.7 and Daunorubicin as described in experimental section 9. hereinunder.
   Fig. 4: HPLC analysis of the crude conjugate;

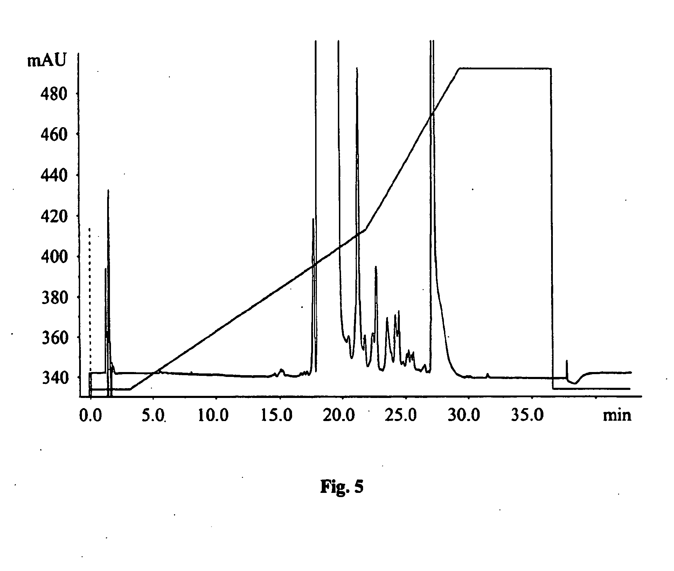

   Fig.5: HPLC analysis of Daunorubicin:

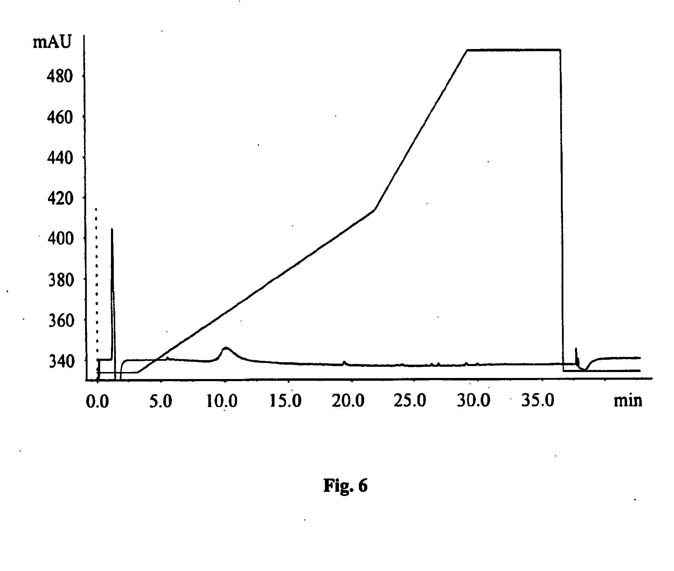

   Fig. 6: HPLC analysis of H-Cys(H)-HES50/0.7.

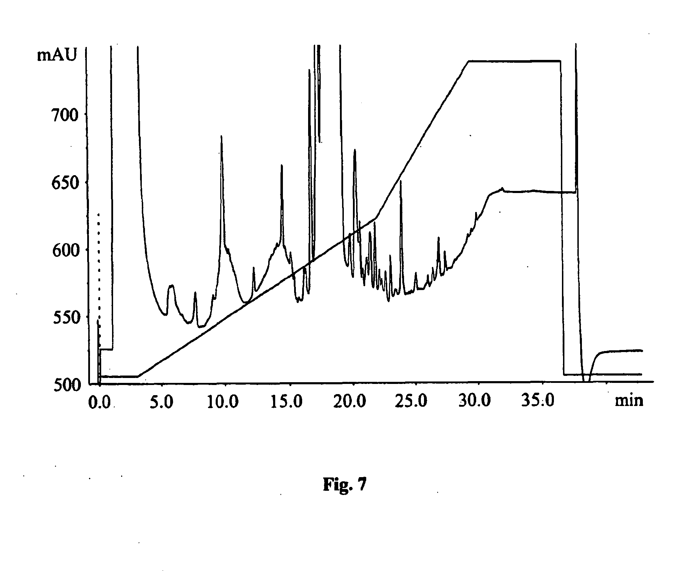

**Figures 7 to 9** show the analysis of the crude Tylosin-HES conjugate by HPLC reversed phase chromatography, UV-detection at 220 nm, obtained by thiazolidine formation from H-Cys(H)-HES50/0.7 and Tylosin as described in experimental section 9. hereinunder.
   Fig. 7: HPLC analysis of the crude conjugate

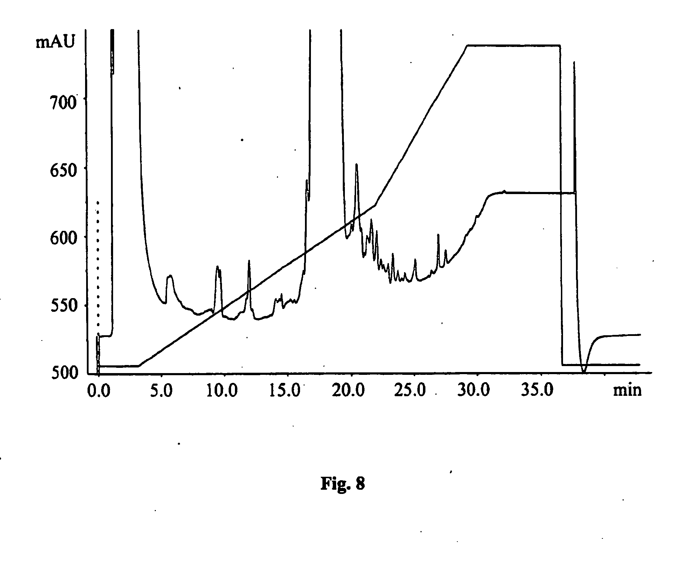

   Fig. 8: HPLC analysis of Tylosin

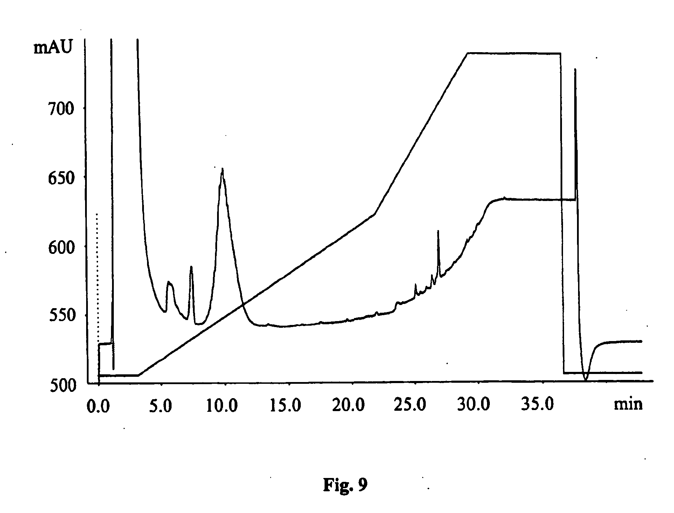

   Fig. 9: HPLC analysis of H-Cys(H)-HES50/0.7

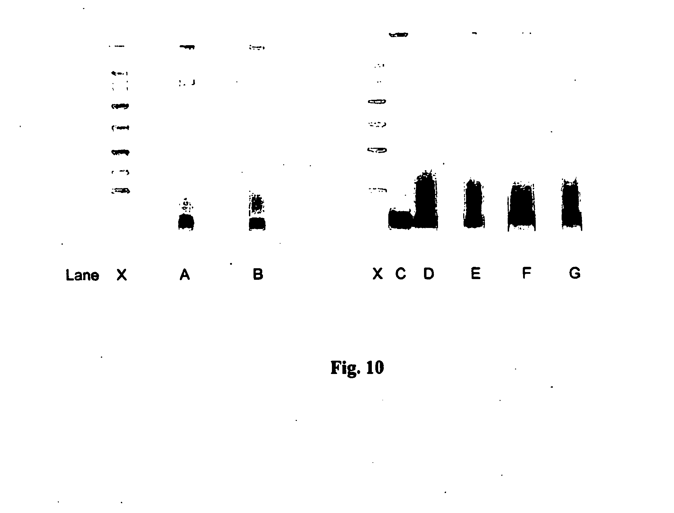

**Figure 10** shows the analysis of the crude peptide-HES conjugates by SDS gel electrophoresis as described in experimental section 10 hereinunder.
   - Lane X:: Roti®-Mark STANDARD (Carl Roth GmbH + Co. KG, Karlsruhe, D) Molecular weight marker from top to bottom: 200 kDa, 119 kDa, 66 kDa, 43 kDa, 29 kDa, 20 kDa, 14.3 kDa.
   - Lane A:: Conjugation of HES10/0.7 to peptide at 21°C, in DMF.
   - Lane B:: Conjugation of HES 10/0.7 to peptide at 37°C, in DMF.
   - Lane C:: Reaction control: Peptide at 50°C in DMF
   - Lane D:: Conjugation of HES 10/0.7 to peptide at 50°C, pH 4.6
   - Lane E:: Conjugation of HES10/0.7 to peptide at 50°C, in DMF
   - Lane F:: Conjugation of HES 10/0.7 to peptide at 50°C, pH 4.6, 1% Triton
   - Lane G:: Conjugation of HES10/0.7 to peptide at 50°C, in DMF, 1 % Triton

The invention will be explained in more detail by way of the following examples.

### Examples

### 1. Synthesis of H-Cys(StBu)-HES10/0.4

### 1.1 Synthesis of AminoHES10/0.4 from oxidized HES

Oxo-HES10/0.4 (4 g, MW = 10.9 kDa, DS = 0.4, Supramol Parenteral Colloids GmbH, Rosbach-Rodheim, D) was heated over night at 80°C in vacuo, dissolved under argon in dry dimethyl sulfoxide (25 mL, Fluka, Sigma-Aldrich Chemie GmbH, Taufkirchen, D) and 1,4-diaminobutane (4.0 mL, Fluka, Sigma-Aldrich Chemie GmbH, Taufkirchen, D) was added. After stirring at 45°C for 24 h the reaction mixture was added to 2-propanol (125 mL, Carl Roth GmbH + Co. KG, Karlsruhe, D) and incubated for 1 h at -20°C. The precipitated product was collected by centrifugation at 4°C, washed with 2-propanol (100 mL) and collected by centrifugation. The crude product was dissolved in water (20 mL, Milli-Q), dialysed for 43 h against Milli-Q water (SnakeSkin dialysis tubing, 3.5 kDa MWCO, Perbio Sciences Deutschland GmbH, Bonn, D) and lyophilized. The yield of isolated product was 65 %.

### 1.2 Synthesis of Fmoc-Cys(StBu)-HES10/0.4 from AminoHES

Fmoc-Cys(S-tBu)-OH (150 mg, Fluka, Sigma-Aldrich Chemie GmbH, Taufkirchen, D) and 1-hydroxy-1H-benzotriazole (61.4 mg, Aldrich, Sigma-Aldrich Chemie GmbH, Taufkirchen, D) were dissolved in N,N-dimethylformamide (3.5 mL Peptide synthesis grade, Biosolve, Valkenswaard, NL) and N,N'-diisopropylcarbodiimide (54.3 µL, Fluka, Sigma-Aldrich Chemie GmbH, Taufkirchen, D) were added. After incubation at 21 °C for 30 min, AminoHES10/0.4 (0.35 g), as obtained in 1.1. was added. After stirring at room temperature over night the reaction mixture was added to an ice-cold 1:1 mixture (35 mL, v/v) of acetone (Carl Roth GmbH + Co. KG, Karlsruhe, D) and ethanol (DAB, Sonnenberg, Braunschweig, D) and incubated for 1 h at -20°C. The precipitated product was collected by centrifugation at 4°C, dissolved in water (20 mL, Milli-Q) and dichloromethane (Carl Roth GmbH + Co. KG, Karlsruhe, D) was added (20 mL). The mixture was mixed thoroughly and centrifuged. The aqueous upper layer was dialysed for 41 h against Milli-Q water (SnakeSkin dialysis tubing, 3.5 kDa MWCO, Perbio Sciences Deutschland GmbH, Bonn, D) and lyophilized. The yield of isolated product was 78 %.

### 1.3 Synthesis of H-Cys(StBu)-HES10/0.4 from Fmoc-Cys(StBu)-HES10/0.4

Fmoc-Cys(S-tBu)HES10/0.4 (0.35 g) as obtained in 1.2 was dissolved in piperidine solution (4mL , 20% in DMF, v/v, Fluka, Sigma-Aldrich Chemie GmbH, Taufkirchen, D). After stirring at room temperature for 15 min the reaction mixture was added to an ice-cold 1:1 mixture of acetone and ethanol (35 mL, v/v) and incubated for 1 h at -20°C. The precipitated product was collected by centrifugation at 4°C and washed with tert-butyl methyl ether (25 mL, Acros Organics, Geel, B) and incubated for 1 h at -20°C. The precipitated product was collected by centrifugation at 4°C and dried in a stream of nitrogen. The yield of isolated product was 68 %.

### 2. Synthesis of AldehydoHES10/0.7

### 2.1 Synthesis of AminoHES10/0.7 from oxidized HES

Oxo-HES10/0.7 (6.02 g, MW = 14.7 kDa, DS = 0.76, Supramol Parenteral Colloids GmbH, Rosbach-Rodheim, D) was heated for 16.5 h at 80°C in vacuo, dissolved under argon in dry dimethyl sulfoxide (25 mL, Fluka, Sigma-Aldrich Chemie GmbH, Taufkirchen, D) and 1,4-diaminobutane (5.1 mL, Fluka, Sigma-Aldrich Chemie GmbH, Taufkirchen, D) was added. After stirring at 40°C for 17 h the reaction mixture was added to an ice-cold 1:1 mixture of acetone and ethanol (150 mL, v/v). The precipitated product was collected by centrifugation at 4°C, washed with an ice-cold 1:1 mixture of acetone and ethanol (40 mL, v/v) and collected by centrifugation. The crude product was dissolved in water (80 mL), dialysed for 42 h against Milli-Q water (SnakeSkin dialysis tubing, 3.5 kDa MWCO, Perbio Sciences Deutschland GmbH, Bonn, D) and lyophilized. The yield of isolated product was 67%.

### 2.2. Synthesis of AldehydoHES10/0.7 from AminoHES

4-Formylbenzoic acid (75 mg, Lancaster Synthesis, Frankfurt/ Main, D) and 1-hydroxy-1H-benzotriazole (115 mg, Aldrich, Sigma-Aldrich Chemie GmbH, Taufkirchen, D) were dissolved in N,N-dimethylformamide (DMF, 5 mL, Peptide synthesis grade, Biosolve, Valkenswaard, NL) and N,N'-diisopropylcarbodiimide (102 µL, Fluka, Sigma-Aldrich Chemie GmbH, Taufkirchen, D) was added. After incubation at room temperature for 30 min AminoHES10/0.7 (0.5 g, MW = 14.7 kDa, DS = 0.76) was added. After shaking at room temperature over night the reaction mixture was added to 2-propanol (30 mL, Carl Roth GmbH + Co. KG, Karlsruhe, D) and incubated for 1 h at -20°C. The precipitated product was collected by centrifugation at 4°C, washed with 2-propanol (30 mL) and collected by centrifugation. The crude product was dissolved in water (10 mL, Milli-Q), dialysed for 44 h against Milli-Q water (SnakeSkin dialysis tubing, 3.5 kDa MWCO, Perbio Sciences Deutschland GmbH, Bonn, D) and lyophilized. The yield of isolated product was 86 %.

### 3. Synthesis of AldehydoHES50/0.7

### 3.1. Synthesis of AminoHES50/0.7 from oxidized HES

Oxo-HES50/0.7 (6.09 g, MW = 56.7 kDa, DS = 0.76, Supramol Parenteral Colloids GmbH, Rosbach-Rodheim, D) was heated for 16.5 h at 80°C in vacuo, dissolved under argon in dry dimethyl sulfoxide (32 mL, Fluka, Sigma-Aldrich Chemie GmbH, Taufkirchen, D) and 1,4-diaminobutane (1.2 mL, Fluka, Sigma-Aldrich Chemie GmbH, Taufkirchen, D) was added. After stirring at 40°C for 17 h the reaction mixture was added to an ice-cold 1:1 mixture of acetone and ethanol (150 mL, v/v). The precipitated product was collected by centrifugation at 4°C, washed with an ice-cold 1:1 mixture of acetone and ethanol (40 mL, v/v) and collected by centrifugation. The crude product was dissolved in water (80 mL), dialysed for 42 h against Milli-Q water (SnakeSkin dialysis tubing, 3.5 kDa MWCO, Perbio Sciences Deutschland GmbH, Bonn, D) and lyophilized. The yield of isolated product was 82%.

### 3.2. Synthesis of AldehydoHES50/0.7 from AminoHES

4-Formylbenzoic acid (124 mg, Lancaster Synthesis, Frankfurt/ Main, D) and 1-hydroxy-1H-benzotriazole (174 mg, Aldrich, Sigma-Aldrich Chemie GmbH, Taufkirchen, D) were dissolved in N,N-dimethylformamide (DMF, 38 mL, Peptide synthesis grade, Biosolve, Valkenswaard, NL) and N,N'-diisopropylcarbodiimide (155 µL, Fluka, Sigma-Aldrich Chemie GmbH, Taufkirchen, D) was added. After incubation at room temperature for 30 min AminoHES50/0.7 (3.80 g, MW = 56.7 kDa, DS = 0.76) was added. After shaking at room temperature over night the reaction mixture was added to 2-propanol (160 mL, Carl Roth GmbH + Co. KG, Karlsruhe, D) and incubated for 1 h at -20°C. The precipitated product was collected by centrifugation at 4°C, dissolved in DMF (20 mL), precipitated with 2-propanol as described above and collected by centrifugation. The crude product was dissolved in water, dialysed for 24 h against Milli-Q water (SnakeSkin dialysis tubing, 3.5 kDa MWCO, Perbio Sciences Deutschland GmbH, Bonn, D) and lyophilized. The product was dissolved in water (20 mL), precipitated with an ice-cold 1:1 mixture of acetone and ethanol (150 mL, v/v), incubated for 1 h at -20°C and collected by centrifugation. The crude product was dissolved in water (29 mL), dialysed for 24 h against Milli-Q water (SnakeSkin dialysis tubing, 10 kDa MWCO, Perbio Sciences Deutschland GmbH, Bonn, D) and lyophilized. The yield of isolated product was 77 %.

### 4. Thiazolidine formation from H-Cys(H)-HES10/0.4 and oxidized EPO

### 4.1. Deprotection of H-Cys(StBu)-HES10/0.4

H-Cys(StBu)-HES10/0.4 (10 mg) as obtained in example 1 was dissolved in sodium acetate buffer (1 mL, 0.1 M, pH 4.6, 10 mM EDTA) and tris-(2-carboxyethyl)-phosphine hydrochloride (2.8 mg, TCEP, Acros Organics, Geel, B) was added. The reaction mixture was incubated for 30 min at room temperature and the TCEP excess was removed by diafiltration:

The reaction mixture was diluted with buffer (sodium phosphate buffer, 0.1 M, pH 8.0, 1 mM EDTA) to 0.5 mL and centrifuged at 20°C for 10 min at 13000 x g in a Vivaspin 500 concentrator (Viva Science, 5 kDa MWCO, Hannover, Germany). The washing procedure was repeated three times by dilution of the residual solution with buffer to 0.5 mL and centrifugation for 35 min as described. The H-Cys(H)-HES10/0.4 solutions was diluted with same reaction buffer to 150 µL having a final calculated concentration of 66.6 µg/mL.

### 4.2. Conjugation to oxidized EPO

To prepare the oxidized EPO, a 2.0 mg/mL solution of EPO (recombinantly produced EPO having amino acid sequence of human EPO and similar or essentially the same characteristics as the commercially available Epoietin alpha: Erypo, ORTHO BIOTECH, Jansen-Cilag or Epoietin beta: NeoRecormon, Roche; cf. EP 0 148 605, EP 0 205 564, EP 0411 678) of total 20 mL kept at 0°C were added 2.2 mL of an ice-cold solution of 10 mM sodium meta-periodate resulting in a final concentration of 1 mM sodium meta-periodate. The mixture was incubated at 0°C for 1 hour in an ice-bath in the dark and the reaction was terminated by addition of 40 µL of glycerol and incubated for further 5 minutes. The buffer of the mixture was changed to sodium acetate buffer pH 5.5.

To the obtained oxidized EPO (14.9 µL, 1.34 mg/mL, sodium acetate buffer pH 5.5), the H-Cys(H)-HES10/0.4 solution (5 µL) as obtained in 4.1. was added. After incubation for 21 h at room temperature, the reaction mixture was analysed by SDS gel electrophoresis. A XCell Sure Lock Mini Cell (Invitrogen GmbH, Karlsruhe, D) and a Consort E143 power supply (CONSORTnv, Turnhout, B) were employed for SDS gel electrophoresis. A 10 % Bis/Tris gel together with a MOPS running buffer at reducing conditions (both Invitrogen GmbH, Karlsruhe, D) were used according to the manufacturers instruction.

### 5. Thiazolidine formation from H-Cys(H)-Peptide-NH2 and AldehydoHES

A solution of the peptide carrying a free cysteine residue at its N-terminus (2 µL, 15 µg, 3147 g/mol, 7.5 mg/mL in DMF, H-CLPSLEGNMQQPSEFHCMMNWSSHIAAC-NH2, obtained by standard Fmoc solid phase synthesis and purified by HPLC) was added to 20 µL of a solution of AldehydoHES (see Table 1) in reaction buffer (see Table 1, degassed for 15 min in an ultrasonic bath) and the mixture was incubated over night at room temperature. For analysis by SDS gel electrophoresis, a XCell Sure Lock Mini Cell (Invitrogen GmbH, Karlsruhe, D) and a Consort E143 power supply (CONSORTnv, Turnhout, B) were employed. A 12 % Bis/Tris gel together with a MES running buffer at reducing conditions (both Invitrogen GmbH, Karlsruhe, D) were used according to the manufacturers instruction.

**Table 1: Reaction conditions**

| **AldehydoHES** | **Concentration of AldehydoHES** | **Reaction buffer** |
|---|---|---|
| AldehydoHES 10/0.7 as obatined in 2. | 119 mg/mL | Sodium acetate, 0.1 M, pH 4.6, 10 mM EDTA |
| AldehydoHES50/0.7 as obtained in 3. | 595 mg/mL | Sodium acetate, 0.1 M, pH 4.6, 10 mM EDTA |
| AldehydoHES 10/0.7 as obtained in 2. | 119 mg/mL | Sodium phosphate, 0.1 M, pH 8.0, 1 mM EDTA |
| AldehydoHES50/0.7 as obtained in 3. | 595 mg/mL | Sodium phosphate, 0.1 M, pH 8.0, 1 mM EDTA |

### 6. Results

The results of the experiments can be seen in the Figures.

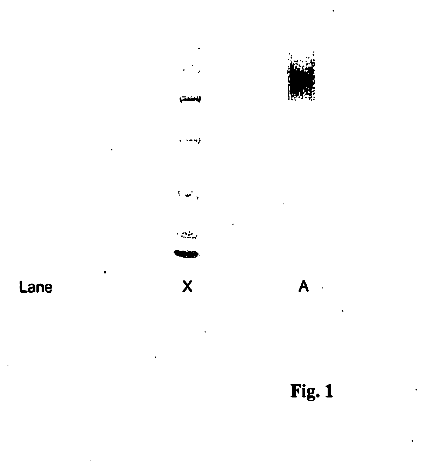

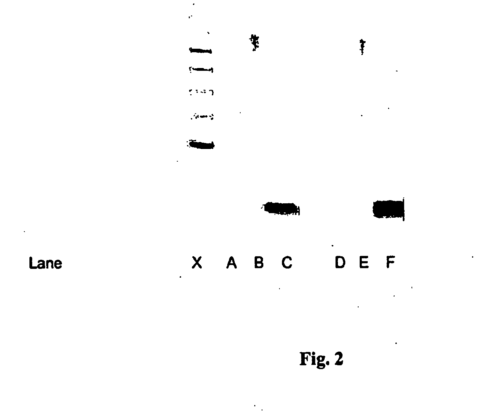

Two different strategies were conducted as can bee seen in the above examples. In one case (see 4.), the carbonyl group was introduced into the glycan of EPO by periodate oxidation and conjugated to an alpha-SH-beta amino group containing HES. This HES10/0.4 derivative was synthesized in two steps from HES, oxidized at the reducing end. The oxidized HES was converted by a known procedure to an AminoHES (see 1.1.) followed by acylation with a protected cysteine (Fmoc-Cys(StBu)-OH) (see 1.2.) and deprotection (see 1.3.). The other route (see 5.) utilised an AldehydoHES10/0.7 and an AldehydoHES50/0.7 (see 2.2. and 3.2.), synthesized by acylation of the known AminoHES (see 2.1. and 3.1.) with 4-formylbenzoic acid and a peptide, carrying an unprotected cysteine at its N-terminus. Complete to almost complete conversion was obtained by both strategies (see Fig. 1 and 2). The conjugation of AldehydoHES to the Cys-peptide proceeded equally well at pH 4.6 and pH 8.0 (see Fig 2, Lane A and D or Lane B and E). The reaction of the peptide at these reaction conditions with HES10/0.7 or HES50/0.7 failed. Under modified reaction conditions, success might be expected.

The method according to this invention has the advantage for proteins that mutants with N-terminal cysteine residues are obtainable through expression, while other modifications of proteins that would also allow for a chemoselective conjugation might not be available through this route. Therewith is it expected that a selective reaction with hydroxyalkyl starch, in particular hydroxyethyl starch through the N-terminal residue of the protein is permitted. A further advantage of the method according to the invention is the chemoselectivity of the reaction of the aldehyde, keto or hemiacetal group with the alpha-SH-beta amino group. Therefore, no reaction with the functional groups of the side chains of e.g. a protein or peptide is expected.

### 7. Synthesis of H-Cys(H)-HES50/0.7

### 7.1 Synthesis of AminoHES50/0.7 from oxidized HES

Oxo-HES50/0.7 (10.1 g, MW = 44.2 kDa, DS = 0.7, Lot 502, Supramol Parenteral Colloids GmbH, Rosbach-Rodheim, D) was heated for 72 h at 80°C in vacuo, dissolved under argon in dry dimethyl sulfoxide (52 mL, Fluka, Sigma-Aldrich Chemie GmbH, Taufkirchen, D) and 1,4-diaminobutane (2.3 mL, Fluka, Sigma-Aldrich Chemie GmbH, Taufkirchen, D) was added. After stirring at 45°C for 19.5 h the reaction mixture was added drop-wise to an 1:1 mixture (400 mL, v/v) of acetone (Carl Roth GmbH + Co. KG, Karlsruhe, D) and ethanol (DAB, Sonnenberg, Braunschweig, D). The precipitated product was collected by centrifugation. The crude product was dissolved in water (100 mL, Milli-Q), dialysed (SnakeSkin dialysis tubing, 10 kDa MWCO, Perbio Sciences Deutschland GmbH, Bonn, D) for 24 h against 20 mM acetic acid and 3.5 h against water. The yield of isolated product after lyophilization was 84 %.

### 7.2 Synthesis of Fmoc-Cys(StBu)-HES50/0.7 from AminoHES50/0.7

Fmoc-Cys(S-tBu)-OH (293.1 mg, Fluka, Sigma-Aldrich Chemie GmbH, Taufkirchen, D) and 1-hydroxy-1H-benzotriazole (155.9 mg, Iris Biotech GmbH, Marktredwitz, D) were dissolved in N,N-dimethylformamide (30 mL Peptide synthesis grade, Biosolve, Valkenswaard, NL) and N,N'-diisopropylcarbodiimide (138 µL, Fluka, Sigma-Aldrich Chemie GmbH, Taufkirchen, D) were added. After incubation at 21°C for 30 min, AminoHES50/0.7 (3.00 g), obtained as described in 7.1 was added. After stirring at room temperature over night the reaction mixture was added to an 1:1 mixture (230 mL, v/v) of acetone (Carl Roth GmbH + Co. KG, Karlsruhe, D) and ethanol (DAB, Sonnenberg, Braunschweig, D). The precipitated product was collected by centrifugation, dissolved in DMF (30 mL) and precipitated again as described above. After centrifugation, the crude product was dissolved in water (30 mL, Milli-Q), dialysed for 28 h against Milli-Q water (SnakeSkin dialysis tubing, 10 kDa MWCO, Perbio Sciences Deutschland GmbH, Bonn, D) and lyophilized. The yield of isolated product was 98 %.

### 7.3 Synthesis of H-Cys(StBu)-HES50/0.7 from Fmoc-Cys(StBu)-HES50/0.7

Fmoc-Cys(StBu)HES50/0.7 (2.62 g), obtained as described in 7.2 was dissolved in DMF (20 mL) and piperidine (5 mL , Fluka, Sigma-Aldrich Chemie GmbH, Taufkirchen, D) was added. After stirring at room temperature for 15 min the reaction mixture was added to an 1:1 mixtures of acetone and ethanol (190 mL, v/v). The precipitated product was collected by centrifugation, dissolved in DMF (25 mL) and precipitated again as described above. After centrifugation, the crude product was dissolved in water (25 mL, Milli-Q) dialysed for 45 h against Milli-Q water (SnakeSkin dialysis tubing, 10 kDa MWCO, Perbio Sciences Deutschland GmbH, Bonn, D) and lyophilized. The yield of isolated product was 83 %.

### 7.4 Synthesis of H-Cys(H)-HES50/0.7 from H-Cys(StBu)-HES50/0.7

H-Cys(S-tBu)HES50/0.7 (0.50 g), obtained as described in 7.3 was dissolved in 50 mM tris-(2-carboxyethyl)-phosphine hydrochloride solution (5 mL, TCEP, Acros Organics, Geel, B in 0.1 M sodium acetate buffer pH 5.0). After stirring at room temperature for 1 h the reaction mixture was dialysed (SnakeSkin dialysis tubing, 10 kDa MWCO, Perbio Sciences Deutschland GmbH, Bonn, D) for 22 h against 5 mM aqueous EDTA (Fluka, Sigma-Aldrich Chemie GmbH, Taufkirchen, D in Milli-Q water) and 1 h against water (Milli-Q) and lyophilized. The yield of isolated product was 97 %.

### 8. Conjugation to DNA

### 8.1 Hybridisation of Formylindol-modified DNA

5'-Formylindol-modified DNA (the Formylindole modification, i.e. the 3-Formylindole-modification, was introduced according to A. Okamoto et al., Tetrahedron Lett. 2002, 43, 4581-4583; 200 µL, atdbio, Southampton, UK, lot A0795, M = 9361 g/mol, c = 37.8 µM in water, sequence: XTACTCACCCTGCGAATTCAACTGCTGCCTC) and the unmodified complementary strand (199 µL, atdbio, Southampton, UK, lot A0794, M = 9376 g/mol, c = 38.2 µM in water, sequence:
GAGGCAGCATTGAATTCGCAGGGTGAGTA) were hybridized in a 1 : 1 molar ratio based on the concentrations given by the manufacturer. The solutions were mixed and incubated at 95°C for 5 min, resulting in a final concentration of 0.356 mg/mL double strand DNA. 157 µL (55.9 µg) was lyophilised over night and dissolved water (27.9, Milli-Q) resulting in a calculated final concentration of 2 mg/mL. The concentrations are calculated and were not checked experimentally.

### 8.2 Hybridization of 5'-Amino-C6-modified DNA

5'-Amino-C6-modified DNA (59.1 nmol, biomers.net GmbH, Ulm, D, lot 00033426_1 DNA, M = 9218 g/mol, 59.1 nmol, sequence:
TACTCACCCTGCGAATTCAACTGCTGCCTC) was dissolved in water (100 µl; Milli-Q). It (38.78 µL, 595 µM) was hybridized in a 1 : 1 molar ratio based on the concentrations given by the manufacturer to the unmodified complementary strand (600 µL, atdbio, Southampton, UK, lot A0794, M = 9376 g/mol, c = 38.2 µM in water, sequence:
   GAGGCAGCATTGAATTCGCAGGGTGAGTA). The solutions were mixed and incubated at 95°C for 5 min, resulting in a final calculated concentration of 0.667 mg/mL double strand DNA. The concentrations were calculated and not checked experimentally.

### 8.3 Formation of an FBA-modified DNA from 5'-Amino-C6-modified DNA

Succinimidyl 4-formylbenzoate (50 µL, 4 mg/mL in DMF, Novabiochem, Merck KGaA, Darmstadt, D) was added to double stranded 5'-Amino-C6-modified DNA, obtained as described in 8.2 (500 µL, 0.667 mg/mL in water) and the clear solution was incubated for 5 h at 21°C. The reaction mixture was centrifuged at 21°C for 15 min at 13000 x g in a Vivaspin 500 concentrator (Viva Science, 5 kDa MWCO, Hannover, Germany). The washing procedure was repeated three times by dilution of the residual solution with water (Milli-Q) to 0.5 mL and centrifugation for 12 min as described. The DNA solution was diluted with water to 167 µL, resulting in a final calculated concentration of 2 mg/mL. The concentrations were calculated and not checked experimentally.

### 8.4 Thiazolidine formation from H-Cys(H)-HES50/0.7 and Formylindol-modified DNA

A HES50/0.7-derivative solution (4 µL, 147.5 mg/mL, see Table 1) in reaction buffer (see Table 2 hereinafter) was added to an aqueous solution of Formylindol-modified DNA (1.4 µL, 2 mg/mL), obtained as described in 8.1. After incubation for 14 h at 21°C, the reaction mixture was analysed by agarose gel electrophoresis. An Agagel Standard system and a Minicell Power Pack P20 power supply (both Biometra GmbH, Göttingen, D) were employed. 2 % agarose NEEO Ultra-Qualität (Carl Roth GmbH, + Co. KG, Karlsruhe, D) together with a 0.5 x TBE running buffer were used for 1 h at 55 V. A Gel Doc 2000 gel documentation system together with the software Quality One V4.0.3 (both BIO-RAD Laboratories, München, D) were employed. The results of the experiments can be seen in Figure 2.

**Table 2: Reaction conditions**

| **HES50/0.7-derivative** | **Reaction buffer** |
|---|---|
| H-Cys(H)-HES50/0.7, as obtained in 7.4. | Sodium acetate, 0.1 M, pH 4.6, 10 mM |
| | EDTA |
| Oxo-HES50/0.7, (Supramol Parenteral | Sodium acetate, 0.1 M, pH 4.6, 10 mM |
| Colloids GmbH, Rosbach-Rodheim, D) | EDTA |
| H-Cys(H)-HES50/0.7, as obtained in 7.4. | Sodium phosphate, 0.1 M, pH 8.0, 5 mM |
| | EDTA |
| Oxo-HES50/0.7, (Supramol Parenteral | Sodium phosphate, 0.1 M, pH 8.0, 5 mM |
| Colloids GmbH, Rosbach-Rodheim, D) | EDTA |

### 8.5 Thiazolidine formation from H-Cys(H)-HES50/0.7 and FBA-modified DNA

A HES50/0.7-derivative solution (4 µL, 147.5 mg/mL, see Table 3 hereinafter) in a reaction buffer (see Table 3 hereinafter) was added to an aqueous solution of FBA-modified DNA (1.5 µL, 2 mg/mL), obtained as described in 8.3. After incubation for 14 h at 21°C, the reaction mixture was analysed by agarose gel electrophoresis as described in 8.4. The results of the experiments can be seen in Figure 3.

**Table 3: Reaction conditions**

| **HES50/0.7-derivative** | **Reaction buffer** |
|---|---|
| H-Cys(H)-HES50/0.7, as obtained in 7.4. | Sodium acetate, 0.1 M, pH 4.6, 10 mM |
| | EDTA |
| Oxo-HES50/0.7, (Supramol Parenteral | Sodium acetate, 0.1 M, pH 4.6, 10 mM |
| Colloids GmbH, Rosbach-Rodheim, D) | EDTA |
| H-Cys(H)-HES50/0.7, as obtained in 7.4. | Sodium phosphate, 0.1 M, pH 8.0, 5 mM |
| | EDTA |
| Oxo-HES50/0.7, (Supramol Parenteral | Sodium phosphate, 0.1 M, pH 8.0, 5 mM |
| Colloids GmbH, Rosbach-Rodheim, D) | EDTA |

### 9. Conjugation to small organic molecules (here: antibiotics (Daunorubicin) and cytostatics (Tylosin))

### Conjugation to small organic molecules by thiazolidine formation

H-Cys(H)-HES50/0.7 (11.0 mg), obtained as described in 7.4, was dissolved in a small organic molecule solution (44 µL, having concentration [A] mg/mL, 13.4 equiv., see Table 4 hereinafter) in DMF (see Table 4 hereinafter), incubated at 21°C over night and 40 µL of the reaction mixture was analysed by HPLC. The results of the experiments can be seen in Figures 4 to 9.

Daunorubicin (Fluka, Sigma-Aldrich, Taufkirchen, D) and Tylosin tartrate (both BioChemika grade, Fluka, Sigma-Aldrich, Taufkirchen, D) were employed as small organic molecules.

For HPLC analysis a ReproSil-PUR Basic C18 column 150 x 4.6 mm i. d. (Order # R15.B9.S1546, Dr. Maisch GmbH, Ammerbuch, D) and an Akta Basic chromatography system (Pump P900 Ser. # 01118816, UV-detector UV900 Ser. # 01120613, pH/conductivity-detector pH/C900 Ser.# 01120665, fraction collector Frac900 Ser. # 01120011 and software Unicorn 5.0 Ser. #01119821, all Amersham Biosciences, Freiburg, D) were used.

The following method was applied for all analyses:
- Buffer A: water with 0.1 TFA (Rotisolv LC-MS Garde, Carl Roth GmbH + Co. KG, Karlsruhe, D)
- Buffer B: A mixture of 15 % water with 0.1 TFA and 85 % acetonitrile with 0.1% TFA (both Rotisolv LC-MS grade, Carl Roth GmbH + Co. KG, Karlsruhe, D).
- Gradient: 0 % buffer B in 2 column volumes (CV); linear gradient 0 - 50 % buffer B in 15 CV, linear gradient 50 - 100 % buffer B in 7 CV, 100 % buffer B for 7 CV, 0 % buffer B for 5 CV.
- UV-Detection at 220 and 290 nm.
- Flow rate: 2 mL/min.

**Table 4: Reaction conditions**

| **Small Organic Molecule** | **Concentration [A] / (mg/mL)** |
|---|---|
| Daunorubicin | 43.0 |
| Tylosin | 80.7 |

### 10. Thiazolidine formation from H-Cys-Peptide and native HES (not according to the invention)

A solution of a peptide carrying a free cysteine residue at its N-terminus (1 µL, 20 µg, 2528 g/mol, 20 mg/mL in DMF, Sequenze H-Cys-Asn-Thr-Arg-Lys-Arg-Ile-Arg-Ile-Gln-Arg-Gly-Pro-Gly-Arg-Ala-Phe-Val-Thr-Ile-Gly-Lys-OH, SC623, NeoMPS S.A., Strasbourg, F) was added to 9 µL of a solution of HES10/0.7 (408 mg/mL in [Reaction Buffer] (see Table 5 below), MW = 9.2 kDa, DS = 0.7, Lot 437, Supramol Parenteral Colloids GmbH, Rosbach-Rodheim, D), Triton-X100 solution (1 µL, 10 % in water) was added to selected reactions (see Table 5 below) and the mixtures were incubated over night at [B] °C (see Table 5 below). For analysis by SDS gel electrophoresis, a XCell Sure Lock Mini Cell (Invitrogen GmbH, Karlsruhe, D) and a Consort E143 power supply (CONSORTnv, Turnhout, B) were employed. A 12 % Bis/Tris gel together with a MOPS running buffer at reducing conditions (both Invitrogen GmbH, Karlsruhe, D) were used according to the manufacturers instruction. The results of the experiment can be seen in Figure 10.

**Table 5: Reaction conditions**

| **Lane** | **[Reaction Buffer]** | **[B] / °C** | **Triton-X100** |
|---|---|---|---|
| A | DMF | 21 | - |
| B | DMF | 37 | - |
| C | DMF | 50 | - |
| D | 0.1 M NaOAc pH 4.6, 10 mM EDTA | 50 | - |
| E | DMF | 50 | - |
| F | 0.1 M NaOAc pH 4.6, 10 mM EDTA | 50 | 1 µL |
| G | DMF | 50 | 1 µL |

| | | | |
|---|---|---|---|
| * without HES | | | |

### 11. Results of the experiments according to items 8 to 10 above

### 11.1 Conjugation to DNA by thiazolidine formation (item 8 above)

Conjugation to DNA by thiazolidine formation was achieved with two different aldehyde-modified DNAs (see item 8. hereinabove). The necessary DNA-aldehyds were either commercially available in the case of Formylindole-modified DNA (see item 8.1 hereinabove) or were prepared from commercially available 5'-Amino-DNA and Succinimidyl 4-formylbenzoate (see item 8.3 hereinabove). The alpha-SH-beta amino group containing HES (H-Cys(H)-HES50/0.7) was prepared from H-Cys(StBu)-HES50/0.7 by reduction withTCEP and purified by precipitation and dialysis (see item 7.4 hereinabove). H-Cys(StBu)-HES50/0.7 was prepared in analogy to H-Cys(StBu)-HES10/0.4 (see item 4.1 hereinabove). The results of the conjugation to both aldehyde-modified DNAs are shown in Fig. 3. Successful conjugation was indicated by the appearance of a new band at higher molecular weight. Its increased band width was due to the molecular weight distribution of the HES part of the conjugate. Conjugation was achieved at pH 4.6 (Lane A) or pH 8.0 (Lane C). No conjugation was observed with OxoHES50/0.7, the starting material for H-Cys(H)-HES50/0.7 (Lane B and D).

### 11.2 Conjugation to small organic molecules (item 9 above)

Conjugation to small organic compounds by thiazolidine formation was achieved for a cytostatic drug (Daunorubicin, see Fig. 4 hereinunder) and an antibiotic (Tylosin, see Fig. 7 hereinunder). The alpha-SH-beta amino group containing HES starting material (the same concentration as in the conjugation reaction) appeared as a broad band with a retention time around 10 min (see Figs. 6, 9 hereinunder). Its increased peak width was due to the molecular weight distribution of the HES. The HPLC analyses of the small organic compounds (the same concentration as in the conjugation reaction) are shown in Fig. 5 and 8. Successful conjugation is indicated by the appearance of a new broad peak at retention times between that of the HES starting material and that of the small organic compound (Figs. 4, and 7). Again, the broad peak form reflected the molecular weight distribution of the HES part of the conjugate.

### 11.3 Conjugation using native HES (item 10 above) (not according to the invention)

Conjugation to native HES by thiazolidine formation was achieved for a peptide carrying a free cysteine residue at its N-terminus (see item 10. hereinabove) in DMF as the solvent in a temperature range between 21 °C - 50° C (Fig. 10 Lanes A, B and E) or in aqueous buffer at pH 4.6 at 50 °C (Fig. 10, Lane D). Conjugation was also observed in the presents of the detergent Triton X-100 at 50 °C (Fig. 10, Lanes F and G). Successful conjugation was indicated by the appearance of a new band at higher molecular weight. Its increased band width was due to the molecular weight distribution of the HES part of the conjugate.

Thus, it could be shown that conjugation of native HES with a Cys-peptide is generally possible, even under various different conditions, as pointed out under item 6. hereinabove.

## Claims

1. A method for preparing a conjugate of an active substance and hydroxyalkyl starch, wherein the active substance and the hydroxyalkyl starch are covalently linked by a chemical residue having a structure according to formula (I) 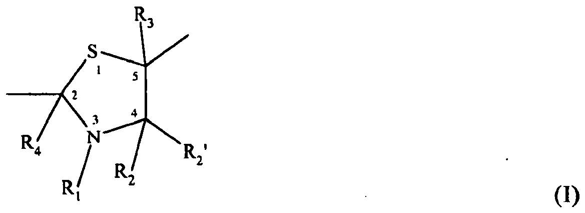 or formula (I') 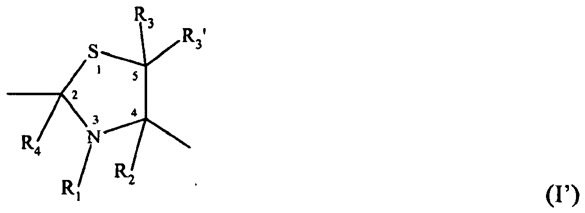 or formula (I") 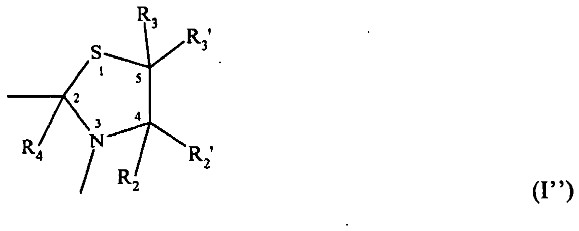 wherein R₁, R₂, R_{2'}, R₃, R_{3'} and R₄ are independently selected from the group consisting of hydrogen, an optionally suitably substituted, linear, cyclic and/or branched alkyl, aryl, heteroaryl, aralkyl, and heteroaralkyl group, preferably hydrogen,
said method comprising
(A) reacting an aldehyde group, a keto group or a hemiacetal group of a hydroxyalkyl starch derivative comprising said aldehyde group, keto group or hemiacetal group with an alpha-SH-beta amino group 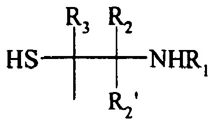 of an active substance or a derivative thereof comprising said alpha-SH-beta amino group, thereby preparing a conjugate of said active substance and said hydroxyalkyl starch being covalently linked by a chemical residue having a structure according to formula (1); or with an alpha-SH-beta amino group 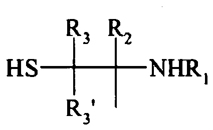 of an active substance or a derivative thereof comprising said alpha-SH-beta amino group, thereby preparing a conjugate of said active substance and said hydroxyalkyl starch being covalently linked by a chemical residue having a structure according to formula (I'), or with an alpha-SH-beta amino group 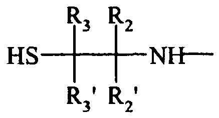 of an active substance or a derivative thereof comprising said alpha-SH-beta amino group, thereby preparing a conjugate of said active substance and said hydroxyalkyl starch being covalently linked by a chemical residue having a structure according to formula (I"), wherein R₁, R₂, R_{2'}, R₃, R_{3'} and R₄ are as defined above;
or
(B) reacting an aldehyde group, a keto group or a hemiacetal group of an active substance or a derivative thereof comprising said aldehyde group, keto group or hemiacetal group with an alpha-SH-beta amino group 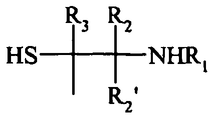 of a hydroxyalkyl starch derivative comprising said alpha-SH-beta amino group, thereby preparing a conjugate of said active substance and said hydroxyalkyl starch being covalently linked by a chemical residue having a structure according to formula (I); or with an alpha-SH-beta amino group 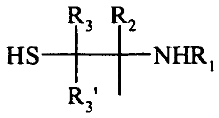 of a hydroxyalkyl starch derivative comprising said alpha-SH-beta amino group, thereby preparing a conjugate of said active substance and said hydroxyalkyl starch being covalently linked by a chemical residue having a structure according to formula (I'); or with an alpha-SH-beta amino group 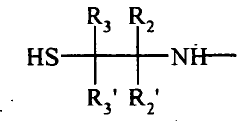 of a hydroxyalkyl starch derivative comprising said alpha-SH-beta amino group, thereby preparing a conjugate of said active substance and said hydroxyalkyl starch being covalently linked by a chemical residue having a structure according to formula (I"), wherein R₁, R₂, R_{2'}, R₃, R_{3'} and R₄ are as defined above
and wherein the hydroxyalkyl starch derivative comprising said aldehyde group, keto group, hemiacetal group or said alpha-SH-beta amino group is obtained by a method comprising reacting the hydroxyalkyl starch with at least one, at least bifunctional compound, said compound comprising two functional groups M₁ and Q, one functional group M₁ being reacted with the hydroxyalkyl starch and one functional group Q being
(i) an aldehyde group, keto group, hemiacetal group or an alpha-SH-beta amino group; or
(ii) a functional group being chemically modified to give the aldehyde group, keto group, hemiacetal group or the alpha-SH-beta amino group.

2. The method of claim 1, wherein the hydroxyalkyl starch has the following structure (II) 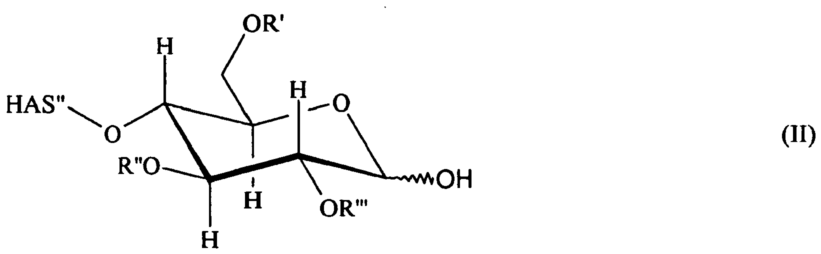 wherein R', R" and R"' are independently hydrogen, a linear or branched hydroxyalkyl group or the group
-[(CR¹R²)ₘO]ₙ[CR³R⁴]ₒ-OH
wherein R¹, R², R³, and R⁴ are independently selected from the group, consisting of hydrogen, and alkyl group, preferably hydrogen and methyl group,
m is 2 to 4, wherein the residues R¹ and R² may be the same or different in the m groups CR¹R²;
n is 0 to 20, preferably 0 to 4;
o is 0 to 20, preferably 2-4, wherein in the case of n = 0, o is not 0, and wherein the residues R³ and R⁴ may be the same or different in the o groups CR³R⁴;
wherein, preferably, R', R" and R"' are independently hydrogen or a 2-hydroxyethyl group.

3. The method of claim 1 or 2, wherein the Hydroxyalkyl starch is hydroxyethyl starch, and wherein, preferably, the hydroxyehtylstarch has a molecular weight of from 1 to 300 kD, more preferably from 2 to 200 kD, more preferably of from 10 to 150 KD or 4 to 130 kD, more preferably from 10 to 100 kD; and/or a molar substitution of from 0.1 to 3, more preferably 0.1 to 2, more preferably 0.1 to 0.9 or 0.4 to 2, more preferably 0.4 to 1.3; and/or a ratio between C₂ : C₆ substitution in the range of from 2 to 20 with respect to the hydroxyethyl groups.

4. The method of any of claims 1 to 3, wherein the active substance is selected from the group consisting of proteins, peptides, small molecule drugs, glycoproteins and oligonucleotides, such as DNA, RNA, PNA or derivatives thereof, preferably from the group consisting of proteins, peptides and PNA comprising an alpha-SH-beta amino group, preferably comprising a cysteine group, in particular an N-terminal cysteine, wherein the protein is preferably selected from the group consisting of EPO, G-CSF, IFN alpha, IFN beta, AT III, IL-2, IL-3, myoglobin, SOD, BSA, rhEPO, rhG-CSF, rhIFN alpha, rhIFN beta, rhAT III, rhIL-2, rhIL-3, A1AT, factor VII, factor VIII, factor IX, tPA, and APC.

5. The method of claim 4, wherein the active substance is selected from the group consisting of proteins, glycoproteins or peptides comprising an aldehyde, keto group or hemiacetal group, preferably glycoproteins containing an aldehyde in a glycan side chain or synthetic peptides.

6. The method of any of the preceding claims, wherein the hydroxyalkyl starch derivative comprises 1 to 100, preferably 1 to 15, in particular 1 aldehyde group(s), keto group(s) and/or hemiacetal group(s) or wherein the hydroxyalkyl starch derivative comprises 1 to 100, preferably 1 to 15, in particular 1 alpha-SH-beta amino group(s).

7. The method of any of the preceding claims, wherein the active substance comprises 1 to 15, preferably 1 to 8, in particular 1 aldehyde group(s), keto group(s) and/or hemiacetal group(s) or wherein the active substance comprises 1 to 15, preferably 1 to 8, in particular 1 alpha-SH-beta amino group(s).

8. The method of claims 1 to 7, wherein, the functional group M₁ is reacted with an OH-group on the hydroxyalkyl starch or the oxidized or non-oxidized reducing end of hydroxyalkyl starch.

9. The method of claims 1 to 7, wherein the functional group M₁ is a carboxy group or a reactive carboxy group and the functional group Q is an aldehyde group, keto group or hemiacetal group, wherein the bifunctional compound comprising M₁ and Q is preferably selected from the group consisting of formylbenzoic acid, 4-formylbenzoic acid pentafluorophenyl ester, 4-formylbenzoic acid N-hydroxysuccinimide ester, and 4-(4-formyl-3,5-dimethoxyphenoxy)butyric acid or a biocompatible compound selected from the group consisting of alpha-keto carboxylic acids, neuraminic acids or derivatives thereof and pyridoxal phosphate.

10. The method of claims 1 to 7, wherein in (ii), the at least bifunctional compound comprises an amino group M₁ and an amino group Q, and wherein the at least bifunctional compound is preferably an optionally substituted diaminoalkane having from 1 to 20 carbon atoms, more preferably a compound being selected from the group consisting of 1,2-diaminoethane, 1,3-diaminopropane, and 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diaminohexadecane, 1,17-diaminoheptadecane, 1,18-diaminooctadecane, 1,19-diaminononadecane, and 1,20-diaminoeicosane or a compound having the formula 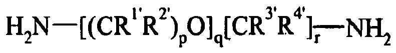 wherein R^{1'}, R^{2'}, R^{3'}, and R^{4'} are independently selected from the group, consisting of hydrogen, and alkyl group, preferably hydrogen and methyl group,
p is 2 to 4, wherein the residues R^{1'} and R^{2'} may be the same or different in the p groups CR^{1'}R^{2'},
q is 0 to 20, preferably 0 to 10;
r is 0 to 20, preferably 2-4, wherein in the case of q = 0, r is not 0, and wherein the residues R^{3'} and R^{4'} may be the same or different in the r groups CR^{3'}R^{4'}.

11. The method of claim 10, additionally comprising reacting the hydroxyalkyl starch derivative, resulting from the reaction of the hydroxyalkyl starch with the at least bifunctional compound comprising two amino groups M₁ and Q, at the amino group Q with a further bifunctional compound comprising an aldehyde group, keto group or hemiacetal group to give a hydroxyalkyl starch derivative having an aldehyde group, keto group or hemiacetal group, wherein the further bifunctional compound is preferably selected from the group consisting of formylbenzoic acid, 4-formylbenzoic acid pentafluorophenyl ester, 4-formylbenzoic acid N-hydroxysuccinimide ester, 4-(4-formyl-3,5-dimethoxyphenoxy)butyric acid, and 4-formylbenzoic acid anhydride.

12. The method of claim 10 or 11, wherein the hydroxyalkyl starch is reacted via its optionally oxidized reducing end with the functional group M₁, and wherein, preferably, statistically more than 50 %, more preferably at least 55 %, more preferably at least 60 %, more preferably at least 65 %, more preferably at least 70 %, more preferably at least 75 %, more preferably at least 80 %, more preferably at least 85 %, more preferably at least 90 %, and still more preferably at least 95 % such as 95 %, 96 %, 97 %, 98 %, or 99 % of the hydroxyalkyl starch molecules employed for a given reaction are reacted via at least one optionally oxidized reducing end per hydroxyalkyl starch molecule.

13. The method of claims 1 to 7, wherein in (i), the functional group M₁ is selected from the group consisting of a carboxy group, a reactive carboxy group, carboxylic acid anhydride, carboxylic acid halogenide, isocyanate, isothiocyanate, chloroformic acid ester, and epoxide groups and the functional group Q is an aldehyde group, keto group or hemiacetal group, wherein the functional group M₁ is preferably reacted with OH-groups on the hydroxyalkyl starch.

14. The method of claims 1 to 7, wherein in (i), the functional group M₁ is selected from the group consisting of an amino group and an alpha-SH-beta amino group and the functional group Q is an alpha-SH-beta amino group, and wherein the functional group M₁ is preferably reacted with the optionally oxidized reducing end of the hydroxyalkyl starch, wherein, preferably, statistically more than 50 %, more preferably at least 55 %, more preferably at least 60 %, more preferably at least 65 %, more preferably at least 70 %, more preferably at least 75 %,more preferably at least 80 %, more preferably at least 85 %, more preferably at least 90 %, and still more preferably at least 95 % such as 95 %, 96 %, 97 %, 98 %, or 99 % of the hydroxyalkyl starch molecules employed for a given reaction are reacted via at least one, optionally oxidized, reducing end per hydroxyalkyl starch molecule.

15. The method of claims 1 to 7, wherein in (i), the hydroxyalkyl starch comprising said alpha-SH-beta-amino group is obtained by a method comprising reacting hydroxyalkyl starch at the optionally oxidized reducing end with a compound comprising a functional group M₁ and a functional group Q being an alpha-SH-beta-amino group, wherein the compound comprising M₁ and the alpha-SH-beta-amino group is preferably 1,3-diamino-2-thio propane, or 2,3-diamino-1-thio propane.

16. The method of claims 1 to 7, wherein in (ii) the at least bifunctional compound comprises M₁ being a carboxy group or a reactive carboxy group and Q being a protected alpha-SH-beta amino group.

17. The method of claims 1 to 7, wherein the at least bifunctional compound is selected from the group consisting of D-, L-PG₁-Cys(PG₂)-OH, or a racemic mixture thereof, and their active ester, wherein PG₁ may be any suitable protecting group for an amino group, preferably selected from the group consisting of *tert*-butyloxycarbonyl (Boc) or 9-fluorenylmethoxycarbonyl (Fmoc), and PG₂ may be any suitable protecting group for a thiol group, preferably selected from the group consisting of trityl (Trt), p-methoxytrityl (Mmt) S-*tert*-butylthio (S-t-Bu), and acetamidomethyl (Acm).

18. The method of claims 1 to 7, wherein in (ii), the hydroxyalkyl starch comprising said alpha-SH-beta-amino group is obtained by a method comprising optionally oxidizing hydroxyalkyl starch at its reducing end, reacting the oxidized or non-oxidized reducing end with a functional group M₁ of a compound comprising, in addition to M₁, a further functional group Q, to give a first hydroxyalkyl starch derivative, and reacting the functional group Q of the first hydroxyalkyl starch derivative with a functional group V of a compound comprising, in addition to V, an optionally protected alpha-SH-beta-amino group, to give the optionally protected alpha-SH-beta-amino functionalized hydroxyalkyl starch derivative, and wherein the compound comprising M₁ and Q is a diamino compound or carbodiimidazol or N,N'-disuccinimidyl carbonate, wherein the at least bifunctional compound is preferably an optionally substituted diaminoalkane having from 1 to 20 carbon atoms, more preferably a compound being selected from the group consisting of 1,2-diaminoethane, 1,3-diaminopropane, and 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane, 1,13-diaminotridecane, 1,14-diaminotetradecane, 1,15-diaminopentadecane, 1,16-diaminohexadecane, 1,17-diaminoheptadecane, 1,18-diaminooctadecane, 1,19-diaminononadecane, and 1,20-diaminoeicosane or a compound having the formula 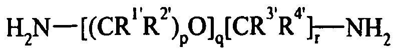 wherein R^{1'}, R^{2'}, R^{3'}, and R^{4'} are independently selected from the group, consisting of hydrogen, and alkyl group, preferably hydrogen and methyl group,
p is 2 to 4, wherein the residues R^{1'} and R^{2'} may be the same or different in the p groups CR^{1'}R^{2'},
q is 0 to 20, preferably 0 to 10;
r is 0 to 20, preferably 2-4, wherein in the case of q = 0, r is not 0, and wherein the residues R^{3'} and R^{4'} may be the same or different in the r groups CR^{3'}R⁴;
and wherein the compound comprising V and the optionally protected alpha-SH-beta-amino group is preferably cysteine or a derivative thereof, V being a carboxy group or a reactive carboxy group, preferably a reactive ester or a carboxylic acid anhydride or the compound comprising V is 1,3-diamino-2-thio propane or 2,3-diamino-1-thio propane.

19. The method of any of the preceding claims, wherein the active substance, preferably an optionally modified protein, peptide, synthetic peptide or oligonucleotide, comprising said aldehyde group, keto group, hemiacetal group or said alpha-SH-beta-amino group is obtained by a method comprising
(b)(1)introducing at least one aldehyde group, keto group, hemiacetal group or at least one alpha-SH-beta-amino group into the active substance during its preparation or by chemical modification, or
(b)(2)reacting the active substance with an at least bifunctional compound, said compound comprising two functional groups M₂ and Q, one functional group M₂ being reacted with the active substance and one functional group Q being
(i) an aldehyde group, keto group, hemiacetal group or an alpha-SH-beta amino group; or
(ii) a functional group being chemically modified to give the aldehyde group, keto group, hemiacetal group or an alpha-SH-beta amino group.

20. The method of claim 19, wherein in (b)(1), the active substance is a protein or peptide which was prepared by organic synthesis, preferably produced using a synthesis resin, allowing for an aldehyde functionalized, keto functionalized, hemiacetal functionalized or alpha-SH-beta-amino functionalized protein or peptide, or wherein in (b)(1), the active substance is a protein or peptide which was produced using an expression vector leading to an aldehyde functionalized, keto functionalized, hemiacetal functionalized or alpha-SH-beta-amino functionalized protein or peptide, or wherein in (b)(1), the active substance is a protein or a peptide and the backbone of the protein or peptide is substituted with an aldehyde group, keto group, hemiacetal group or alpha-SH-beta-amino group, or wherein in (b)(1), the active substance is a protein or a peptide where said aldehyde group, keto group , hemiacetal group or said alpha-SH-beta-amino group is linked directly to the backbone of the protein or peptide or is part of a side-chain of the backbone.

21. The method of claim 19, wherein in (b)(1) the active substance is a protein or peptide and the aldehyde group, keto group or hemiacetal group is comprised in a carbohydrate moiety of the polypeptide, wherein, preferably,
the carbohydrate moiety is a derivative of a naturally occurring carbohydrate moiety and is selected from the group consisting of glucose, galactose, mannose, and sialic acid, which are optionally chemically or enzymatically oxidized, preferably an oxidized galactose or an oxidized sialic acid residue of a carbohydrate side chain, more preferably the terminal galactose or sialic acid residue of a carbohydrate side chain, the oxidation of a terminal carbohydrate moiety preferably being performed either enzymatically or chemically, a chemical oxidation preferably carried out using a periodate,
or the carbohydrate moiety is selected from the group consisting of hydroxyaldehydes, hydroxyketones and chemical modifications thereof,
or the carbohydrate moiety is a derivative of a naturally occurring carbohydrate moiety and is a terminal galactose, which is chemically or enzymatically oxidized, wherein the terminal galactose residue is optionally obtained after cleavage of a terminal sialic acid.

22. The method of claim 19, wherein in (b)(2)(i), the alpha-SH-beta-amino group is comprised in a cysteine residue of the active substance, preferably a protein or peptide, the cysteine residue preferably being a N-terminal cysteine residue of the active substance.

23. The method of any of the preceding claims, wherein the active substance is a modified protein or peptide with an N-terminal cysteine residue, which is not part of a disulfide bridge, wherein the modified protein or peptide possessing an N-terminal cysteine residue is preferably a mutant of a naturally occurring protein or peptide, obtained by (1) adding a cysteine residue to the N-terminal amino acid, (2) substituting the N-terminal amino acid with cysteine, or by (3) deleting the N-terminal amino acid(s) until a terminal cysteine is obtained.

24. The method of any of the preceding claims, wherein the reaction (A) or (B) is carried out at a temperature of 0 to 40 °C, preferably 0 to 25 °C, in particular 20 to 25 °C, in the presence of a solvent, and at a pH of 3.5 to 10, preferably 4 to 8, in particular 4.8 to 8.0 with a reaction time of preferably 0.1 to 24 h, in particular about 21 h, wherein, preferably, the solvent is selected from the group consisting of water, aqueous buffer, DMF, DMSO, DMA and mixtures thereof and/or the molecular ratio of hydroxalkyl starch to active substance is about 1:1 to 200:1, preferably 10:1 to 100:1, in particular 40:1 to 70:1.

25. A conjugate of an active substance and hydroxyalkyl starch, as obtainable by a method of any of claims 1 to 24.

26. A conjugate of an active substance and hydroxyalkyl starch, wherein the active substance and the hydroxyalkyl starch are covalently linked by a chemical residue having a structure according to formula (I) 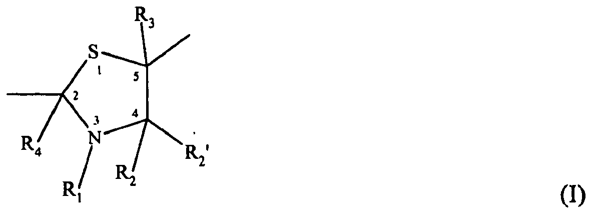 or formula (I') 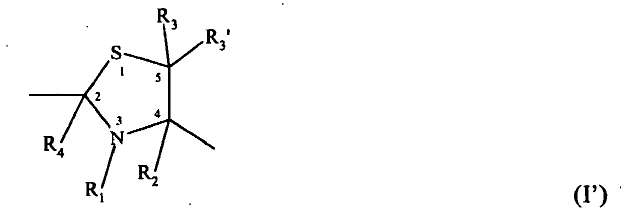 or formula (I") 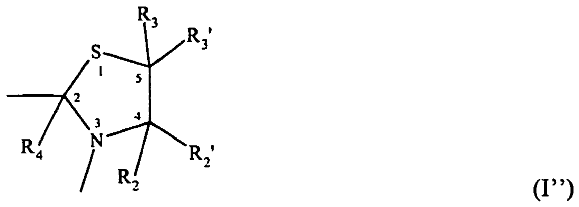 wherein R₁, R₂, R₂', R₃, R₃' and R₄ are independently selected from the group consisting of hydrogen, an optionally suitably substituted, linear, cyclic and/or branched alkyl, aryl, heteroaryl, aralkyl, and heteroaralkyl group, preferably hydrogen,
said conjugate having a structure according to formula (IV), (IV'), or (IV") 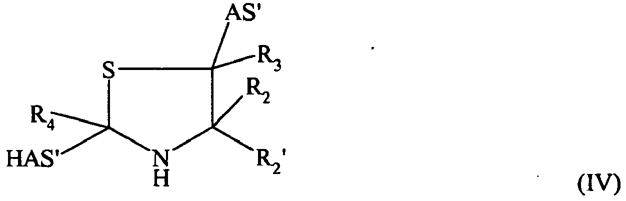 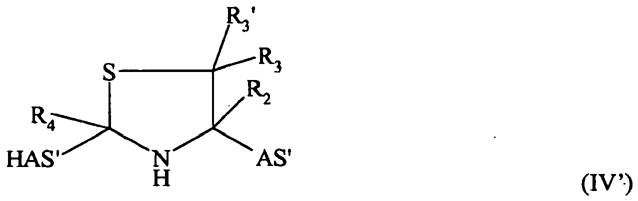 or 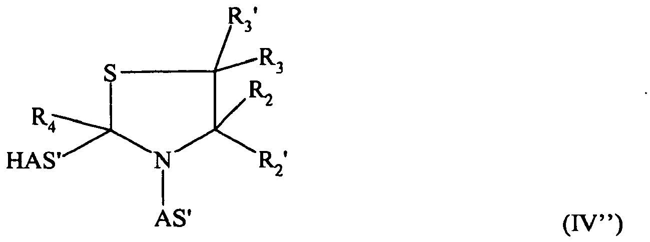 wherein HAS' is the residue of the hydroxyalkyl starch derivative which was linked to an aldehyde group, keto group or hemiacetal group by reacting hydroxyalkyl starch with at least one, at least bifunctional compound, said compound comprising two functional groups M₁ and Q, one functional group M₁ being reacted with the hydroxyalkyl starch and one functional group Q being
(i) an aldehyde group, keto group, hemiacetal group; or
(ii) a functional group being chemically modified to give the aldehyde group, keto group, hemiacetal group,
and wherein AS' is the residue of the active substance or a derivative thereof which was linked to the alpha-SH-beta-amino group,
or a structure according to formula (V), (V'), or (V") 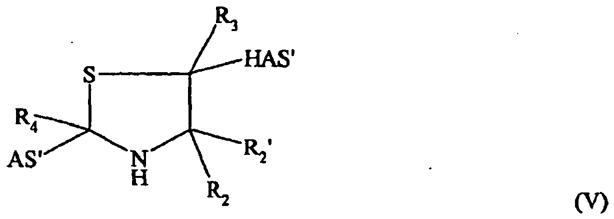 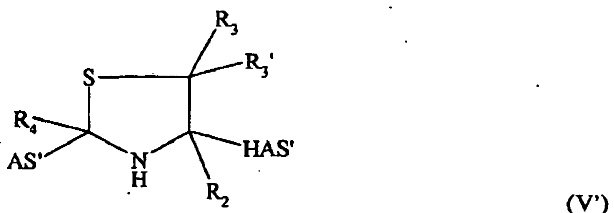 or 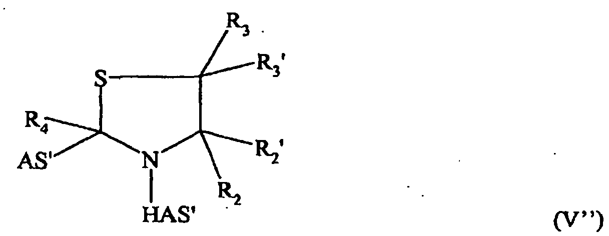 wherein HAS' is the residue of the hydroxyalkyl starch derivative which was linked to the alpha-SH-beta-amino group by a method comprising reacting the hydroxyalkyl starch with at least one, at least bifunctional compound, said compound comprising two functional groups M₁ and Q, one functional group M₁ being reacted with the hydroxyalkyl starch and one functional group Q being
(i) an alpha-SH-beta amino group; or
(ii) a functional group being chemically modified to give the alpha-SH-beta amino group,
and wherein AS' is the residue of the active substance or a derivative thereof which was linked to the aldehyde group, keto group or hemiacetal group.

27. The conjugate of claim 26, wherein the conjugate is selected from the group consisting of 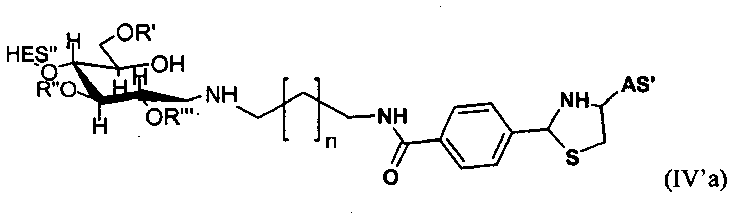 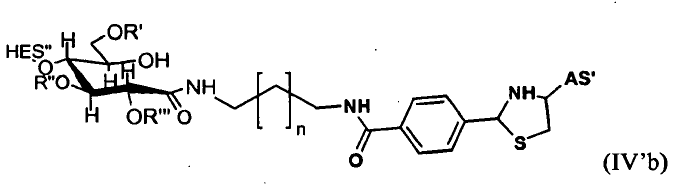 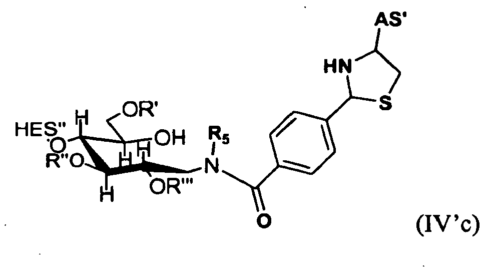 wherein R₅ is as defined for R₁ to R₄ above, 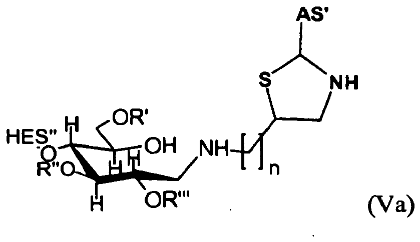 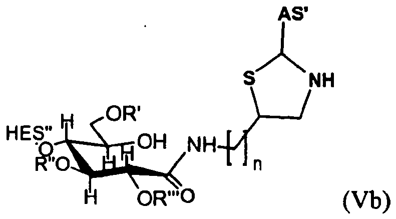 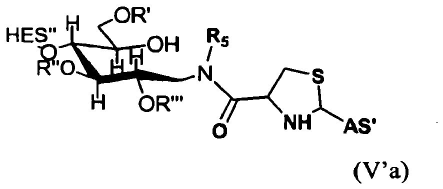 wherein R₅ is as defined for R₁ to R₄ above, 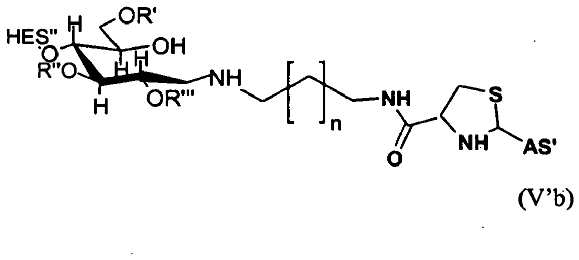 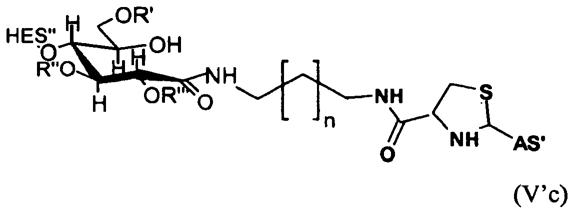 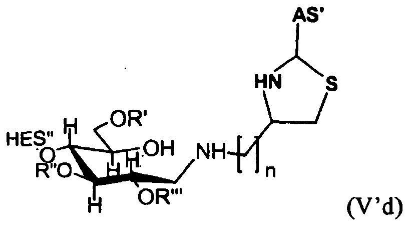 and 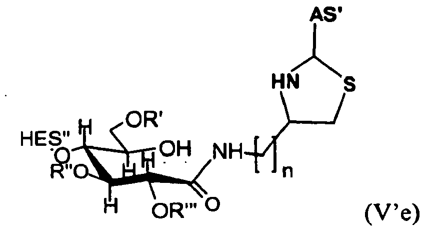 wherein in formula IV'a, IV'b, V'b, or V'c n is an integer, preferably n is 0 to 20, and where in formula Va, Vb, V'd, or V'e n is an integer, preferably n is 1 to 20.

28. The conjugate of claim 26, wherein the conjugate is 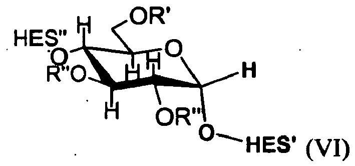 wherein R', R" and/or R"' are as defined for formula II and wherein, in at least one glucose unit of HES, at least one of R', R" and/or R"' is independently selected from the group consisting of 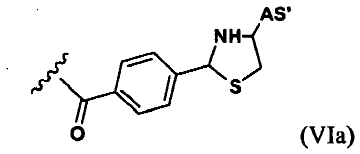 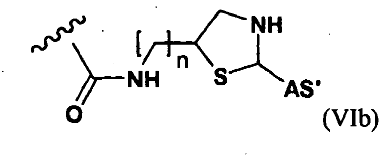 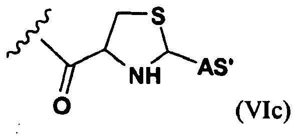 and 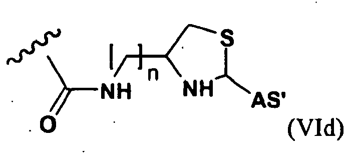 and wherein n is an integer, preferably 1 to 20 and/or wherein at least one of R', R" and/or R"' is -(CH₂CH₂O)ₘ-R^{#}, wherein m is an integer, preferably 1 to 3, and R⁴ is selected from the group consisting of formula (VIa), (VIb), VIc) and (VId).

29. An alpha-SH-beta amino functionalized hydroxyalkyl starch derivative selected from the group consisting of 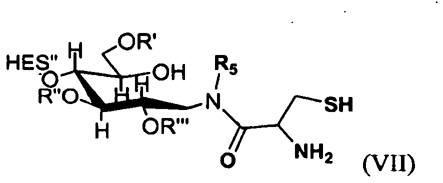 wherein R₅ is as defined for R₁ to R₄ above,
and 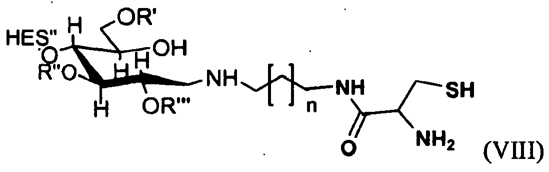 wherein n is an integer, preferably 0 to 20, or the group consisting of 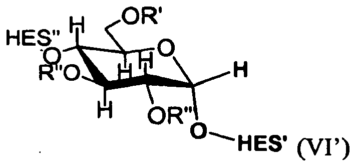 wherein R', R" and/or R"' are as defined for formula II and wherein in at least one glucose unit of HES, at least one of R', R" and/or R"' is independently selected from the group consisting of 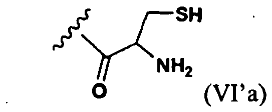 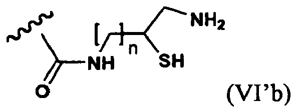 and 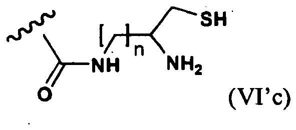 and wherein n is an integer, preferably 1 to 20 and/or wherein at least one of R', R" and/or R"' is -(CH₂CH₂O)ₘ-R^{##}, wherein m is an integer, preferably 1 to 3, and R^{##} is selected from the group consisting of formula (VI'a), (VI'b), and (VI'c).

30. A conjugate of any of claims 25 to 28 for use in a method for the treatment of the human or animal body.

31. A conjugate of any of claims 25 to 28 as a therapeutic agent.

32. A pharmaceutical composition comprising a conjugate of any of claims 25 to 28, preferably further comprising at least one pharmaceutically acceptable diluent, adjuvant, or carrier.

33. A composition comprising a conjugate of an active substance and hydroxyalkyl starch of any of claims 25 to 28.

## Patentansprüche

1. Verfahren zur Herstellung eines Konjugats aus einem Wirkstoff und Hydroxyalkylstärke, wobei der Wirkstoff und die Hydroxyalkylstärke kovalent über einen chemischen Rest der Struktur gemäß der Formel (I) 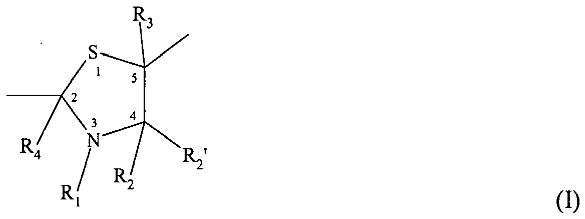 oder der Formel (I') 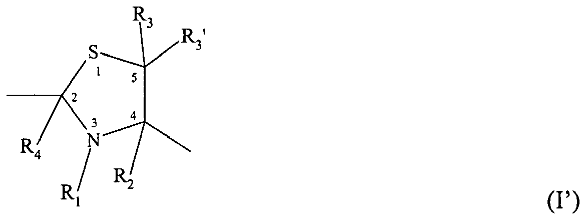 oder der Formel (I") 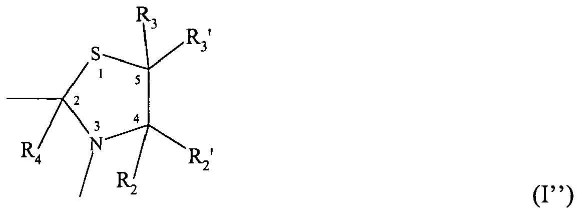 verknüpft sind, wobei R₁, R₂, R_{2'}, R₃, R_{3'} und R₄ unabhängig voneinander ausgewählt sind aus der Gruppe bestehend aus Wasserstoff, einem optional geeignet substituierten, linearen, cyclischen und/oder verzweigten Alkyl, Aryl, Heteroaryl, Aralkyl und Heteroaralkylrest, bevorzugt Wasserstoff,
wobei das Verfahren umfasst:
(A) Reagieren einer Aldehydgruppe, einer Ketogruppe oder einer Halbacetalgruppe eines Hydroxyalkylstärkederivates enthaltend diese Aldehydgruppe, Ketogruppe oder Halbacetalgruppe mit einer alpha-SH-beta-Aminogruppe 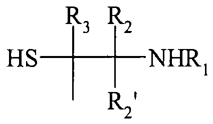 eines Wirkstoffs oder eines Derivates davon, enthaltend diese alpha-SH-beta-Aminogruppe, unter Herstellung eines Konjugates aus diesem Wirkstoff und dieser Hydroxyalkylstärke, welche kovalent über einen chemischen Rest der Struktur gemäß der Formel (I) verknüpft sind;
oder mit einer alpha-SH-beta-Aminogruppe 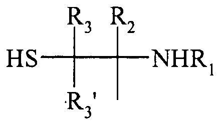 eines Wirkstoffs oder eines Derivates davon, enthaltend diese alpha-SH-beta-Aminogruppe, unter Herstellung eines Konjugates aus diesem Wirkstoff und dieser Hydroxyalkylstärke, welche kovalent über einen chemischen Rest der Struktur gemäß der Formel (I') verknüpft sind,
oder mit einer alpha-SH-beta-Aminogruppe 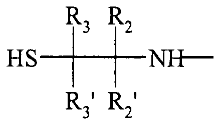 eines Wirkstoffs oder eines Derivates davon, enthaltend diese alpha-SH-beta-Aminogruppe unter Herstellung eines Konjugates aus diesem Wirkstoff und dieser Hydroxyalkylstärke, welche kovalent über einen chemischen Rest der Struktur gemäß der Formel (I") verknüpft sind, wobei R₁, R₂, R₂, R₃, R_{3'} und R₄ wie oben definiert sind;
oder
(B) Reagieren einer Aldehydgruppe, einer Ketogruppe oder einer Halbacetalgruppe eines Wirkstoffs oder eines Derivates davon, enthaltend diese Aldehydgruppe, Ketogruppe oder Halbacetalgruppe mit einer alpha-SH-beta-Aminogruppe 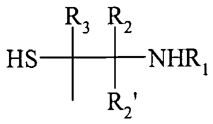 eines Hydroxyalkylstärkederivates enthaltend diese alpha-SH-beta-Aminogruppe, unter Herstellung eines Konjugates aus diesem Wirkstoff und dieser Hydroxyalkylstärke, welche kovalent über einen chemischen Rest der Struktur gemäß der Formel (I) verknüpft sind;
oder mit einer alpha-SH-beta-Aminogruppe 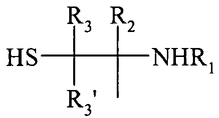 eines Hydroxyalkylstärkederivates enthaltend diese alpha-SH-beta-Aminogruppe, unter Herstellung eines Konjugates aus diesem Wirkstoff und dieser Hydroxyalkylstärke, welche kovalent über einen chemischen Rest der Struktur gemäß der Formel (I') verknüpft sind;
oder mit einer alpha-SH-beta-Aminogruppe 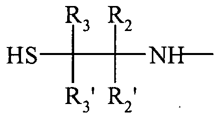 eines Hydroxyalkylstärkederivates enthaltend diese alpha-SH-beta-Aminogruppe, unter Herstellung eines Konjugates aus diesem Wirkstoff und dieser Hydroxyalkylstärke, welche kovalent über einen chemischen Rest der Struktur gemäß der Formel (I") verknüpft sind, wobei R₁, R₂, R_{2'}, R₃, R_{3'} und R₄ wie oben definiert sind, und wobei das Hydroxyalkylstärkederivat enthaltend diese Aldehydgruppe, Ketogruppe, Halbacetalgruppe oder diese alpha-SH-beta-Aminogruppe über ein Verfahren umfassend das Reagieren der Hydroxyalkylstärke mit mindestens einer mindestens bifunktionellen Verbindung erhalten wird, wobei diese Verbindung zwei funktionelle Gruppen M₁ und Q enthält, wobei eine funktionelle Gruppe M₁ mit der Hydroxyalkylstärke umgesetzt wird und eine funktionelle Gruppe Q
(i) eine Aldehydgruppe, Ketogruppe, Halbacetalgruppe oder eine alpha-SH-beta-Aminogruppe; oder
(ii) eine funktionelle Gruppe, die chemisch modifiziert wird, um die Aldehydgruppe, Ketogruppe, Halbacetalgruppe oder die alpha-SH-beta-Aminogruppe zu bilden,
ist.

2. Verfahren nach Anspruch 1, wobei die Hydroxyalkylstärke die folgende Struktur (II) aufweist 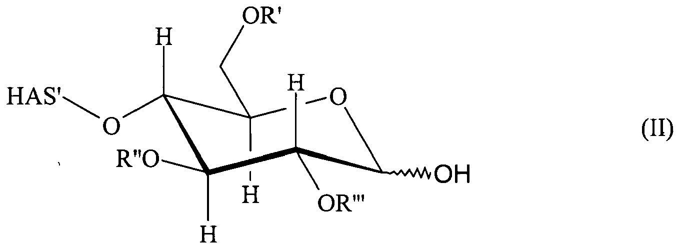 wobei R', R" und R"', unabhängig voneinander, Wasserstoff, eine lineare oder verzweigte Hydroxyalkylgruppe oder die Gruppe
-[(CR¹R²)ₘO]ₙ[CR³R⁴]ₒ-OH
sind, wobei R¹, R², R³ und R⁴, unabhängig voneinander, ausgewählt sind aus der Gruppe bestehend aus Wasserstoff und Alkylgruppe, bevorzugt Wasserstoff und Methylgruppe,
m ist 2 bis 4, wobei die Reste R¹ und R² in den m Gruppen CR¹R² gleich oder verschieden sein können;
n ist 0 bis 20, bevorzugt 0 bis 4;
o ist 0 bis 20, bevorzugt 2-4, wobei, im Falle von n = 0, o ungleich 0 ist, und wobei die Reste R³ und R⁴ in den o Gruppen CR³R⁴ gleich oder verschieden sein können;
wobei bevorzugt R', R" und R"', unabhängig voneinander, Wasserstoff oder eine 2-Hydroxyethylgruppe sind.

3. Verfahren nach Anspruch 1 oder 2, wobei die Hydroxyalkylstärke Hydroxyethylstärke ist, und wobei bevorzugt die Hydroxyethylstärke ein Molekulargewicht von 1 bis 300 kD, weiter bevorzugt von 2 bis 200 kD, weiter bevorzugt von 10 bis 150 kD oder 4 bis 130 kD, weiter bevorzugt von 10 bis 100 kD; und/oder eine molare Substitution von 0,1 bis 3, weiter bevorzugt 0,1 bis 2, weiter bevorzugt 0,1 bis 0,9 oder 0,4 bis 2, weiter bevorzugt 0,4 bis 1,3; und/oder ein Verhältnis zwischen C₂ : C₆-Substitution im Bereich von 2 bis 20 bezogen auf die Hydroxyethylgruppen aufweist.

4. Verfahren gemäß einem der Ansprüche 1 bis 3, wobei der Wirkstoff ausgewählt ist aus der Gruppe bestehend aus Proteinen, Peptiden, kleinen molekularen Wirkstoffen, Glykoproteinen und Oligonukleotiden, wie DNA, RNA, PNA oder Derivaten davon, bevorzugt aus der Gruppe bestehend aus Proteinen, Peptiden und PNA enthaltend eine alpha-SH-beta-Aminogruppe, bevorzugt enthaltend eine Cysteingruppe, insbesondere ein N-terminales Cystein, wobei das Protein bevorzugt ausgewählt ist aus der Gruppe bestehend aus EPO, G-CSF, IFN alpha, IFN beta, AT III, IL-2, IL-3, Myoglobin, SOD, BSA, rhEPO, rhG-CSF, rhIFN alpha, rhIFN beta, rhAT III, rhIL-2, rhIL-3, A1AT, Faktor VII, Faktor VIII, Faktor IX, tPA und APC.

5. Verfahren nach Anspruch 4, wobei der Wirkstoff ausgewählt ist aus der Gruppe bestehend aus Proteinen, Glykoproteinen oder Peptiden enthaltend eine Aldehyd-, Ketogruppe oder Halbacetalgruppe, bevorzugt aus Glykoproteinen, enthaltend einen Aldehyd in einer Glykanseitenkette oder aus synthetischen Peptiden.

6. Verfahren nach einem der vorhergehenden Ansprüche, wobei das Hydroxyalkylstärkederivat 1 bis 100, bevorzugt 1 bis 15, insbesondere 1 Aldehydgruppe(n), Ketogruppe(n) und/oder Halbactealgruppe(n) enthält, oder wobei das Hydroxyalkylstärkederivat 1 bis 100, bevorzugt 1 bis 15, insbesondere 1 alpha-SH-beta-Aminogruppe(n) enthält.

7. Verfahren nach einem der vorhergehenden Ansprüche, wobei der Wirkstoff 1 bis 15, bevorzugt 1 bis 8, insbesondere 1 Aldehydgruppe(n), Ketogruppe(n) und/oder Halbacetalgruppe(n) enthält, oder wobei der Wirkstoff 1 bis 15, bevorzugt 1 bis 8, insbesondere 1 alpha-SH-beta-Aminogruppe(n) enthält.

8. Verfahren nach den Ansprüchen 1 bis 7, wobei die funktionelle Gruppe M₁ mit einer OH-Gruppe der Hydroxyalkylstärke oder dem oxidierten oder nichtoxidierten reduzierenden Ende der Hydroxyalkylstärke reagiert.

9. Verfahren nach den Ansprüchen 1 bis 7, wobei die funktionelle Gruppe M₁ eine Carboxygruppe oder eine reaktive Carboxygruppe ist und die funktionelle Gruppe Q eine Aldehydgruppe, Ketogruppe oder Halbacetalgruppe ist, wobei die bifunktionelle Verbindung, enthaltend M₁ und Q, bevorzugt ausgewählt ist aus der Gruppe bestehend aus Formylbenzoesäure, 4-Formylbenzoesäurepentafluorophenylester, 4-Formylbenzoesäure-N-hydroxysuccinimidester und 4-(4-Formyl-3,5-dimethoxyphenoxy)buttersäure oder eine biokompatible Verbindung ist ausgewählt aus der Gruppe bestehend aus alpha-Ketocarbonsäuren, Neuraminsäuren oder Derivaten davon und Pyridoxalphosphat.

10. Verfahren nach den Ansprüchen 1 bis 7, wobei in (ii) die mindestens bifunktionelle Verbindung eine Aminogruppe M₁ und eine Aminogruppe Q enthält, und wobei die mindestens bifunktionelle Verbindung bevorzugt ein optional substituiertes Diaminoalkan mit 1 bis 20 Kohlenstoffatomen ist, besonders bevorzugt eine Verbindung ausgewählt aus der Gruppe bestehend aus 1,2-Diaminoethan, 1,3-Diaminopropan, und 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,11-Diaminoundecan, 1,12-Diaminododecan, 1,13-Diaminotridecan, 1,14-Diaminotetradecan, 1,15-Diaminopentadecan, 1,16-Diaminohexadecan, 1,17-Diaminoheptadecan, 1,18-Diaminooctadecan, 1,19-Diaminononadecan und 1,20-Diaminoeicosan oder einer Verbindung der Formel 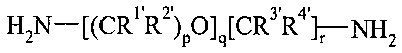 wobei R^{1'}, R^{2'}, R^{3'}, und R^{4'}, unabhängig voneinander, ausgewählt sind aus der Gruppe bestehend aus Wasserstoff und Alkylgruppe, bevorzugt Wasserstoff und Methylgruppe,
p ist 2 bis 4, wobei die Reste R^{1'} und R^{2'} in den p Gruppen CR^{1'}R^{2'} gleich oder verschieden sein können;
q ist 0 bis 20, bevorzugt 0 bis 10;
r ist 0 bis 20, bevorzugt 2-4, wobei, im Falle von q = 0, r ungleich 0 ist, und wobei die Reste R^{3'} und R^{4'} in den r Gruppen CR^{3'}R^{4'} gleich oder verschieden sein können.

11. Verfahren nach Anspruch 10, zusätzlich umfassend das Reagieren des Hydroxyalkylstärkederivates, das aus der Reaktion der Hydroxyalkylstärke mit der mindestens bifunktionellen Verbindung, die zwei Aminogruppen M₁ und Q enthält, erhalten wurde, an der Aminogruppe Q mit einer weiteren bifunktionellen Verbindung, enthaltend eine Aldehydgruppe, Ketogruppe oder Halbacetalgruppe, um ein Hydroxyalkylstärkederivat enthaltend eine Aldehydgruppe, Ketogruppe oder Halbacetalgruppe zu erhalten, wobei die weitere bifunktionelle Verbindung bevorzugt ausgewählt ist aus der Gruppe bestehend aus Formylbenzoesäure, 4-Formylbenzoesäurepentafluorophenylester, 4-Formylbenzoesäure-N-hydroxysuccinimidester, 4-(4-Formyl-3,5-dimethoxyphenoxy)buttersäure und 4-Formylbenzoesäureanhydrid.

12. Verfahren nach Anspruch 10 oder 11, wobei die Hydroxyalkylstärke über ihr optional oxidiertes reduzierendes Ende mit der funktionellen Gruppe M₁ umgesetzt wird, und wobei bevorzugt statistisch mehr als 50 %, weiter bevorzugt mindestens 55 %, weiter bevorzugt mindestens 60 %, weiter bevorzugt mindestens 65 %, weiter bevorzugt mindestens 70 %, weiter bevorzugt mindestens 75 %, weiter bevorzugt mindestens 80 %, weiter bevorzugt mindestens 85 %, weiter bevorzugt mindestens 90 % und noch weiter bevorzugt mindestens 95 %, wie 95 %, 96 %, 97 %, 98 %, oder 99 % der Hydroxyalkylstärkemoleküle, welche für eine gegebene Reaktion eingesetzt werden, über mindestens ein optional oxidiertes reduzierendes Ende pro Hydroxyalkylstärkemolekül, ungesetzt werden.

13. Verfahren nach den Ansprüchen 1 bis 7, wobei in (i) die funktionelle Gruppe M₁ ausgewählt ist aus der Gruppe bestehend aus einer Carboxygruppe, einer reaktiven Carboxygruppe, Carbonsäureanhydrid, Carbonsäurehalogeniden, Isocyanaten, Isothiocyanaten, Chlorameisensäureester und Epoxidgruppen, und die funktionelle Gruppe Q eine Aldehydgruppe, Ketogruppe oder Halbacetalgruppe ist, wobei die funktionelle Gruppe M₁ vorzugsweise über OH-Gruppen auf der Hydroxyalkylstärke reagiert.

14. Verfahren nach den Ansprüchen 1 bis 7, wobei in (i) die funktionelle Gruppe M₁ ausgewählt ist aus der Gruppe bestehend aus einer Aminogruppe und einer alpha-SH-beta-Aminogruppe und die funktionelle Gruppe Q eine alpha-SH-beta-Aminogruppe ist, und wobei die funktionelle Gruppe M₁ vorzugsweise mit dem optional oxidierten reduzierenden Ende der Hydroxyalkylstärke reagiert, wobei bevorzugt statistisch mehr als 50 %, weiter bevorzugt mindestens 55 %, weiter bevorzugt mindestens 60 %, weiter bevorzugt mindestens 65 %, weiter bevorzugt mindestens 70 %, weiter bevorzugt mindestens 75 %, weiter bevorzugt mindestens 80 %, weiter bevorzugt mindestens 85 %, weiter bevorzugt mindestens 90 % und noch weiter bevorzugt mindestens 95 %, wie 95 %, 96 %, 97 %, 98 %, oder 99 % der Hydroxyalkylstärkemoleküle, welche für eine gegebene Reaktion eingesetzt werden, über mindestens ein optional oxidiertes reduzierendes Ende pro Hydroxyalkylstärkemolekül, umgesetzt werden.

15. Verfahren nach den Ansprüchen 1 bis 7, wobei in (i) die Hydroxyalkylstärke, enthaltend die alpha-SH-beta-Aminogruppe, durch ein Verfahren umfassend das Reagieren der Hydroxyalkylstärke an ihrem optional oxidierten reduzierenden Ende mit einer Verbindung, enthaltend eine funktionelle Gruppe M₁ und eine funktionelle Gruppe Q, die eine alpha-SH-beta-Aminogruppe ist, erhalten wird, wobei die Verbindung, enthaltend M₁ und die alpha-SH-beta-Aminogruppe, bevorzugt 1,3-Diamino-2-thiopropan oder 2,3-Diamino-1-thiopropan ist.

16. Verfahren nach den Ansprüchen 1 bis 7, wobei in (ii) die mindestens bifunktionelle Verbindung eine Carboxygruppe oder eine reaktive Carboxygruppe M₁ und eine geschützte alpha-SH-beta-Aminogruppe Q umfasst.

17. Verfahren nach den Ansprüchen 1 bis 7, wobei die mindestens bifunktionelle Verbindung ausgewählt ist aus der Gruppe bestehend aus D-, L-PG₁-Cys(PG₂)-OH oder einer racemischen Mischung davon und ihren aktiven Estern, wobei PG₁ eine geeignete Schutzgruppe für eine Aminogruppe sein kann, bevorzugt ausgewählt aus der Gruppe bestehend aus tert-Butyloxycarbonyl (Boc) oder 9-Fluorenylmethoxycarbonyl (Fmoc), und PG₂ eine geeignete Schutzgruppe für eine Thiolgruppe sein kann, bevorzugt ausgewählt aus der Gruppe bestehend aus Trityl (Trt), p-Methoxytrityl (Mmt), S-tert-Butylthio (S-t-Bu) und Acetamidomethyl (Acm).

18. Verfahren nach den Ansprüchen 1 bis 7, wobei in (ii) die Hydroxyalkylstärke, die die alpha-SH-beta-Aminogruppe enthält, hergestellt ist nach einem Verfahren umfassend die optionale Oxidation der Hydroxyalkylstärke an ihrem reduzierenden Ende, Reagieren des oxidierten oder nichtoxidierten reduzierenden Endes mit einer funktionellen Gruppe M₁ einer Verbindung, enthaltend zusätzlich zu M₁ eine funktionelle Gruppe Q, um ein erstes Hydroxyalkylstärkederivat zu ergeben, und Reagieren der funktionellen Gruppe Q des ersten Hydroxyalkylstärkederivats mit einer funktionellen Gruppe V einer Verbindung enthaltend zusätzlich zu V eine optional geschützte alpha-SH-beta-Aminogruppe, um das optional geschützte alpha-SH-beta-aminofunktionalisierte Hydroxyalkylstärkederivat zu ergeben, und wobei die Verbindung enthaltend M₁ und Q eine Diaminoverbindung oder Carbodiimidazol oder N,N'-Disuccinimidylcarbonat ist, wobei die mindestens bifunktionelle Verbindung bevorzugt ein optional substituiertes Diaminoalkan ist, enthaltend 1 bis 20 Kohlenstoffatome, weiter bevorzugt eine Verbindung ausgewählt aus der Gruppe bestehend aus 1,2-Diaminoethan, 1,3-Diaminopropan und 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,11-Diaminoundecan, 1,12-Diaminododecan, 1,13-Diaminotridecan, 1,14-Diaminotetradecan, 1,15-Diaminopentadecan, 1,16-Diaminohexadecan, 1,17-Diaminoheptadecan, 1,18-Diaminooctadecan, 1,19-Diaminononadecan und 1,20-Diaminoeicosan oder eine Verbindung gemäß der Formel 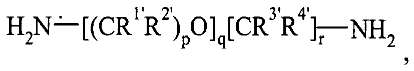 wobei R^{1'}, R^{2'}, R^{3'} und R^{4'}, unabhängig voneinander, ausgewählt sind aus der Gruppe bestehend aus Wasserstoff und Alkylgruppe, bevorzugt Wasserstoff und Methylgruppe,
p ist 2 bis 4, wobei die Reste R^{1'} und R^{2'} in den p Gruppen CR^{1'}R^{2'} gleich oder verschieden sein können,
q ist 0 bis 20, bevorzugt 0 bis 10;
r ist 0 bis 20, bevorzugt 2-4, wobei, im Falle von q = 0, r ungleich 0 ist, und wobei die Reste R^{3'} und R^{4'} in den r Gruppen CR^{3'}R^{4'} gleich oder verschieden sein können,
und wobei die Verbindung enthaltend V und die optional geschützte alpha-SH-beta-Aminogruppe bevorzugt Cystein oder ein Derivat davon ist, V eine Carboxygruppe oder eine reaktive Carboxygruppe ist, bevorzugt ein reaktiver Ester oder ein Carbonsäureanhydrid, oder die Verbindung, enthaltend V, 1,3-Diamino-2-thiopropan oder 2,3-Diamino-1-thiopropan ist.

19. Verfahren nach einem der vorhergehenden Ansprüche, wobei der Wirkstoff, der bevorzugt ein optional modifiziertes Protein, Peptid, synthetisches Peptid oder Oligonukleotid, enthaltend diese Aldehydgruppe, Ketogruppe, Halbacetalgruppe oder diese alpha-SH-beta-Aminogruppe ist, hergestellt ist nach einem Verfahren umfassend
(b)(1)Einführen mindestens einer Aldehydgruppe, Ketogruppe, Halbacetalgruppe oder mindestens einer alpha-SH-beta-Aminogruppe in den Wirkstoff während seiner Herstellung oder durch chemische Modifikation oder
(b)(2)Reagieren des Wirkstoffs mit einer mindestens bifunktionellen Verbindung, die zwei funktionelle Gruppen M₂ und Q enthält, wobei die funktionelle Gruppe M₂ mit dem Wirkstoff reagiert und die funktionellen Gruppe Q
(i) eine Aldehydgruppe, Ketogruppe, Halbacetalgruppe oder eine alpha-SH-beta-Aminogruppe; oder
(ii) eine funktionelle Gruppe, die chemisch modifiziert wird, um die Aldehydgruppe, Ketogruppe, Halbacetalgruppe oder die alpha-SH-beta-Aminogruppe zu bilden,
ist.

20. Verfahren nach Anspruch 19, wobei der Wirkstoff in (b)(1) ein Protein oder Peptid ist, welches mittels organischer Synthese hergestellt wurde, bevorzugt über ein Syntheseharz, welches kompatibel mit einem aldehydfunktionalisierten, ketofunktionalisierten, halbacetalfunktionalisierten oder alpha-SH-betaaminogruppenfunktionalisierten Protein oder Peptid ist, oder wobei in (b)(1) der Wirkstoff ein Protein oder Peptid ist, welches unter Verwendung eines Expressionsvektors mit dem Ergebnis eines aldehydfunktionalisierten, ketofunktionalisierten, halbacetalfunktionalisierten oder alpha-SH-betaaminogruppenfunktionalisierten Proteins oder Peptids hergestellt wurde, oder wobei in (b)(1) der Wirkstoff ein Protein oder ein Peptid ist und das Rückgrat des Proteins oder Peptids mit einer Aldehydgruppe, Ketogruppe, Halbacetalgruppe oder alpha-SH-beta-Aminogruppe substituiert ist, oder wobei in (b)(1) der Wirkstoff ein Protein oder ein Peptid ist, bei dem diese Aldehydgruppe, Ketogruppe, Halbacetalgruppe oder diese alpha-SH-beta-Aminogruppe direkt an das Rückgrat des Proteins oder Peptids gebunden ist oder Teil einer Seitenkette des Rückgrats ist.

21. Verfahren nach Anspruch 19, wobei der Wirkstoff in (b)(1) ein Protein oder Peptid ist und die Aldehydgruppe, Ketogruppe oder Halbacetalgruppe in einem Kohlenhydratrest des Polypeptids enthalten ist, wobei bevorzugt der Kohlenhydratrest ein Derivat eines natürlich vorkommenden Kohlenhydratrestes ist, welcher ausgewählt ist aus der Gruppe bestehend aus Glukose, Galaktose, Mannose und Sialinsäure, welche optional chemisch oder enzymatisch oxidiert sind, bevorzugt ein oxidierter Galaktose- oder ein oxidierter Sialinsäurerest einer Kohlenhydratseitenkette, weiterhin bevorzugt der terminale Galaktose- oder Sialinsäurerest einer Kohlenhydratseitenkette, wobei die Oxidation des terminalen Kohlenhydratrestes bevorzugt entweder enzymatisch oder chemisch ausgeführt wird, wobei eine chemische Oxidation bevorzugt über ein Periodat ausgeführt wird,
oder der Kohlenhydratrest ausgewählt ist aus der Gruppe bestehend aus Hydroxyaldehyden, Hydroxyketonen und chemischen Modifikationen davon,
oder der Kohlenhydratrest ein Derivat eines natürlich vorkommenden Kohlenhydratrestes ist und eine terminale Galaktose ist, welche chemisch oder enzymatisch oxidiert ist, wobei der terminale Galaktoserest optional nach Abspaltung einer terminalen Sialinsäure hergestellt ist.

22. Verfahren nach Anspruch 19, wobei in (b)(2)(i) die alpha-SH-beta-Aminogruppe in einem Cysteinrest des Wirkstoffs enthalten ist, bevorzugt eines Proteins oder Peptids, wobei der Cysteinrest bevorzugt ein N-terminaler Cysteinrest des Wirkstoffs ist.

23. Verfahren nach einem der vorhergehenden Ansprüche, wobei der Wirkstoff ein modifiziertes Protein oder Peptid mit einem N-terminalen Cysteinrest ist, der nicht Teil einer Disulfidbrücke ist, wobei das ein N-terminales Cystein aufweisende, modifizierte Protein oder Peptid bevorzugt eine Mutante eines natürlich vorkommenden Proteins oder Peptids ist, hergestellt durch (1) Hinzufügen eines Cysteinrestes zu einer N-terminalen Aminosäure, (2) Substituieren der N-terminalen Aminosäure mit Cystein oder (3) Deletion der N-terminalen Aminosäure(n), bis ein terminales Cystein erhalten wird.

24. Verfahren nach einem der vorhergehenden Ansprüche, wobei die Reaktion (A) oder (B) ausgeführt wird bei einer Temperatur von 0 bis 40 °C, bevorzugt 0 bis 25 °C, insbesondere 20 bis 25 °C, in Anwesenheit eines Lösungsmittels bei einem pH-Wert von 3,5 bis 10, bevorzugt 4 bis 8, insbesondere 4,8 bis 8,0 mit einer Reaktionszeit von bevorzugt 0,1 bis 24 h, insbesondere etwa 21 h, wobei das Lösungsmittel bevorzugt ausgewählt ist aus der Gruppe bestehend aus Wasser, wässrigem Puffer, DMF, DMSO, DMA und Mischungen davon und/oder wobei das molekulare Verhältnis von Hydroxyalkylstärke zum Wirkstoff ungefähr 1:1 1 bis 200:1, bevorzugt 10:1 bis 100:1, insbesondere 40:1 bis 70:1 ist.

25. Ein Konjugat aus einem Wirkstoff und Hydroxyalkylstärke, herstellbar über ein Verfahren gemäß einem der Ansprüche 1 bis 24.

26. Ein Konjugat aus einem Wirkstoff und Hydroxyalkylstärke, wobei der Wirkstoff und die Hydroxyalkylstärke kovalent über einen chemischen Rest der Struktur gemäß der Formel (I) 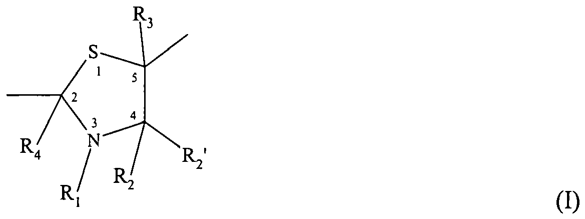 oder der Formel (I') 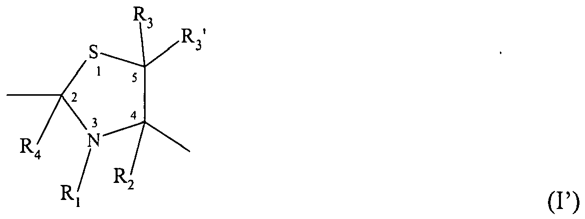 oder der Formel (I") 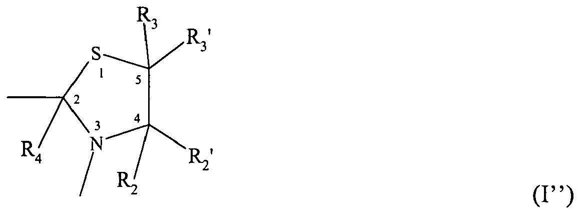 verknüpft sind, wobei R₁, R₂, R_{2'}, R₃, R_{3'} und R₄ unabhängig voneinander ausgewählt sind aus der Gruppe bestehend aus Wasserstoff, einem optional geeignet substituierten, linearen, cyclischen und/oder verzweigten Alkyl, Aryl, Heteroaryl, Aralkyl und einer Heteroaralkylgruppe, bevorzugt Wasserstoff,
wobei dieses Konjugat eine Struktur gemäß der Formel (IV), (IV'), oder (IV") hat 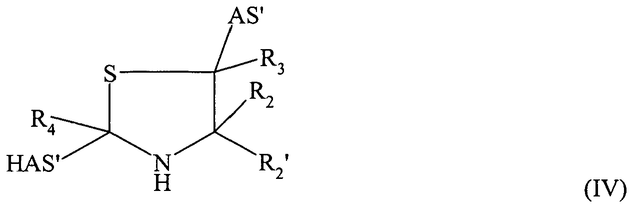 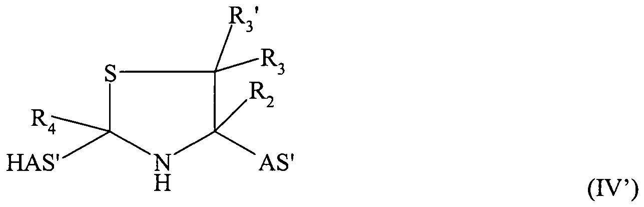 oder 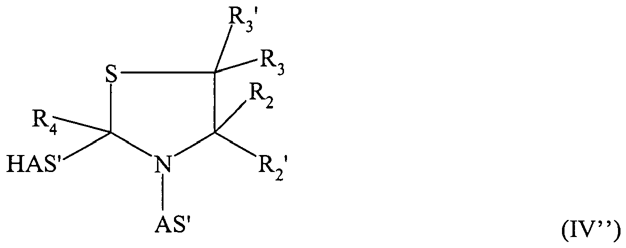 wobei HAS' der Rest des Hydroxyalkylstärkederivats ist, welcher mit einer Aldehydgruppe, Ketogruppe oder Halbacetalgruppe verknüpft wurde durch Reagieren der Hydroxyalkylstärke mit mindestens einer mindestens bifunktionellen Verbindung, wobei diese Verbindung zwei funktionelle Gruppen M₁ und Q enthält, wobei eine funktionelle Gruppe M₁ mit der Hydroxyalkylstärke reagiert und eine funktionelle Gruppe Q
(i) eine Aldehydgruppe, Ketogruppe, Halbacetalgruppe; oder
(ii) eine funktionelle Gruppe, die chemisch modifiziert wird, um eine Aldehydgruppe, Ketogruppe, Halbacetalgruppe zu bilden,
ist, und wobei AS' der Rest des Wirkstoffs oder eines Derivats davon ist, welcher mit der alpha-SH-beta-Aminogruppe verknüpft wurde,
oder eine Struktur gemäß der Formel (V), (V') oder (V") ist 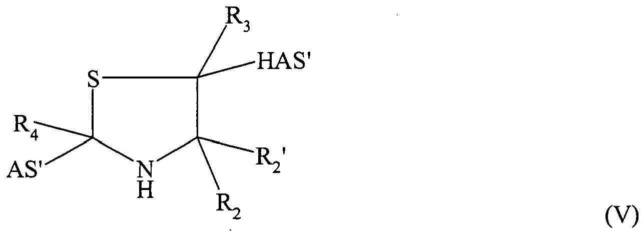 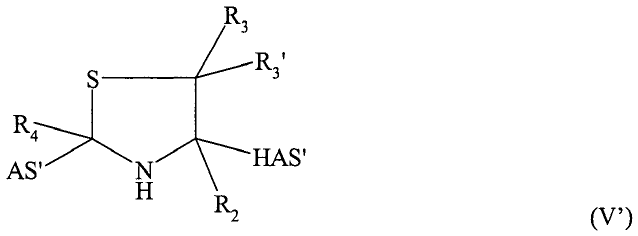 oder 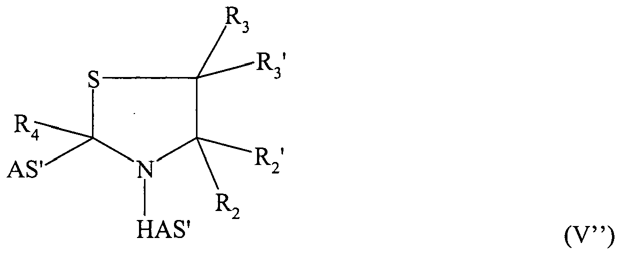 wobei HAS' der Rest des Hydroxyalkylstärkederivats ist, welcher mit der alpha-SH-beta-Aminogruppe über ein Verfahren verknüpft wurde, enthaltend das Reagieren der Hydroxyalkylstärke mit mindestens einer mindestens bifunktionellen Verbindung, wobei die Verbindung zwei funktionelle Gruppen M₁ und Q enthält, wobei eine funktionelle Gruppe M₁ mit der Hydroxyalkylstärke reagiert und eine funktionelle Gruppe Q
(i) eine alpha-SH-beta-Aminogruppe oder
(ii) eine funktionelle Gruppe, die chemisch modifiziert wird, um die alpha-SH-beta-Aminogruppe zu bilden,
ist, und wobei AS' der Rest des Wirkstoffs oder eines Derivates davon ist, der mit der Aldehydgruppe, Ketogruppe oder Halbacetalgruppe verknüpft wurde.

27. Das Konjugat nach Anspruch 26, wobei das Konjugat ausgewählt ist aus der Gruppe bestehend aus 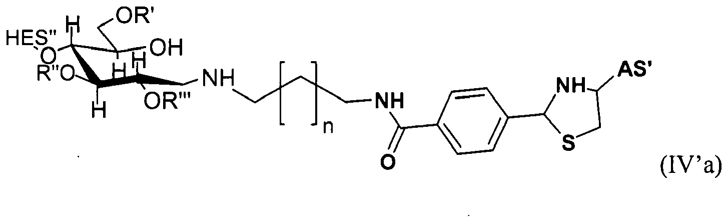 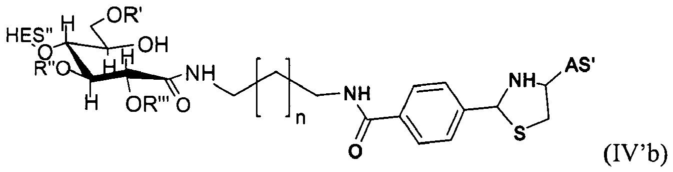 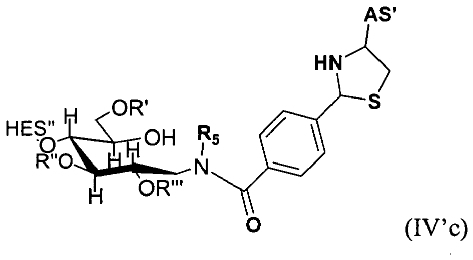 wobei R₅ wie oben für R₁ bis R₄ definiert ist, 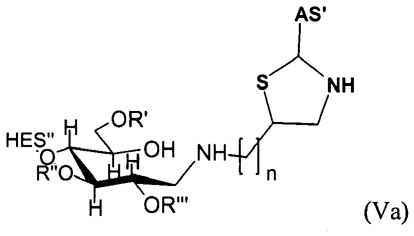 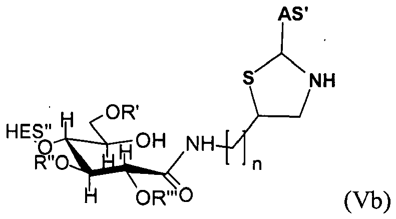 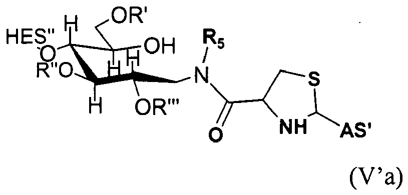 wobei R₅ wie oben für R₁ bis R₄ definiert ist, 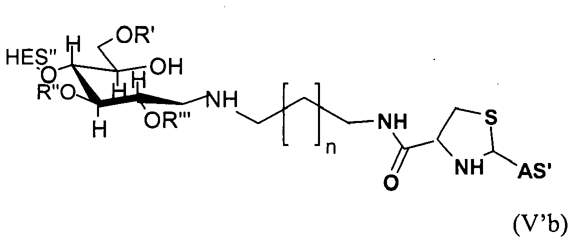 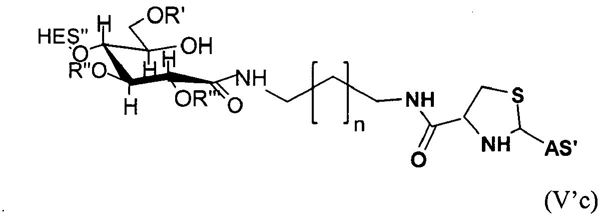 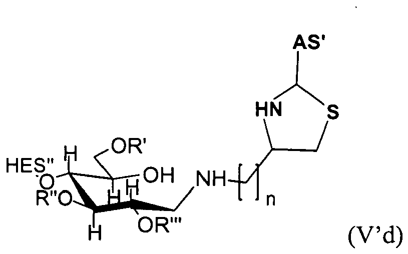 und 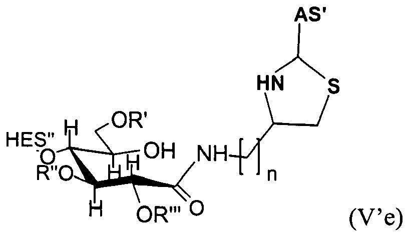 wobei in den Formeln IV'a, IV'b, V'b oder V'c n eine ganze Zahl ist, bevorzugt ist n 0 bis 20, und wobei in den Formeln Va, Vb, V'd oder V'e n eine ganze Zahl ist, bevorzugt ist n 1 bis 20.

28. Das Konjugat nach Anspruch 26, wobei das Konjugat 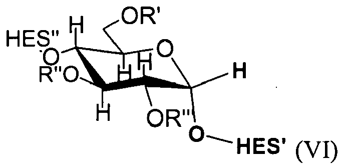 ist, wobei R', R" und/oder R"' wie oben für Formel II definiert sind, und wobei in mindestens einer Glukoseeinheit von HES mindestens einer der Reste R', R" und/oder R'" unabhängig voneinander ausgewählt ist aus der Gruppe bestehend aus 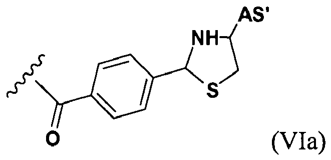 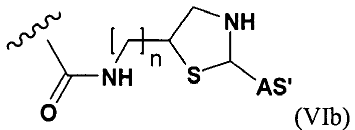 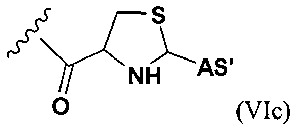 und 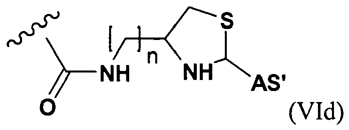 und wobei n eine ganze Zahl ist, bevorzugt 1 bis 20, und/oder wobei mindestens einer der Reste R', R" und/oder R"' -(CH₂CH₂O)ₘ,-R^{#} ist, wobei m eine ganze Zahl ist, bevorzugt 1 bis 3, und R^{#} ausgewählt ist aus der Gruppe bestehend aus den Formeln (VIa), (VIb), (VIc) und (VId).

29. Ein alpha-SH-beta-aminofunktionalisiertes Hydroxyalkylstärkederivat ausgewählt
aus der Gruppe bestehend aus 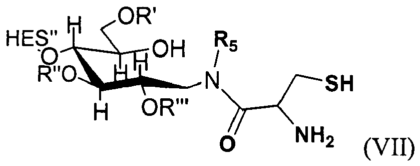 wobei R₅ wie oben für R₁ bis R₄ definiert ist, und 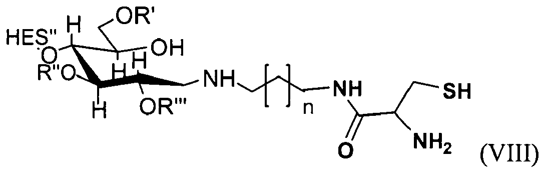 wobei n eine ganze Zahl ist, bevorzugt 0 bis 20, oder eine Gruppe bestehend aus 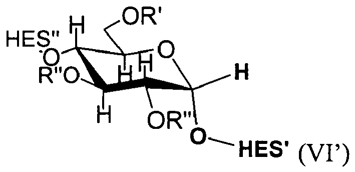 wobei R', R" und/oder R"' wie für Formel II definiert sind, und wobei in mindestens einer Glukoseeinheit von HES mindestens einer der Reste R', R" und/oder R'" unabhängig voneinander ausgewählt ist aus der Gruppe bestehend aus 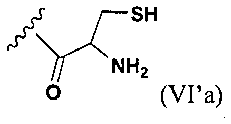 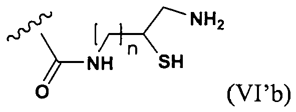 und 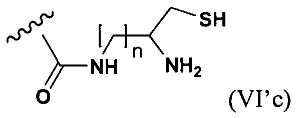 und wobei n eine ganze Zahl ist, bevorzugt 1 bis 20, und/oder wobei mindestens einer der Reste R', R" und/oder R"' -(CH₂CH₂O)ₘ-R^{##} ist, wobei m eine ganze Zahl ist, bevorzugt 1 bis 3, und R^{##} ausgewählt ist aus der Gruppe bestehend aus den Formeln (VI'a), (VI'b) und (VI'c).

30. Ein Konjugat nach einem der Ansprüche 25 bis 28 zur Verwendung in einem Verfahren zur Behandlung eines menschlichen oder tierischen Körpers.

31. Ein Konjugat nach einem der Ansprüche 25 bis 28 als therapeutisches Mittel.

32. Eine pharmazeutische Zusammensetzung enthaltend ein Konjugat nach einem der Ansprüche 25 bis 28, vorzugsweise weiter enthaltend mindestens ein pharmazeutisch geeignetes Verdünnungsmittel, einen Hilfsstoff oder Träger.

33. Eine Zusammensetzung enthaltend ein Konjugat aus einem Wirkstoff und Hydroxyalkylstärke gemäß einem der Ansprüche 25 bis 28.

## Revendications

1. Méthode de préparation d'un conjugué d'une substance active et d'hydroxyalkylamidon, **caractérisée en ce que** la substance active et l'hydroxyalkylamidon sont liés par covalence par un résidu chimique ayant une structure selon la formule (I) 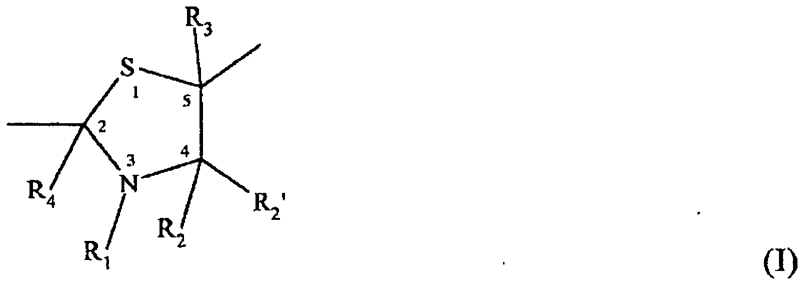 ou la formule (I') 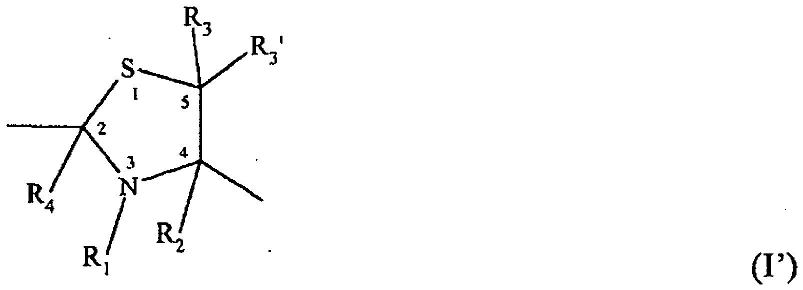 ou la formule (I") 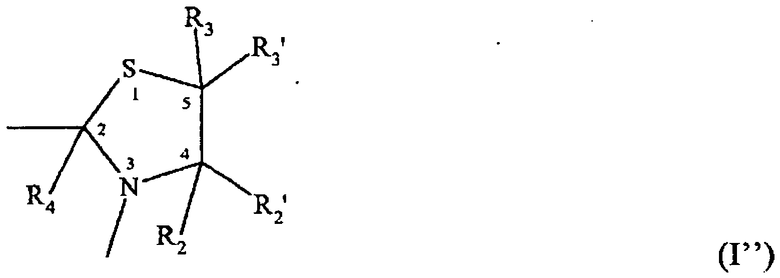 dans lesquelles R₁, R₂, R_{2'}, R₃, R_{3'} et R₄ sont choisis indépendamment dans le groupe constitué d'hydrogène, d'un groupement alkyle, aryle, hétéroaryle, aralkyle et hétéroaralkyle linéaire, cyclique et/ou ramifié, éventuellement substitué de façon appropriée, de préférence hydrogène,
ladite méthode comprenant
(A) la réaction d'un groupement aldéhyde, d'un groupement céto ou d'un groupement hémiacétal d'un dérivé d'hydroxyalkylamidon comprenant ledit groupement aldéhyde, ledit groupement céto ou ledit groupement hémiacétal avec un groupement alpha-SH-bêta-amino 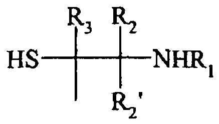 d'une substance active ou d'un dérivé de celle-ci comprenant ledit groupement alpha-SH-bêta-amino, préparant ainsi un conjugué de ladite substance active et dudit hydroxyalkylamidon étant liés par covalence par un résidu chimique ayant une structure selon la formule (I) ;
ou avec un groupement alpha-SH-bêta-amino 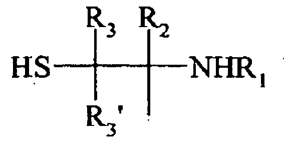 d'une substance active ou d'un dérivé de celle-ci comprenant ledit groupement alpha-SH-bêta-amino, préparant ainsi un conjugué de ladite substance active et dudit hydroxyalkylamidon étant liés par covalence par un résidu chimique ayant une structure selon la formule (I') ;
ou avec un groupement alpha-SH-bêta-amino 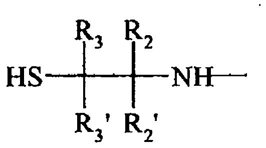 d'une substance active ou d'un dérivé de celle-ci comprenant ledit groupement alpha-SH-bêta-amino, préparant ainsi un conjugué de ladite substance active et dudit hydroxyalkylamidon étant liés par covalence par un résidu chimique ayant une structure selon la formule (I"), où R₁, R₂, R_{2'}, R₃, R_{3'} et R₄ sont tels que définis ci-dessus ;
ou
(B) la réaction d'un groupement aldéhyde, d'un groupement céto ou d'un groupement hémiacétal d'une substance active ou d'un dérivé de celle-ci comprenant ledit groupement aldéhyde, ledit groupement céto ou ledit groupement hémiacétal avec un groupement alpha-SH-bêta-amino 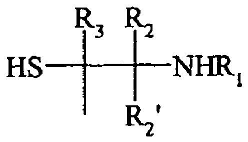 d'un dérivé d'hydroxyalkylamidon comprenant ledit groupement alpha-SH-bêta-amino, préparant ainsi un conjugué de ladite substance active et dudit hydroxyalkylamidon étant liés par covalence par un résidu chimique ayant une structure selon la formule (I) ;
ou avec un groupement alpha-SH-bêta-amino 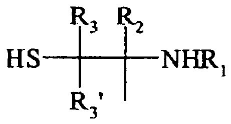 d'un dérivé d'hydroxyalkylamidon comprenant ledit groupement alpha-SH-bêta-amino, préparant ainsi un conjugué de ladite substance active et dudit hydroxyalkylamidon étant liés par covalence par un résidu chimique ayant une structure selon la formule (I') ;
ou avec un groupement alpha-SH-bêta-amino 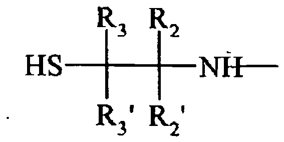 d'un dérivé d'hydroxyalkylamidon comprenant ledit groupement alpha-SH-bêta-amino, préparant ainsi un conjugué de ladite substance active et dudit hydroxyalkylamidon étant liés par covalence par un résidu chimique ayant une structure selon la formule (I"), où R₁, R₂, R_{2'}, R₃, R_{3'} et R₄ sont tels que définis ci-dessus
et où le dérivé d'hydroxyalkylamidon comprenant ledit groupement aldéhyde, ledit groupement céto, ledit groupement hémiacétal ou ledit groupement alpha-SH-bêta-amino est obtenu par une méthode comprenant la réaction de l'hydroxyalkylamidon avec au moins un composé au moins bifonctionnel, ledit composé comprenant deux fonctions M₁ et Q, une fonction M₁ étant mise en réaction avec l'hydroxyalkylamidon et une fonction Q étant
(i) un groupement aldéhyde, un groupement céto, un groupement hémiacétal ou un groupement alpha-SH-bêta-amino ; ou
(ii) une fonction étant chimiquement modifiée pour donner le groupement aldéhyde, le groupement céto, le groupement hémiacétal ou le groupement alpha-SH-bêta-amino.

2. Méthode selon la revendication 1, **caractérisée en ce que** l'hydroxyalkylamidon a la structure (II) suivante 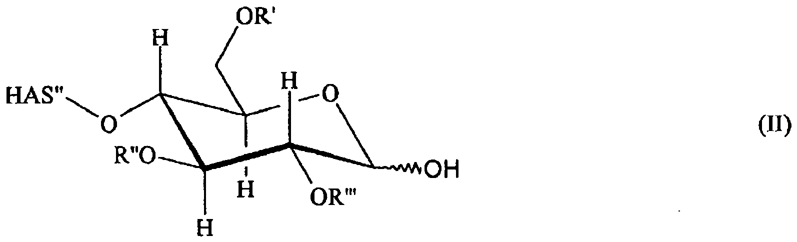 dans laquelle R', R" et R"' sont indépendamment hydrogène, un groupement hydroxyalkyle linéaire ou ramifié ou le groupement
-[(CR¹R²)ₘO]ₙ[CR³R⁴]ₒ-OH
dans lequel R¹, R², R³ et R⁴ sont choisis indépendamment dans le groupe constitué d'hydrogène et d'un groupement alkyl, de préférence hydrogène et un groupement méthyle,
m est 2 à 4, les résidus R¹ et R² pouvant être identiques ou différents dans les m groupements CR¹R² ;
n est 0 à 20, de préférence 0 à 4 ;
o est 0 à 20, de préférence 2-4, où dans le cas de n = 0, o n'est pas 0, et où les résidus R³ et R⁴ peuvent être identiques ou différents dans les o groupements CR³R⁴ ;
où, de préférence, R', R" et R"' sont indépendamment hydrogène ou un groupement 2-hydroxyéthyle.

3. Méthode selon la revendication 1 ou 2, **caractérisée en ce que** l'hydroxyalkylamidon est l'hydroxyéthylamidon, et où, de préférence, l'hydroxyéthylamidon a un poids moléculaire de 1 à 300 kD, plus préférablement de 2 à 200 kD, plus préférablement de 10 à 150 kD ou 4 à 130 kD, plus préférablement de 10 à 100 kD ; et/ou une substitution molaire de 0,1 à 3, plus préférablement 0,1 à 2, plus préférablement 0,1 à 0,9 ou 0,4 à 2, plus préférablement 0,4 à 1,3 ; et/ou un rapport entre la substitution C₂:C₆ dans la gamme de 2 à 20 par rapport aux groupements hydroxyéthyle.

4. Méthode selon l'une quelconque des revendications 1 à 3, **caractérisée en ce que** la substance active est choisie dans le groupe constitué de protéines, peptides, médicaments à petites molécules, glycoprotéines et oligonucléotides, tels que l'ADN, l'ARN, l'ANP ou des dérivés de ceux-ci, de préférence dans le groupe constitué de protéines, peptides et ANP comprenant un groupement alpha-SH-bêta-amino, de préférence comprenant un groupement cystéine, en particulier une cystéine N-terminale, la protéine étant choisie de préférence dans le groupe constitué de EPO, G-CSF, IFN alpha, IFN bêta, AT III, IL-2, IL-3, myoglobine, SOD, BSA, rhEPO, rhG-CSF, rhIFN alpha, rtIFN bêta, rhAT III, rhIL-2, rhIL-3, A1AT, facteur VII, facteur VIII, facteur IX, tPA et APC.

5. Méthode selon la revendication 4, **caractérisée en ce que** la substance active est choisie dans le groupe constitué de protéines, glycoprotéines ou peptides comprenant un aldéhyde, un groupement céto ou un groupement hémiacétal, de préférence de glycoprotéines contenant un aldéhyde dans une chaîne latérale glycane ou de peptides de synthèse.

6. Méthode selon l'une quelconque des revendications précédentes, **caractérisée en ce que** le dérivé d'hydroxyalkylamidon comprend de 1 à 100, de préférence de 1 à 15, en particulier 1 groupement(s) aldéhyde, groupement(s) céto et/ou groupement(s) hémiacétal ou **en ce que** le dérivé d'hydroxyalkylamidon comprend de 1 à 100, de préférence de 1 à 15, en particulier 1 groupement(s) alpha-SH-bêta-amino.

7. Méthode selon l'une quelconque des revendications précédentes, **caractérisée en ce que** la substance active comprend de 1 à 15, de préférence de 1 à 8, en particulier 1 groupement(s) aldéhyde, groupement(s) céto et/ou groupement(s) hémiacétal ou **en ce que** la substance active comprend de 1 à 15, de préférence de 1 à 8, en particulier 1 groupement(s) alpha-SH-bêta-amino.

8. Méthode selon les revendications 1 à 7, **caractérisée en ce que** la fonction M₁ est mise en réaction avec un groupement OH sur l'hydroxyalkylamidon ou l'extrémité réductrice non oxydée ou oxydée de l'hydroxyalkylamidon.

9. Méthode selon les revendications 1 à 7, **caractérisée en ce que** la fonction M₁ est un groupement carboxy ou un groupement carboxy réactif et la fonction Q est un groupement aldéhyde, un groupement céto ou un groupement hémiacétal, **en ce que** le composé bifonctionnel comprenant M₁ et Q est choisi de préférence dans le groupe constitué d'acide formylbenzoïque, d'ester pentafluorophénylique d'acide 4-formylbenzoïque, d'ester de N-hydroxysuccinimide d'acide 4-formylbenzoïque et d'acide 4-(4-formyl-3,5-diméthoxyphénoxy)butyrique ou un composé biocompatible choisi dans le groupe constitué d'acides alpha-céto-carboxyliques, d'acides neuraminiques ou de dérivés de ceux-ci et de pyridoxal phosphate.

10. Méthode selon les revendications 1 à 7, **caractérisée en ce que** dans (ii), le composé au moins bifonctionnel comprend un groupement amino M₁ et un groupement amino Q, et **en ce que** le composé au moins bifonctionnel est de préférence un diaminoalcane éventuellement substitué ayant de 1 à 20 atomes de carbone, plus préférablement un composé étant choisi dans le groupe constitué de 1,2-diaminoéthane, 1,3-diaminopropane, et 1,4-diaminobutane, 1,5-diaminopentane, 1,6-diaminohexane, 1,7-diaminoheptane, 1,8-diaminooctane, 1,9-diaminononane, 1,10-diaminodécane, 1,11-diaminoundécane, 1,12-diaminododécane, 1,13-diaminotridécane, 1,14-diaminotétradécane, 1,15-diaminopentadécane, 1,16-diaminohexadécane, 1,17-diaminoheptadécane, 1,18-diaminooctadécane, 1,19-diaminononadécane, et 1,20-diaminoeicosane ou un composé ayant la formule
H₂N-[(CR^{1'}R^{2'})ₚO]_{q}[CR^{3'}R^{4'}]ᵣ-NH₂
dans laquelle R^{1'}, R^{2'}, R^{3'} et R^{4'} sont choisis indépendamment dans le groupe constitué d'hydrogène et d'un groupement alkyle, de préférence hydrogène et un groupement méthyle,
p est 2 à 4, les résidus R^{1'} et R^{2'} pouvant être identiques ou différents dans les p groupements CR^{1'}R^{2'},
q est 0 à 20, de préférence 0 à 10 ;
r est 0 à 20, de préférence 2-4, où dans le cas de q = 0, r n'est pas 0, et où les résidus R^{3'} et R^{4'} peuvent être identiques ou différents dans les r groupements CR^{3'}R^{4'}.

11. Méthode selon la revendication 10, comprenant en outre la réaction du dérivé d'hydroxyalkylamidon, provenant de la réaction de l'hydroxyalkylamidon avec le composé au moins bifonctionnel comprenant deux groupements amino M₁ et Q, au niveau du groupement amino Q avec un autre composé bifonctionnel comprenant un groupement aldéhyde, un groupement céto ou un groupement hémiacétal pour donner un dérivé d'hydroxyalkylamidon ayant un groupement aldéhyde, un groupement céto ou un groupement hémiacétal, l'autre composé bifonctionnel étant choisi de préférence dans le groupe constitué d'acide formylbenzoïque, d'ester pentafluorophénylique d'acide 4-formylbenzoïque, d'ester de N-hydroxysuccinimide d'acide 4-formylbenzoïque, d'acide 4-(4-formyl-3,5-diméthoxyphénoxy)butyrique et d'anhydride d'acide 4-formylbenzoïque.

12. Méthode selon la revendication 10 ou 11, **caractérisée en ce que** l'hydroxyalkylamidon est mis en réaction par l'intermédiaire de son extrémité réductrice éventuellement oxydée avec la fonction m₁, et **en ce que** de préférence, statistiquement plus de 50 %, plus préférablement au moins 55 %, plus préférablement au moins 60 %, plus préférablement au moins 65 %, plus préférablement au moins 70 %, plus préférablement au moins 75 %, plus préférablement au moins 80 %, plus préférablement au moins 85 %, plus préférablement au moins 90 %, et encore plus préférablement au moins 95 %, par exemple 95 %, 96 %, 97 %, 98 % ou 99 %, des molécules d'hydroxyalkylamidon utilisées pour une réaction donnée sont mises en réaction par l'intermédiaire d'au moins une extrémité réductrice éventuellement oxydée par molécule d'hydroxyalkylamidon.

13. Méthode selon les revendications 1 à 7, **caractérisée en ce que** dans (i), la fonction M₁ est choisie dans le groupe constitué d'un groupement carboxy, d'un groupement carboxy réactif, d'anhydride d'acide carboxylique, d'halogénure d'acide carboxylique, d'isocyanate, d'isothiocyanate, d'ester d'acide chloroformique, et de groupements époxyde et la fonction Q est un groupement aldéhyde, un groupement céto ou un groupement hémiacétal, la fonction M₁ étant de préférence mise en réaction avec des groupements OH sur l'hydroxyalkylamidon.

14. Méthode selon les revendications 1 à 7, **caractérisée en ce que** dans (i), la fonction M₁ est choisie dans le groupe constitué d'un groupement amino et d'un groupement alpha-SH-bêta-amino et la fonction Q est un groupement alpha-SH-bêta-amino, la fonction M₁ étant de préférence mise en réaction avec l'extrémité réductrice éventuellement oxydée de l'hydroxyalkylamidon, **en ce que** de préférence, statistiquement plus de 50 %, plus préférablement au moins 55 %, plus préférablement au moins 60 %, plus préférablement au moins 65 %, plus préférablement au moins 70 %, plus préférablement au moins 75 %, plus préférablement au moins 80 %, plus préférablement au moins 85 %, plus préférablement au moins 90 %, et encore plus préférablement au moins 95 %, par exemple 95 %, 96 %, 97 %, 98 % ou 99 %, des molécules d'hydroxyalkylamidon utilisées pour une réaction donnée sont mises en réaction par l'intermédiaire d'au moins une extrémité réductrice éventuellement oxydée par molécule d'hydroxyalkylamidon.

15. Méthode selon les revendications 1 à 7, **caractérisée en ce que** dans (i), l'hydroxyalkylamidon comprenant ledit groupement alpha-SH-bêta-amino est obtenu par une méthode comprenant la réaction de l'hydroxyalkylamidon au niveau de l'extrémité réductrice éventuellement oxydée avec un composé comprenant une fonction M₁ et une fonction Q étant un groupement alpha-SH-bêta-amino, le composé comprenant M₁ et le groupement alpha-SH-bêta-amino étant de préférence le 1,3-diamino-2-thiopropane ou le 2,3-diamino-1-thiopropane.

16. Méthode selon les revendications 1 à 7, **caractérisée en ce que** dans (ii) le composé au moins bifonctionnel comprend M₁ étant un groupement carboxy ou un groupement carboxy réactif et Q étant un groupement alpha-SH-bêta-amino protégé.

17. Méthode selon les revendications 1 à 7, **caractérisée en ce que** le composé au moins bifonctionnel est choisi dans le groupe constitué de D-, L-PG₁-Cys(PG₂)-OH, ou un mélange racémique de ceux-ci, et leur ester actif, PG₁ pouvant être tout groupement protecteur convenable pour un groupement amino, de préférence choisi dans le groupe constitué de tert-butyloxycarbonyle (Boc) ou 9-fluorénylméthoxycarbonyle (Fmoc), et PG₂ pouvant être tout groupement protecteur convenable pour un groupement thiol, de préférence choisi dans le groupe constitué de trityle (Trt), p-méthoxytrityle (Mmt), S-tert-butylthio (S-t-Bu) et acétamidométhyle (Acm).

18. Méthode selon les revendications 1 à 7, **caractérisée en ce que** dans (ii), l'hydroxyalkylamidon comprenant ledit groupement alpha-SH-bêta-amino est obtenu par une méthode comprenant l'oxydation éventuelle de l'hydroxyalkylamidon à son extrémité réductrice, la réaction de l'extrémité réductrice non oxydée ou oxydée avec une fonction M₁ d'un composé comprenant, outre M₁, une autre fonction Q, pour donner un premier dérivé d'hydroxyalkylamidon, et la réaction de la fonction Q du premier dérivé d'hydroxyalkylamidon avec une fonction V d'un composé comprenant, outre V, un groupement alpha-SH-bêta-amino éventuellement protégé, pour donner le dérivé d'hydroxyalkylamidon fonctionnalisé par alpha-SH-bêta-amino éventuellement protégé, et le composé comprenant M₁ et Q étant un composé diamino ou le carbodiimidazole ou le carbonate de N,N'-disuccinimidyle, le composé au moins bifonctionnel étant de préférence un diaminoalcane éventuellement substitué ayant de 1 à 20 atomes de carbone, plus préférablement un composé étant choisi dans le groupe constitué de 1,2-diaminoéthane, 1,3-diaminopropane, et 1,4-diaminobutane, 1,5-diaminopentane, 1,6-diaminohexane, 1,7-diaminoheptane, 1,8-diaminooctane, 1,9-diaminononane, 1,10-diaminodécane, 1,11-diaminoundécane, 1,12-diaminododécane, 1,13-diaminotridécane, 1,14-diaminotétradécane, 1,15-diaminopentadécane, 1,16-diaminohexadécane, 1,17-diaminoheptadécane, 1,18-diaminooctadécane, 1,19-diaminononadécane, et 1,20-diaminoeicosane ou un composé ayant la formule
H₂N-[(CR^{1'}R^{2'})ₚO]_{q}[CR^{3'}R^{4'}]ᵣ-NH₂
dans laquelle R^{1'}, R^{2'}, R^{3'} et R^{4'} sont choisis indépendamment dans le groupe constitué d'hydrogène et d'un groupement alkyle, de préférence hydrogène et un groupement méthyle,
p est 2 à 4, les résidus R^{1'} et R^{2'} pouvant être identiques ou différents dans les p groupements CR^{1'}R^{2'},
q est 0 à 20, de préférence 0 à 10 ;
r est 0 à 20, de préférence 2-4, où dans le cas de q = 0, r n'est pas 0, et où les résidus R^{3'} et R^{4'} peuvent être identiques ou différents dans les r groupements CR^{3'}R^{4'} ;
et où le composé comprenant V et le groupement alpha-SH-bêta-amino éventuellement protégé est de préférence la cystéine ou un dérivé de celle-ci, V étant un groupement carboxy ou un groupement carboxy réactif, de préférence un ester réactif ou un anhydride d'acide carboxylique ou le composé comprenant V est le 1,3-diamino-2-thiopropane ou le 2,3-diamino-1-thiopropane.

19. Méthode selon l'une quelconque des revendications précédentes, **caractérisée en ce que** la substance active, de préférence une protéine, un peptide, un peptide de synthèse ou un oligonucléotide éventuellement modifié(e), comprenant ledit groupement aldéhyde, ledit groupement céto, ledit groupement hémiacétal ou ledit groupement alpha-SH-bêta-amino est obtenue par une méthode comprenant
(b)(1) l'introduction d'au moins un groupement aldéhyde, groupement céto, groupement hémiacétal ou au moins un groupement alpha-SH-bêta-amino dans la substance active pendant sa préparation ou par modification chimique, ou
(b)(2) la réaction de la substance active avec un composé au moins bifonctionnel, ledit composé comprenant deux fonctions M₂ et Q, une fonction M₂ étant mise en réaction avec la substance active et une fonction Q étant
(i) un groupement aldéhyde, un groupement céto, un groupement hémiacétal ou un groupement alpha-SH-bêta-amino ; ou
(ii) une fonction étant chimiquement modifiée pour donner le groupement aldéhyde, le groupement céto, le groupement hémiacétal ou un groupement alpha-SH-bêta-amino.

20. Méthode selon la revendication 19, **caractérisée en ce que** dans (b)(1), la substance active est une protéine ou un peptide qui a été préparé par synthèse organique, de préférence produit en utilisant une résine de synthèse, permettant d'obtenir une protéine ou un peptide à fonction aldéhyde, à fonction céto, à fonction hémiacétal ou à fonction alpha-SH-bêta-amino, ou **en ce que** dans (b)(1), la substance active est une protéine ou un peptide qui a été produit en utilisant un vecteur d'expression conduisant à une protéine ou un peptide à fonction aldéhyde, à fonction céto, à fonction hémiacétal ou à fonction alpha-SH-bêta-amino, ou **en ce que** dans (b)(1), la substance active est une protéine ou un peptide et le squelette de la protéine ou du peptide est substitué par un groupement aldéhyde, un groupement céto, un groupement hémiacétal ou un groupement alpha-SH-bêta-amino, ou **en ce que** dans (b)(1), la substance active est une protéine ou un peptide où ledit groupement aldéhyde, ledit groupement céto, ledit groupement hémiacétal ou ledit groupement alpha-SH-bêta-amino est lié directement au squelette de la protéine ou du peptide ou fait partie d'une chaîne latérale du squelette.

21. Méthode selon la revendication 19, **caractérisée en ce que** dans (b)(1) la substance active est une protéine ou un peptide et le groupement aldéhyde, le groupement céto ou le groupement hémiacétal est contenu dans un motif glucide du polypeptide, où, de préférence,
le motif glucide est un dérivé d'un motif glucide naturel et est choisi dans le groupe constitué de glucose, de galactose, de mannose et d'acide sialique, qui sont éventuellement oxydés par voie chimique ou enzymatique, de préférence un résidu de galactose oxydé ou un résidu d'acide sialique oxydé d'une chaîne latérale glucidique, plus préférablement le résidu de galactose ou d'acide sialique terminal d'une chaîne latérale glucidique, l'oxydation d'un motif glucide terminal étant de préférence effectuée soit par voie enzymatique soit par voie chimique, une oxydation chimique étant effectuée de préférence en utilisant un periodate,
ou le motif glucide est choisi dans le groupe constitué d'hydroxyaldéhydes, d'hydroxycétones et de modifications chimiques de ceux-ci,
ou le motif glucide est un dérivé d'un motif glucide naturel et est un galactose terminal, qui est oxydé par voie chimique ou enzymatique, le résidu galactose terminal étant éventuellement obtenu après clivage d'un acide sialique terminal.

22. Méthode selon la revendication 19, **caractérisée en ce que** dans (b)(2)(i), le groupement alpha-SH-bêta-amino est contenu dans un résidu cystéine de la substance active, de préférence une protéine ou un peptide, le résidu cystéine étant de préférence un résidu cystéine N-terminal de la substance active.

23. Méthode selon l'une quelconque des revendications précédentes, **caractérisée en ce que** la substance active est une protéine ou un peptide modifié avec un résidu cystéine N-terminal, qui ne fait pas partie d'un pont disulfure, la protéine ou le peptide modifié possédant un résidu cystéine N-terminal étant de préférence un mutant d'une protéine ou d'un peptide naturel, obtenu par (1) l'addition d'un résidu cystéine à l'acide aminé N-terminal, (2) la substitution de l'acide aminé N-terminal par la cystéine, ou par (3) la délétion du ou des acides aminés N-terminaux jusqu'à l'obtention d'une cystéine terminale.

24. Méthode selon l'une quelconque des revendications précédentes, **caractérisée en ce que** la réaction (A) ou (B) est effectuée à une température de 0 à 40°C, de préférence de 0 à 25°C, en particulier de 20 à 25°C, en présence d'un solvant, et à un pH de 3,5 à 10, de préférence de 4 à 8, en particulier de 4,8 à 8,0, avec un temps de réaction de préférence de 0,1 à 24 h, en particulier d'environ 21 h, de préférence le solvant étant choisi dans le groupe constitué d'eau, de tampon aqueux, de DMF, DMSO, DMA et de mélanges de ceux-ci et/ou le rapport moléculaire entre l'hydroxyalkylamidon et la substance active étant d'environ 1:1 à 200:1, de préférence de 10:1 à 100:1, en particulier de 40:1 à 70:1.

25. Conjugué d'une substance active et d'hydroxyalkylamidon, tel qu'il est susceptible d'être obtenu par une méthode selon l'une quelconque des revendications 1 à 24.

26. Conjugué d'une substance active et d'hydroxyalkylamidon, **caractérisé en ce que** la substance active et l'hydroxyalkylamidon sont liés par covalence par un résidu chimique ayant une structure selon la formule (I) 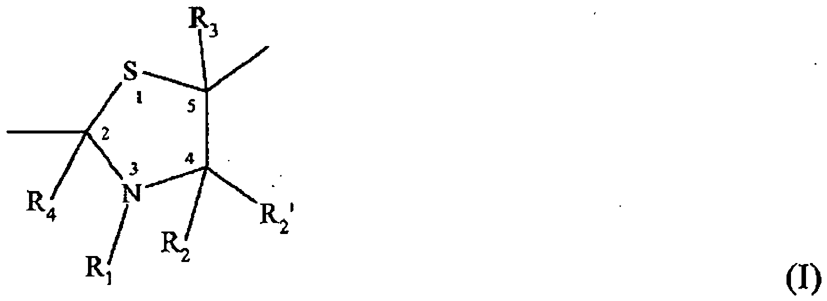 ou la formule (I') 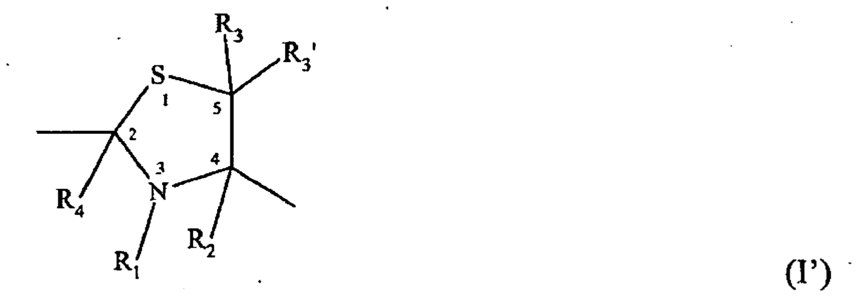 ou la formule (I") 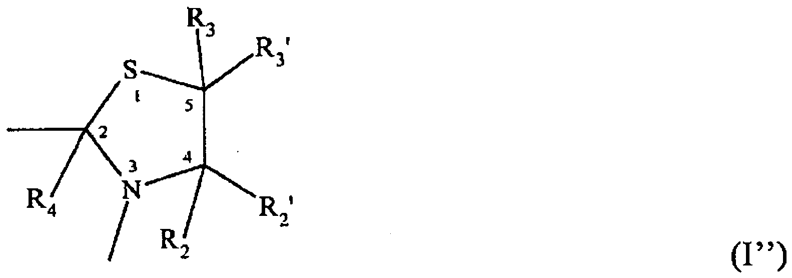 dans lesquelles R₁, R₂, R_{2'}, R₃, R₃, et R₄ sont choisis indépendamment dans le groupe constitué d'hydrogène, d'un groupement alkyle, aryle, hétéroaryle, aralkyle et hétéroaralkyle linéaire, cyclique et/ou ramifié, éventuellement substitué de façon appropriée, de préférence hydrogène, ledit conjugué ayant une structure selon la formule (IV), (IV') ou (IV") 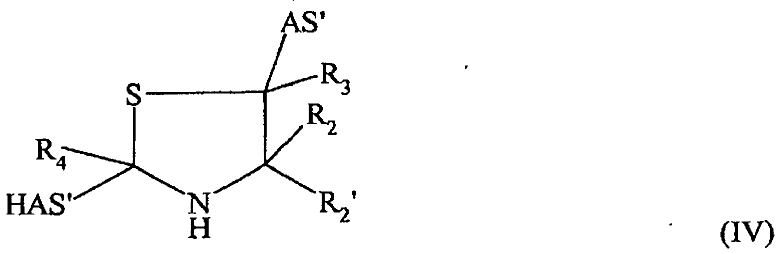 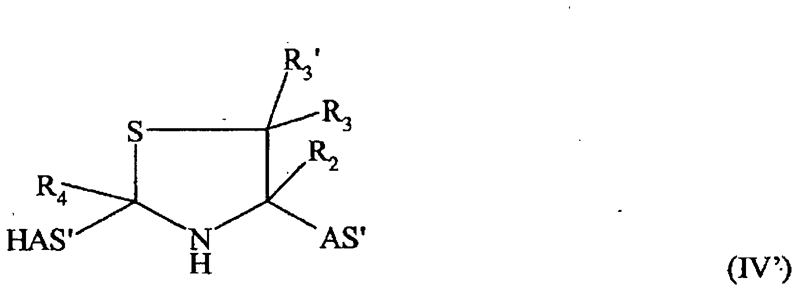 ou 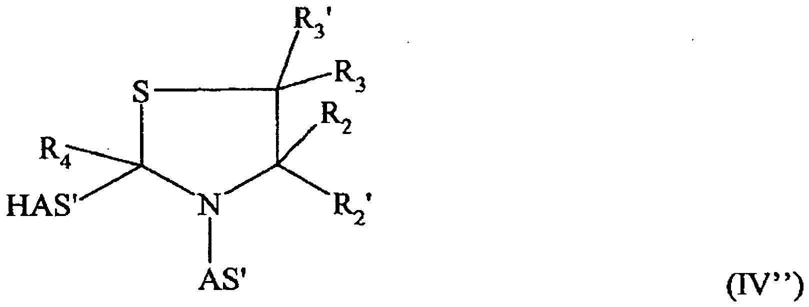 dans lesquelles HAS' est le résidu du dérivé d'hydroxyalkylamidon qui a été lié à un groupement aldéhyde, un groupement céto ou un groupement hémiacétal par la réaction de l'hydroxyalkylamidon avec au moins un composé au moins bifonctionnel, ledit composé comprenant deux fonctions M₁ et Q, une fonction M₁ étant mise en réaction avec l'hydroxyalkylamidon et une fonction Q étant
(i) un groupement aldéhyde, un groupement céto, un groupement hémiacétal ; ou
(ii) une fonction étant modifiée par voie chimique pour donner le groupement aldéhyde, le groupement céto, le groupement hémiacétal,
et dans lesquelles AS' est le résidu de la substance active ou d'un dérivé de celle-ci qui a été lié au groupement alpha-SH-bêta-amino,
ou une structure selon la formule (V), (V') ou (V") 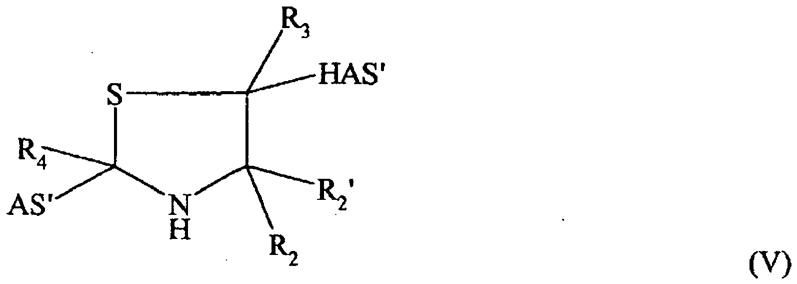 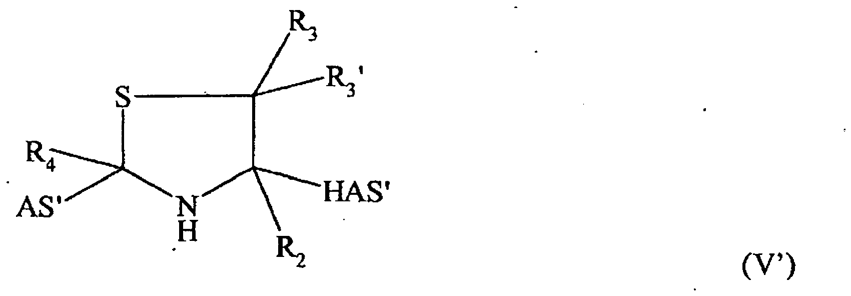 ou 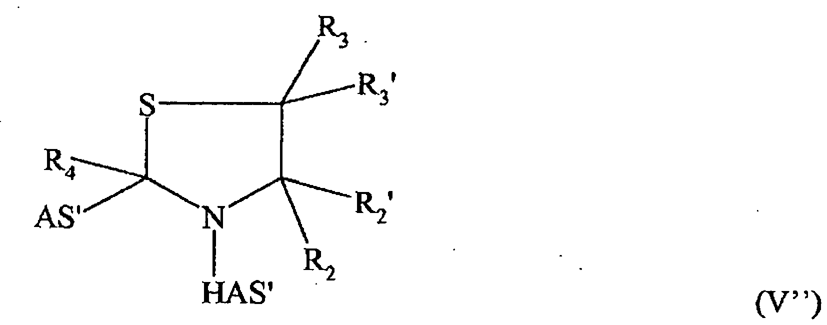 dans lesquelles HAS' est le résidu du dérivé d'hydroxyalkylamidon qui a été lié au groupement alpha-SH-bêta-amino par une méthode comprenant la réaction de l'hydroxyalkylamidon avec au moins un composé au moins bifonctionnel, ledit composé comprenant deux fonctions M₁ et Q, une fonction M₁ étant mise en réaction avec l'hydroxyalkylamidon et une fonction Q étant
(i) un groupement alpha-SH-bêta-amino ; ou
(ii) une fonction étant modifiée par voie chimique pour donner le groupement alpha-SH-bêta-amino,
et dans lesquelles AS' est le résidu de la substance active ou d'un dérivé de celle-ci qui a été lié au groupement aldéhyde, au groupement céto ou au groupement hémiacétal.

27. Conjugué selon la revendication 26, **caractérisé en ce que** le conjugué est choisi dans le groupe constitué de 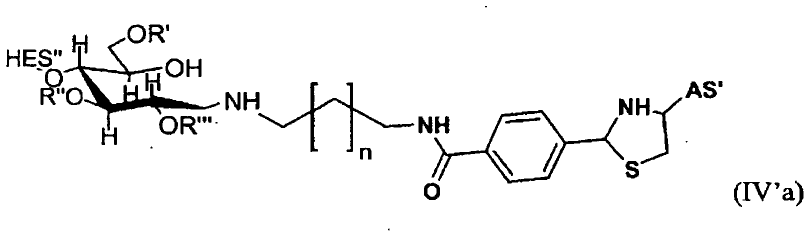 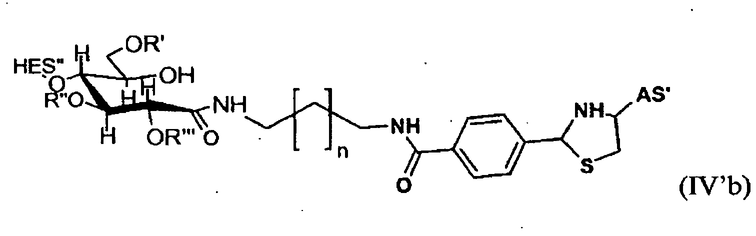 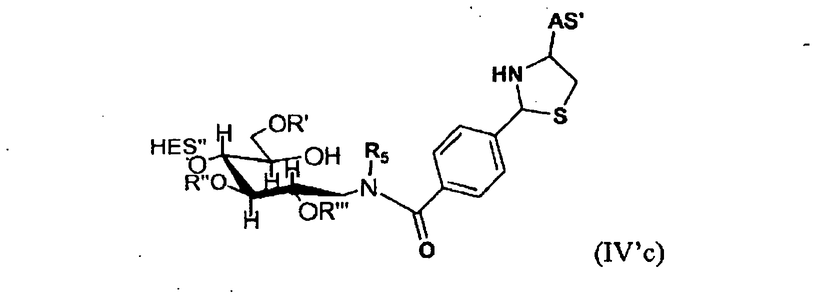 où R₅ est tel que défini pour R₁ à R₄ ci-dessus, 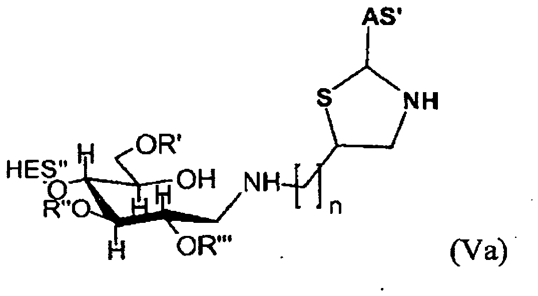 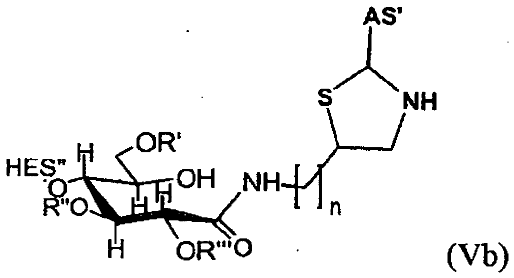 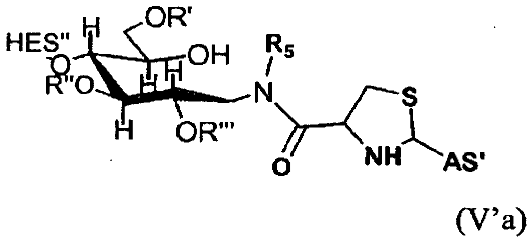 où R₅ est tel que défini pour R₁ à R₄ ci-dessus, 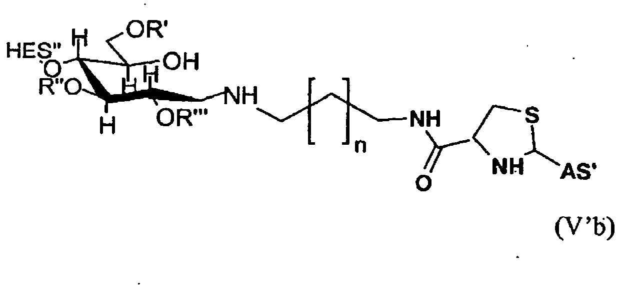 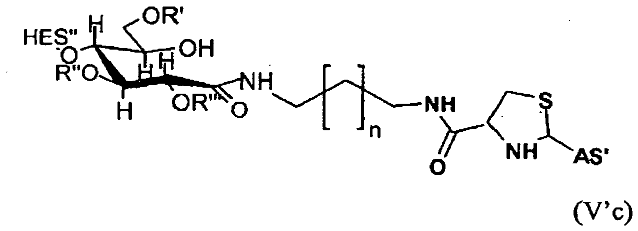 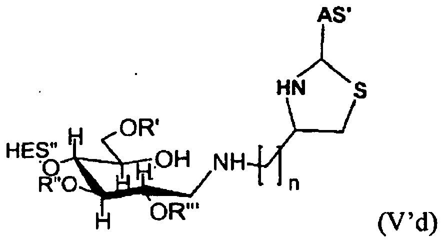 et 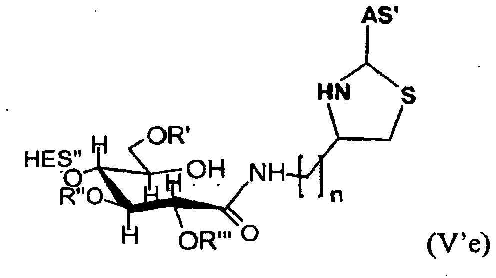 où dans la formule IV'a, IV'B, V'B ou V'C, n est un entier, de préférence n est 0 à 20,
et où dans la formule Va, Vb, V'd ou V'e, n est un entier, de préférence n est 1 à 20.

28. Conjugué selon la revendication 26, **caractérisé en ce que** le conjugué est 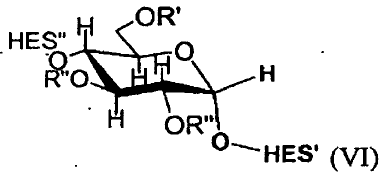 où R', R" et/ou R"' sont tels que définis pour la formule II et où, dans au moins un motif glucose de HES, au moins l'un de R', R" et/ou R"' est choisi indépendamment dans le groupe constitué de 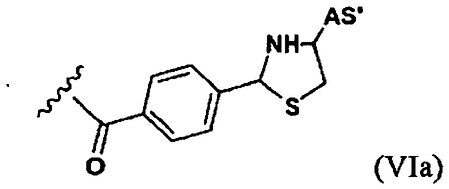 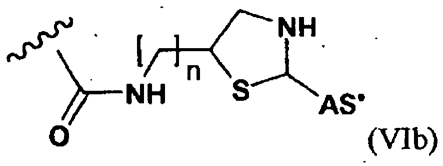 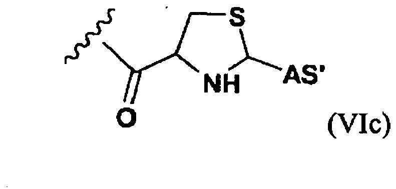 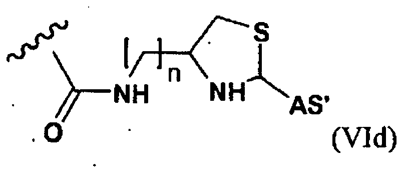 et où n est un entier, de préférence 1 à 20 et/ou où au moins l'un de R', R" et/ou R"' est -(CH₂CH₂O)ₘ-R^{#}, où m est un entier, de préférence de 1 à 3, et R^{#} est choisi dans le groupe constitué des formules (VIa), (VIb), (VIc) et (VId).

29. Dérivé d'hydroxyalkylamidon à fonction alpha-SH-bêta-amino choisi dans le groupe constitué de 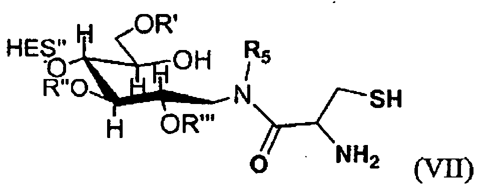 où R₅ est tel que défini pour R₁ à R₄ ci-dessus,
et 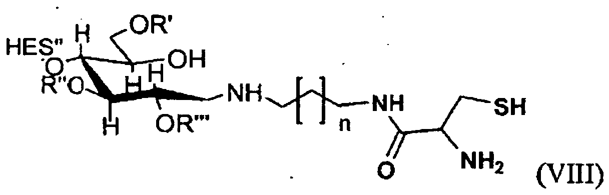 où n est un entier, de préférence 0 à 20,
ou le groupe constitué de 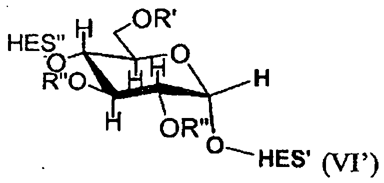 où R', R" et/ou R"' sont tels que définis pour la formule II et où dans au moins un motif glucose de HES, au moins l'un de R', R" et/ou R"' est choisi indépendamment dans le groupe constitué de 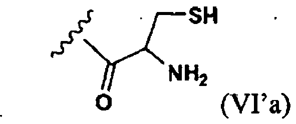 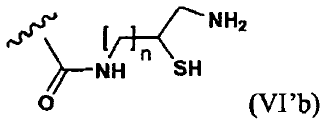 et 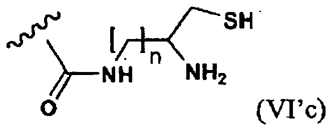 et où n est un entier, de préférence de 1 à 20 et/ou où au moins l'un de R', R" et/ou R"' est - (CH₂CH₂O)ₘ-R^{##}, où m est un entier, de préférence de 1 à 3, et R^{##} est choisi dans le groupe constitué des formules (VI'a), (VI'b) et (VI'c).

30. Conjugué selon l'une quelconque des revendications 25 à 28, destiné à être utilisé dans une méthode de traitement du corps humain ou animal.

31. Conjugué selon l'une quelconque des revendications 25 à 28, en tant qu'agent thérapeutique.

32. Composition pharmaceutique comprenant un conjugué selon l'une quelconque des revendications 25 à 28, de préférence comprenant en outre au moins un diluant, un adjuvant ou un support pharmaceutiquement acceptable.

33. Composition comprenant un conjugué d'une substance active et d'hydroxyalkylamidon selon l'une quelconque des revendications 25 à 28.
